# EUROPEAN PATENT SPECIFICATION

(11) **EP 0 876 160 B1**
(45) Date of publication and mention of the grant of the patent: **22.12.1999**
(21) Application number: 97901190.5
(22) Date of filing: 24.01.1997
(51) Int. Cl.: A61K 47/48

(54) **LIGAND DIRECTED ENZYME PRODRUG THERAPY**
LIGANDGERICHTETE ENZYME-PRODRUG THERAPIE
THERAPIE PAR PROMEDICAMENT ENZYMATIQUE CIBLE PAR UN LIGAND

(30) Priority: 26.01.1996 GB 9601640
(43) Date of publication of application: 11.11.1998
(73) Proprietor: CANCER RESEARCH CAMPAIGN TECHNOLOGY LIMITED, London NW1 4JL (GB)
(72) Inventor: SPRINGER, Caroline Joy, Institute of Cancer Res., Sutton, Surrey SM2 2NG (GB); MARAIS, Richard, Chester Beatty Labs., London SW3 6JB (GB)
(74) Representative: Brasnett, Adrian Hugh
(86) International application number: GB9700221
(87) International publication number: WO9726918

(56) References cited:
- EP-A- 0 121 352
- WO-A-92/14748
- WO-A-94/21679
- WO-A-95/02420
- WO-A-95/03830
- WO-A-95/24928
- WO-A-96/06641
- WO-A-96/21416
- MELTON RG ET AL: "Tumour necrosis factor increases tumour uptake of co-administered antibody-carboxypeptidase G2 conjugate." EUR J CANCER, 1993, VOL. 29A, NO. 8, PAGE(S) 1177-1183, XP002038769
- N.P. MINTON ET AL.: "The complete nucleotide sequence of the Pseudomonas gene coding for carboxypeptidase G2" GENE, vol. 31, 1984, AMSTERDAM NL, pages 31-38, XP002038770
- LEUNG D W ET AL: "VASCULAR ENDOTHELIAL GROWTH FACTOR IS A SECRETED ANGIOGENIC MITOGEN" SCIENCE, vol. 246, no. 4935, 8 December 1989, pages 1306-1309, XP000083690
- TERMAN B I ET AL: "IDENTIFICATION OF THE KDR TYROSINE KINASE AS A RECEPTOR FOR VASCULAR ENDOTHELIAL CELL GROWTH FACTOR" BIOCHEMICAL AND BIOPHYSICAL RESEARCH COMMUNICATIONS, vol. 187, no. 3, 30 September 1992, pages 1579-1586, XP002013861
- MATTHEWS W ET AL: "A RECEPTRO TYROSINE KINASE CDNA ISOLATED FROM A POPULATION OF ENRICHED PRIMITIVE HEMATOPOIETIC CELLS AND EXHIBITING CLOSE GENETIC LINKAGE TO C-KIT" PROCEEDINGS OF THE NATIONAL ACADEMY OF SCIENCES OF USA, vol. 88, no. 20, 15 October 1991, pages 9026-9030, XP000615976
- P.S. STEYGER ET AL.: "Intratumoural distribution as a determinant of tumour responsiveness to therapy using polymer-based macromolecular prodrug" JOURNAL OF CONTROLLED RELEASE., vol. 39, 1996, AMSTERDAM NL, pages 35-46, XP002038771
- MARINI FC 3RD ET AL: "In vivo marking of spontaneous or vaccine-induced fibrosarcomas in the domestic house cat, using an adenoviral vector containing a bifunctional fusion protein, GAL-TEK." HUM GENE THER, SEP 1995, VOL. 6, NO. 9, PAGE(S) 1215-23, XP002038772
- MELTON R.G. ET AL: "Antibody-enzyme conjugates for cancer therapy" JOURNAL OF THE NATIONAL CANCER INSTITUTE, 21-2-1996, VOL. 88, NO. 3-4, PAGE(S) 153-165, XP002038773
- COOPER D.L. ET AL: "Gene therapy advances: Utilization of alternative splicing as a control element in the chimeric enzyme/prodrug therapy (CEPT) approach to primary and metastatic tumors" JOURNAL OF CLINICAL LIGAND ASSAY, 1996, VOL. 19, NO. 1, PAGE(S) 80-84, XP002038774

## Description

### Introduction and background to the invention

The present invention relates to ligand directed enzyme prodrug therapy (LIDEPT) and its use in the treatment of disease, including tumours.

A therapeutic approach termed "antibody-directed enzyme prodrug therapy" (ADEPT) has been proposed as a method for treating tumour cells in patients using prodrugs. Tumour cells are targeted with an antibody conjugated to an enzyme capable of activating a prodrug. The antibody in the conjugate binds to tumour cells in order that the enzyme can convert the prodrug to an active drug outside the tumour cells (see e.g. WO88/07378). Alternatively, methods for the delivery of genes which encode an enzyme have been used (see e.g. EP-A-415731). Such methods include calcium phosphate co-precipitation, microinjection, liposomes, directed DNA uptake, and receptor-mediated DNA transfer. These are reviewed in Morgan & French Anderson, Annu. Rev. Biochem., 1993, 62; 191. The term "GDEPT" (gene-directed enzyme prodrug therapy) is used to include both viral and non-viral delivery systems.

The present invention relates to a new class of enzyme delivery systems, involving the use of ligands which recognise receptors on the surface of tumour cells. Tumour cells are targeted with a ligand-enzyme conjugate or a ligand-enzyme fusion.

### Disclosure of the invention

The present invention provides a two component system for use in association with one another comprising:
(a) a fusion protein or conjugate of a ligand with enzyme; said ligand being:
   (i) a naturally occurring polypeptide whose biological role is to bind to a cognate receptor on the surface of the cell;
   (ii) a fragment of said polypeptide which still binds to its cognate receptor; or
   (iii) derivatives of (i) and (ii) above with altered receptor specificity; and
(b) a prodrug which can be converted into an active drug by said enzyme.

The invention also provides the system of the invention for use in a method of treatment of a patient, and a method of treating a tumour in a patient in need of treatment which comprises administering to said patient an effective ligand-enzyme capable of binding to the receptor and a prodrug capable of being converted by said enzyme to an active drug. The invention further provides novel conjugates of a ligand (or fragments or derivatives thereof) with an enzyme.

### Brief description of the drawings

Figure 1 shows schematically a ligand, an enzyme, and some fusion proteins containing them.
Figure 2 shows Western blots of tissue culture supernatants from cells expressing constructs of the invention.
Figure 3 shows the purification of CPV₁₆₅ by chromatography.
Figure 4 shows purification of fusion proteins by affinity chromatography.
Figure 5 shows a dimer analysis of fusion proteins.
Figure 6 shows a schematic representation of DNA and protein sequences of the VEGF receptor.
Figure 7 shows expression of the VEGF receptor in Sf9 cells.
Figure 8 shows binding of fusion proteins to the receptor.
Figure 9 shows non-specific cell cytotoxicity directed by fusion proteins of the invention.
Figure 10 shows VEGF dependent cell cytotoxicity directed by a fusion protein of the invention.

### Detailed description of the invention.

### A. Ligands

Ligands which are naturally occurring polypeptides whose biological role is to bind to a cognate receptor are preferably naturally occurring mammalian polypeptides. Desirably, such polypeptides will be selected from the species of mammal which is to be treated by the invention. Thus, when the system of the invention is intended for human use, the polypeptide will preferably be of human origin. In veterinary applications, the polypeptide may be, for example, bovine, ovine, porcine, canine or feline. In research applications, the polypeptide may be of these or other origin, e.g. primate, rodent (such as mouse, rat or rabbit).

Examples of ligands include epidermal growth factor, EGF, (which binds to epidermal growth factor receptor), heregulin and c-erbB2 ligand. EGFR and c-ErbB2 are expressed in a number of tumour types.

In general, examples of suitable ligands include those that recognise receptors that are expressed exclusively or, in greater number, on tumour cells or on vasculature that is in the vicinity of the tumour.

Such an example of the latter is the vascular endothelial growth factor (VEGF) receptor. VEGF is a regulator of tumour angiogenesis which is produced by malignant cells and acts on tumour endothelial cells which express high affinity VEGF receptors. The known VEGF receptors are tyrosine kinases called flt-1 and flk-1, which are specifically expressed in endothelial cells of the tumour and in the border between the tumour and normal tissue (Science 253, 989, 1992; Cell 72 835, 1993). Flk-1 is specifically expressed in endothelial cells during embryonic development but is down-regulated in the adult, when angiogenesis stops. Tumours are dependent on angiogenesis since they require a continuous source of nutrients and oxygen by tumour vasculature in order to grow further. Therefore the ligand, VEGF which binds to flt-1 and/or flk-1 may be used to selectively target an enzyme to the tumour environment. The advantage in targeting the tumour endothelium is that it overcomes the problem of poor penetration of large molecules (eg. antibodies) due to lack of accessibility. Also, the clearance time *in vivo* for a ligand is much shorter, so that less time is required between administration of the ligand-enzyme, for clearance from normal tissues and blood, before injection of the prodrug, than is required for antibody-enzyme conjugates.

The VEGF may be conjugated to, or made as a fusion protein with, a non-endogenous enzyme that can selectively catalyse a prodrug to a toxic drug in LIDEPT. In this LIDEPT system, flt-1 and/or flk-1 would effectively be targeted with a non-endogenous enzyme that converts prodrugs to toxic drugs. Enzymes and prodrugs which may be used include those disclosed in WO88/07378, WO89/10140, WO90/02729, EP-A-415 731, WO91/03460, WO93/08288, WO94/25429, WO95/02420, WO95/03830, PCT/GB95/01783 and PCT/GB95/01782, the disclosures of which are incorporated herein by reference.

Using this anti-angiogenic approach will serve a triple purpose since the toxic drugs are small molecules that are able to diffuse through the tumour vasculature and the tumour. Firstly it will kill the tumour vasculature endothelial cells. Secondly, it will kill the tumour cells by exerting a bystander effect. Thirdly, it will also kill the cells of the tumour by starving them of nutrients and oxygen.

Fragments of a ligand may also be used, providing they still bind to the cognate receptor to which the ligand binds. Desirably, the binding will be of substantially similar affinity, e.g. from about 10-fold lower to 10-fold higher, or higher affinity, e.g. up to about 20 to 100-fold higher. The binding will also be of substantially similar selectivity for the cognate receptor, so that the fragment does not cross react or bind to other cell surface receptors to a substantially greater degree than the ligand itself.

The binding affinity of fragments may be determined by standard techniques, e.g. I¹²⁵ ligand competition assays. The binding specificity may be determined by e.g. I¹²⁵ ligand-enzyme biodistribution *in vitro*.

Fragments of ligands may be generated in any desired way. For example, ligands may be cleaved chemically, eg. using trypsin or other protein fragmentation compounds such as cyanogen bromide, or other proteolytic enzymes. Fragments of the ligands may be separated by chromatography and recovered.

Conditions for cleavage, chromatography and recovery of proteins are well known in the art. More usually, fragments will be generated by recombinant DNA techniques. DNA encoding the ligands can be spliced at restriction sites to delete regions of the DNA encoding the ligand. Where the fragment of the ligand is a C-terminal truncated fragment, the DNA may be altered by site directed mutagenesis to introduce a stop codon at the desired point of truncation. Other methods of manipulating DNA may be found in standard reference books, eg Sambrook *et al*, Molecular Cloning, CSH, 1987.

The truncated DNA may then be expressed in a recombinant expression system to produce the ligand fragment, and the fragment recovered. As described herein, the DNA encoding the fragment may also be linked to DNA encoding the enzyme of the LIDEPT system so as to provide a fusion protein.

Derivatives of ligands and their fragments may also be made by recombinant DNA techniques. Site directed mutagenesis may be used to introduce amino acid substrations, deletions or insertions into the ligand. One class of substitution which may be introduced is conservative substitutions.

Conservative substitutions may be made according to the following table, where amino acids on the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |
| OTHER | | N Q D E |

As with ligand fragments, it is preferred that the derivatives bind to their cognate receptor with substantially similar or higher affinity and selectivity as the ligand itself, wherein the preferred degree of selectivity and affinity is as defined above for ligand fragments.

### B. Enzymes

The enzyme may be any enzyme which is not normally expressed on the surface of a cell, nor released into the circulation, particularly a mammalian (especially human) cell, and which is capable of converting a prodrug into an active drug. The enzyme may be a mammalian enzyme which does not naturally occur in a human or a human enzyme which is not normally accessible to the prodrug. This includes enzymes from other species as well as mammalian enzymes which are altered in a manner which is selective for the prodrug. In other words, the alteration means that the conversion of the prodrug to an active drug by the natural enzyme will be at a rate one or more orders of magnitude less than the rate at which the altered enzyme operates. Altered enzymes may be made by standard recombinant DNA techniques, e.g. by cloning the enzyme, determining its gene sequence and altering the gene sequence by methods such as site-directed mutagenesis.

The enzyme will usually convert the prodrug into an active drug by removing a protecting group from the prodrug. In most cases, the protecting group will be cleaved as a whole from the prodrug. However, it is also possible for the enzyme to cleave or simply alter part of the protecting group, resulting in a partially cleaved or altered protecting group which is unstable, resulting in spontaneous removal of the remainder of the group. Such prodrugs are of particular use in association with the nitroreductase enzyme described below.

Preferably, the enzyme is a non-mammalian enzyme. Suitable non-mammalian enzymes include bacterial enzymes. Bacterial enzymes include carboxypeptidases, such as carboxypeptidase G2 (CPG2), as disclosed in WO88/07378, and Pseudomonas γ-glutamylhydrolase EC3.4.22.12 (Levy CC & Goldstein P J. Biol. Chem. 242; p2933 (1967)) and nitroreductases, such as an E.coli nitroreductase as disclosed in WO93/08288. Examples of other suitable enzymes include thymidine kinase (tk), especially viral tk such as VZV or HSV tk; and β-lactamase and β-glucoronidase. Other enzymes include penicillin V amidase, penicillin G amidase and cytosine deaminase.

Fusion proteins will be expressed in both eukaryotic (e.g. insect, mammalian) cells and non-eukaryotic (bacterial) cells as required for each ligand. Many ligands are processed through the Golgi apparatus and endoplasmic reticulum, where they become glycosylated; this may be important for binding activity. In these cases, eukaryotic based expression systems will be employed. In the case of fusion proteins, especially for fusions between non-eukaryotic enzymes and ligands, where the ligand needs to pass through the golgi, the enzyme may also pass through the golgi/ER and may consequently also become glycosylated; this may lead in a reduction in activity of the enzyme compared to its non-glycosylated form.

In a preferred aspect of the invention the enzyme has been altered by substitution, deletion or insertion at one or more (e.g. two, three or four) glycosylation sites. For example, within the primary amino acid sequence of CPG2, there are three such consensus motifs, located at residues Asn 222, Asn 264 and Asn 272. Alteration of one or more of these sites is preferred when CPG2 is used in the present invention. Desirably, the alteration is substitution to leucine or glutamine.

In general, alterations to enzymes which are amino acid substitutions of from at least 1 to 11 glycosylation sites are particularly preferred, although deletions or insertions of for example 1, 2, 3, 4, 5 or more amino acids are also possible. In any event, the alteration will be such that the enzyme retains its ability to convert a prodrug to an active drug at substantially the same rate as the unchanged, unglycosylated enzyme. In this context, "substantially unchanged" will desirably be within 1 order of magnitude, and preferably from about 2-fold less to 2, 5 or 10 fold more.

In addition to specific changes the enzyme may otherwise be altered by truncation, substitution, deletion or insertion as long as the activity of the enzyme is substantially unchanged as defined above. For example, small truncations in the N-and/or C-terminal sequence may occur as a result of the manipulations required to produce a vector in which a nucleic acid sequence encoding the enzyme is linked to the various other signal sequences described herein. The activity of the altered enzyme may be measured in suitable model systems which can be prepared in routine ways known in the art.

In a further aspect of the present invention, there is provided a vector comprising a bacterial carboxypeptidase gene which has been altered by substitution, deletion or insertion at one or more glycosylation sites, fused in-frame to a gene encoding a ligand or fragment or derivative thereof. The ligand gene is fused at either the 5' or 3' end of the carboxypeptidase gene, and the two may be fused directly or spaced by a linker sequence encoding one or more (eg. up to 10, 20 or 100) amino acids. The fusion will be operably linked to a promoter capable of expressing the fusion construct in a host cell. The carboxypeptidase is desirably CPG2 with the amino acid sequence of SEQ ID No. 2 except for said one or more substitutions, deletions or insertions. Variants of such carboxypeptidases containing further substitutions, deletions or insertions but which retain substantially unchanged carboxypeptidase activity are a further part of this aspect of the invention. Such variants may for example include truncated enzymes as discussed above.

Alterations may also be introduced into the sequences encoding the ligand. Truncation, insertions deletions and specific point mutations may be required to stabilise the fusion protein and to prevent it from being internalised. Also, additional "linker regions" may be required between the ligand and enzyme to give flexibility between the two components and thus allow each to be active.

The invention also provides a nucleic acid which may be RNA or DNA encoding such fusion protein and vectors comprising such a nucleic acid. The nucleic acid fusion is preferably between that of SEQ ID No. 2 for the enzyme (except where altered to remove one or more glycosylation sites such as in SEQ ID No. 4), and SEQ ID No. 6 for the ligand or fragments thereof encoding the above mentioned variants of carboxypeptidase. The vector may be an expression vector, wherein said nucleic acid is operably linked to a promoter compatible with a host cell for expression of the fusion protein. The invention thus also provides a host cell which contains an expression vector of the invention. The host cell may be bacterial (e.g. E.coli), insect, yeast or mammalian (e.g. hamster or human).

Host cells of the invention may be used in a method of making a fusion protein or fragment thereof as defined above which comprises culturing the host cell under conditions in which said enzyme or fragment thereof is expressed, and recovering the enzyme or fragment thereof in substantially isolated form. Polyhistidine residues in conjunction with nickle-affinity based systems may be used for this purpose.

### C. Other vector components

In the system according to the invention the fusion may be linked to a signal sequence which directs the fusion to be exported from the cells. This will usually be a mammalian signal sequence or a derivative thereof which retains the ability to direct the enzyme to the cell surface. Some ligands have a naturally occurring signal sequence, which may also be used. For example the VEGF signal sequence may be employed for this purpose. If the fusion does have such a signal sequence, it can be replaced by another signal sequence where this is desirable or appropriate. Suitable signal sequences include those found in transmembrane receptor tyrosine kinases such as the c-erbB2 (HER2/neu) signal sequence or variants thereof which retain the ability to direct expression of the enzyme at the cell surface. The c-erbB2 signal sequence can be obtained by reference to Coussens *et al* (1985) Science 230; 1132-1139.

The experiments described in the Examples herein may be used to determine variants of this, or other signal sequences, for their ability to express the enzyme at the cell surface. The variants may be produced using standard techniques known as such in molecular biology, eg. site-directed mutagenesis of a vector containing the signal sequence.

Further suitable signal sequences include those which may be found in the review by von Heijne (1985) J. Mol. Biol. 184; 99-105.

Vectors encoding the ligand and enzyme, together with, when required, a signal sequence may be made using recombinant DNA techniques known per se in the art. The sequences encoding the ligand enzyme and signal sequence may be constructed by splicing synthetic or recombinant nucleic acid sequences together, or modifying existing sequences by techniques such as site directed mutagenesis. Reference may be made to "Molecular Cloning" by Sambrook *et al* (1989, Cold Spring Harbor) for discussion of standard recombinant DNA techniques.

### D. Promoters

The enzyme will be expressed in the vector using a promoter capable of being expressed in the cell in which the vector is to be expressed. The promoter will be operably linked to the sequences encoding the enzyme and its associated sequences. Suitable host cells include those mentioned above, and promotes from such host cells may be used. Viral promoters operable in such host cells may also be used.

### E. Prodrugs

The prodrug for use in the system will be selected to be compatible with the enzyme, ie. such that the enzyme will be capable of converting the prodrug into an active drug. Desirably, the toxicity of the prodrug to the patient being treated will be at least one order of magnitude less toxic to the patient than the active drug. Preferably, the active drug will be several, eg 2, 3, 4 or more orders of magnitude more toxic. Suitable prodrugs include nitrogen mustard prodrugs and other compounds such as those described in WO88/07378, WO89/10140, WO90/02729, WO91/03460, EP-A-540 263, WO94/02450, WO95/02420 or WO95/03830 which are incorporated herein by reference.

### E(i) - Nitrogen mustard prodrugs

Nitrogen mustard prodrugs include compounds of the formula:

M-Ar-CONH-R

where Ar represents an optionally substituted ring aromatic ring system, R-NH is the residue of an α-amino acid R-NH₂ or oligopeptide R-NH₂ and contains at least one carboxylic acid group, and M represents a nitrogen mustard group.

The residue of the amino acid R-NH is preferably the residue of glutamic acid. It is disclosed in WO88/07378 that the enzyme carboxypeptidase G2 is capable of removing the glutamic acid moiety from compounds of the type shown above, and the removal of the glutamic acid moiety results in the production of an active nitrogen mustard drug.

Thus nitrogen mustard prodrugs of use in the invention include the prodrugs of generic formula I of WO94/02450 and salts thereof, and in particular those of formula (I): 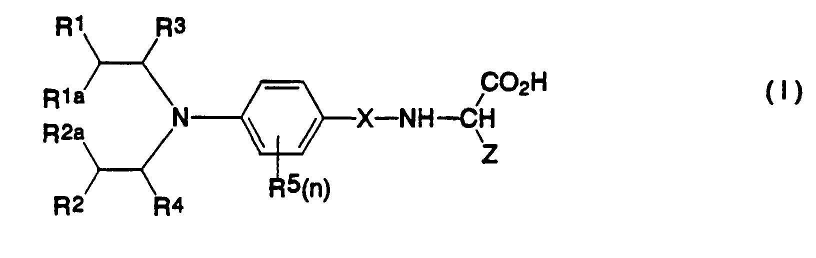
wherein R¹ and R² each independently represent chlorine, bromine, iodine, OSO₂Me, OSO₂phenyl (wherein phenyl is optionally substituted with 1,2,3,4 or 5 substituents independently selected from C₁₋₄alkyl, halogen, -CN or NO₂);
R^{1a} and R^{2a} each independently represents hydrogen, C₁₋₄ alkyl or C₁₋₄ haloalkyl;
R³ and R⁴ each independently represents hydrogen, C₁₋₄ alkyl or C₁₋₄ haloalkyl;
n is an integer from 0 to 4;
each R⁵ independently represents hydrogen, C₁₋₄ alkyl optionally containing one double bond or one triple bond, C₁₋₄ alkoxy, halogen, cyano, -NH₂, -CONR⁷R⁸ (wherein R⁷ and R⁸ are independently hydrogen, C₁₋₆ alkyl or C₃₋₆ cycloalkyl) or two adjacent R⁵ groups together represent
   a) C4 alkylene optionally having one double bond;
   b) C3 alkylene; or
   c) -CH=CH-CH=CH-, -CH-CH-CH2- or -CH2-CH=CH- each optionally substituted with 1, 2, 3 or 4 substituents said substituents each independently selected from the group consisting of C₁₋₄ alkyl, C₁₋₄ alkoxy, halogen, cyano and nitro;
X is a group -C(O)-, -O-C(O)-, -NH-C(O)- or -CH₂-C(O)-; and
Z is a group -CH₂-T-C(O)-OR⁶ where T is CH₂, -O-, -S-, -(SO)- or -(SO₂)-, and R⁶ is hydrogen, C₁₋₆ alkyl, C₃₋₆ cycloalkyl amino, mono- di-C₁₋₆ alkylamino or mono or diC₃₋₆ cycloalkyl amino, provided that when R⁶ is hydrogen T is -CH₂-; and physiologically acceptable derivatives, including salts, of the compounds of formula (I).

Halogen includes fluorine, chlorine, bromine and iodine. Preferred values for the groups R^{1a} and R^{2a} are methyl and hydrogen, especially hydrogen. Preferred values for the groups R³ and R⁴ are hydrogen, methyl and trifluoromethyl, especially hydrogen. Preferred values for the groups R¹ and R² are I, Br, Cl, OSO₂Me and OSO₂phenyl wherein phenyl is substituted with one or two substituents in the 2 and/or 4 positions. I, Cl and OSO₂Me are especially preferred.

Preferred values for R⁵ when n is an integer from 1 to 4 are fluorine, chlorine, methyl-CONH₂ and cyano. Preferably, n is 0, 1 or 2. When n is 1 or 2 it is preferred that R⁵ is fluorine at the 3 and/or 5 positions of the ring. The group X is preferably -C(O)-, -O-C(O)- or -NH-C(O)-. Z is preferably a group -CH₂CH₂-COOH.

Preferred specific compounds include:
N-4-[(2-chloroethyl)(2-mesyloxyethyl)amino]benzoyl-L-glutamic acid (referred to below as "CMDA") and salts thereof;
N-(4-[bis(2-chloroethyl)amino]-3-fluorophenylcarbamoyl)-L-glutamic acid and salts thereof;
N-(4-[bis(2-chloroethyl)amino]phenylcarbamoyl)-L-glutamic acid and salts thereof;
N-(4-[bis(2-chloroethyl)amino]phenoxycarbonyl)-L-glutamic acid and salts thereof; and
N-(4-[bis(2-iodoethyl)amino]phenoxycarbonyl)-L-glutamicacid (referred to below as "prodrug 2") and salts thereof.

Particular sub-groups of the compounds of the present invention of interest may be obtained by taking any one of the above mentioned particular or generic definitions for R¹-R⁴, R⁵, X or Z either singly or in combination with any other particular or generic definition for R¹-R⁴, R⁵, X or Z.

Other prodrugs include compounds of the formula (II): 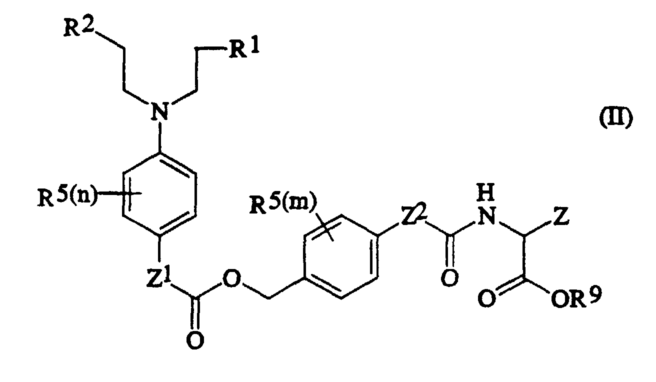
wherein R¹, R², R⁵, n and Z are as defined for compounds of the formula (I) above;
m is an integer from 0 to 4,
Z¹ and Z² are each independently -O- or -NH-; and
R⁹ is hydrogen, t-butyl or allyl;
   and physiologically acceptable derivatives of the compound of formula (I). Preferred values of R¹, R², R⁵, n and Z are as defined above for compounds of the formula (I). Preferred values of m are 0, 1 or 2 as defined for n above. R⁹ is preferably hydrogen, but can be protected especially during synthesis by groups such as allyl or t-butyl.

These prodrugs can be activated at the site of a tumour by a carboxypeptidase enzyme, for example, CPG2 as disclosed in WO88/07378 or WO94/02450.

Nitrogen mustard prodrugs of the formula (III): 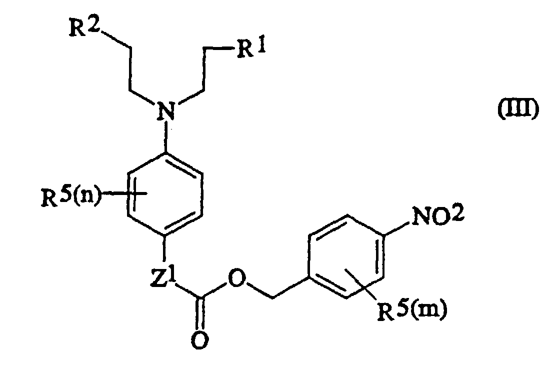 wherein R¹, R², R⁵, Z¹, n, and m are as defined for compounds of the formula (II), and physiologically acceptable derivatives thereof, may also be used in the present invention.

These prodrugs can be activated at the site of a tumour by a nitroreductase enzyme, for example, as disclosed in WO93/08288.

Usually to ensure enzyme activity a cofactor such as riboside or a ribotide of nicotinic acid or nicotinamide will be required and may be administered with the prodrug.

Compounds of the formulae (II) and (III) may be made using reactions and methods known *per se* in the art of chemistry, and also by reference to GB patent application 9501052.6 and PCT/GB96/00112 (WO96/22277) filed 19 January 1996 which claims priority from it. The following methods are of particular use:

### A: Compounds of formula (II) where Z¹ is -O-:

Compounds of the formula (I) in which Z¹ is -O- may be made by reacting a nitrogen mustard of formula (IV) 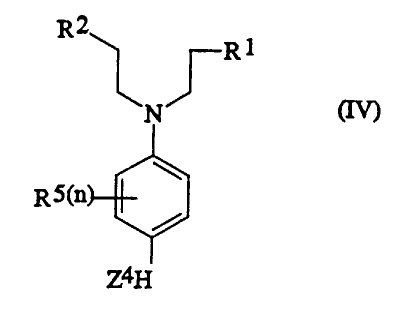 Where R¹, R², and R⁵ and n are as defined above and Z⁴ is -O- with a linker of formula (V) 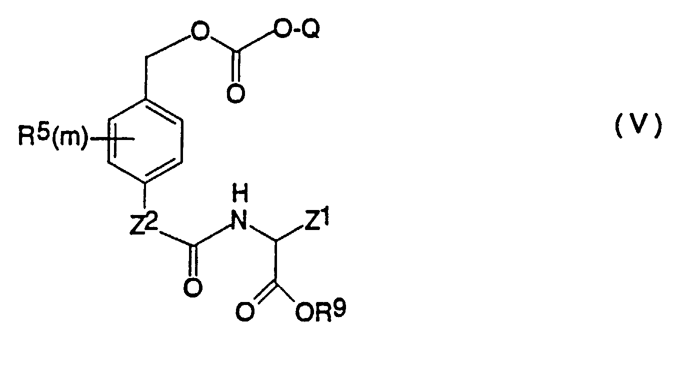 where R⁵, m, Z², R⁹ and Z¹ are as defined above, and Q is hydrogen or a leaving group. This reaction may be done in aprotic solvents in the presence of a base, for example DMF and triethylamine.

Preferred leaving groups Q include a succinimidyl group, a 4-nitrophenyl carbonate group, a pentafluorophenyl carbonate and a tetrachloroethyl group CH(Cl)CCl₃.
(ii) Compounds of the formula (IV) may be made starting from 4-nitrophenol optionally substituted with the group(s) R⁵₍ₙ₎ (as defined above). The phenolic group is protected as an adamantanyloxycarbonyl-derivative (by reacting the starting materials with adamantanyl-fluoroformate and triethylamine in THF at rt). The protected 4-nitrophenyl carbonate is reduced to the corresponding amine by hydrogen transfer in ethanol using ammonium formate and Pd/C 10% as catalyst at room temperature. The amine is then hydroxyethylated with ethylene oxide in AcOH at 20°C and then reacted to the desired nitrogen mustard. Reference may be made to EP-A-433 360 or EP-A-490970 for suitable conditions. The compounds may be purified by column chromatography. Deprotection to remove the adamantyl group may be carried out in trifluoroacetic acid.
(iii) Alternatively, the nitrogen mustard of formula (IV) may be activated as a chloroformate by treatment with phosgene or triphosgene in an aprotic solvent and triethylamine followed by coupling with a compound of formula (VI): 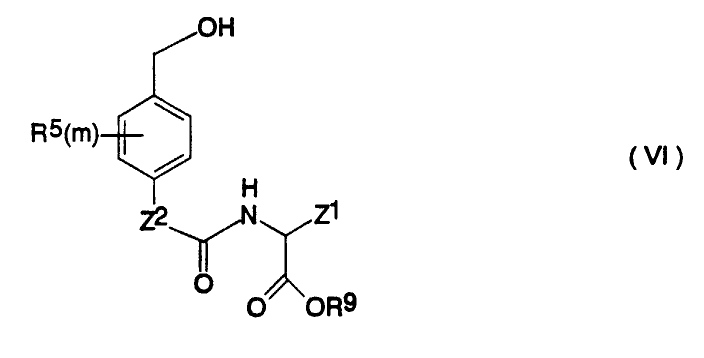 where R⁵, m, Z², R⁹ and Z¹ are as defined above. This may be carried out in THF or other aprotic solvents in the present of a base (for example triethylamine or pyridine).
(iv) A further alternative route of synthesis of compounds of the formula (II) in which Z¹ is -O- involves direct coupling of 4-nitrophenol optionally substituted with the group(s) R⁵₍ₙ₎ (as defined above) with the compound of the formula (V) or by reaction of the said optionally substituted 4-nitrophenol compound chloroformate with the compound of formula (V), followed in each case by the reaction described above to convert the nitro group, via an amine, to a mustard group.

### B: Compounds of formula (II) where Z¹ is -NH-:

(i) Compounds of the formula (II) in which Z¹ is -NH- may be made by reaction of a compound of formula (IV) in which Z⁴ is -NH- with a linker of the formula (V) in aprotic solvents and in the presence of a base. Compounds of the formula (IV) in which Z⁴ is -NH- may be made from a 1-halo-4-nitrobenzy compound, optionally substituted with the group(s) R⁵₍ₙ₎ (as defined above). This is converted to the corresponding 1-bis-hydroxyethylamino-4-nitro-benzyl compound by reaction with diethanolamine with heat and the resulting product purified by column chromatography. The corresponding 4-nitro nitrogen mustard may be made by for example mesylation using mesyl chloride in pyridine and subsequent reaction to other halo mustards, e.g. bromo or iodo mustards if required. The 4-nitro group may be reduced by hydrogen transfer in ethanol using ammonium formate and a Pd/C 10% catalyst at 20°C.
(ii) Alternatively the 1-bis-hydroxyethylamino-4-nitrobenzyl compound mentioned above can be reduced using ammonium formate and Pd/C 10% as catalyst in ethanol at 20°C to provide the corresponding phenylene-diamino derivative. This derivative can be converted into the corresponding 4-amino nitrogen mustard as described in the above paragraph, e.g. initially by reaction with mesyl chloride.

### C: Compounds of formula (III):

(i) Compounds of the formula (III) may be obtained by coupling nitrogen mustard phenol compounds described in section A(i) above with 4-nitrobenzyl choloroformate optionally substituted with the group(s) R⁵₍ₘ₎ (as defined above) in the presence or absence of triethylamine at 20°C.
(ii) Alternatively aniline nitrogen mustards as described in section B(ii) above may be used with the chloroformated as described in section C(i) above.

### D: Compounds of the formula (V) in which Z² is -NH-:

(i) Compounds of the formula (IV) in which Z² is -NH-may be made from a 4-nitro benzylic alcohol optionally substituted with the group(s) R⁵₍ₙ₎ (as defined above). The hydroxyl function is protected as a pyranyl- or *t*-butyl-dimethylsilyl (TBDMSi)-ether by treatment at 20°C with 3,4-2H-dihydropyran and pyridinium-p-toluensulfonate (PPTS) in an aprotic solvent or with TBDMSi chloride and imidazole in dimethylformamide (DMAC), respectively. The intermediate thus obtained is reduced to the corresponding amine by hydrogen transfer in ethanol using ammonium formate and Pd/C 10% as catalyst at 20°C. This amine is converted to a glutamyl ester intermediate of formula (VII): 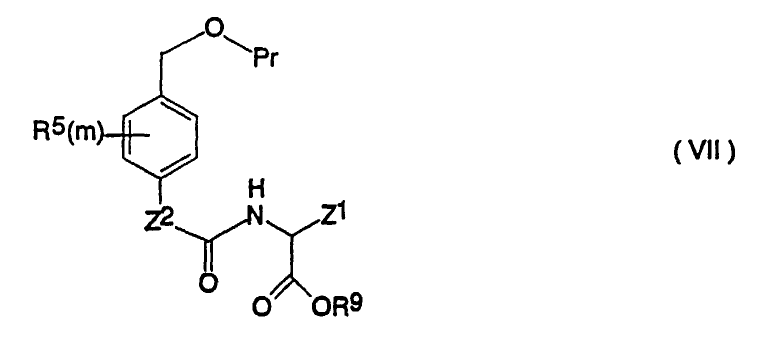 where R⁵, m, R⁹ and Z¹ are as defined above, Z² is -NH- and Pr is the pyranyl- or *t*-butyl-dimethylsilyl (TBDMSi)-ether protecting group. This may be done by treating the amine with triphosgene and triethylamine in toluene at 60°C to provide the corresponding isocyante, which is treated with a glutamate derivative of formula R⁹-C(O)-CH(NH₂)-Z¹ where R⁹ and Z¹ are as defined above. Alternatively the corresponding glutamyl-isocyanate obtained from the corresponding glutamate by treatment with triphosgene and triethylamine in toluene at -78°C may be reacted with the amine in a one pot procedure.
(ii) The compound of formula (VII) is deprotected to remove the TBDMSi or pyranyl groups by treatment with mild acidic media (AcOH, THF and H₂O or PPTS, EtOH, 55°C). This yields a compound of formula (VII) in which Pr is hydrogen. Compounds of the formula (V) in which Q is a leaving group may be prepared using standard reactions known in the art.
(iii) For example where Q is a succinimidyl group the compound of formula (VII) where Pr is hydrogen may be treated with disuccinimidyl-carbonate and triethylamine in acetonitrile. Where a 4-nitrophenyl carbonate group is desired treatment with 4-nitrophenyl chlorformate and triethylamine in THF may be used. A pentafluorophenyl carbonate may be added by in situ phosgenation of pentafluorophenol followed by coupling to the linker of formula (VII) in which Pr is hydrogen.

### E: Compounds of the formula (V) in which Z² is -O-:

(i) The starting materials for the linkers possessing a carbamic bond are unsubstituted or substituted (with the group(s) R⁵₍ₙ₎ (as defined above)) 4-hydroxy-benzylic alcohols. These type of linkers may require an extra electron withdrawing group on the aromatic nucleus in order to undergo 1,4-elimination. The 4-hydroxy group is specifically protected as an acetate by treating the starting material with acetyl-v-triazolo-[4,5-b]pyridine, 1N NaOH in THF at 20°C. The alcohol function of the acetate is further protected as pyranyl- or TBDMSi-ether by the procedures described in section D above. The acetate function is then deprotected to restore the 4-hydroxy group in NaHCO₃ aq. MeOH at 20°C. The resulting phenol compounds are reacted in a one pot procedure with a protected glutamyl-isocyanate as described in section D(i) above. This yields a compound of the formula of (VII) as shown above in which Z² is -O- and Pr is the pyanyl- or *t*-butyl-dimethylsilyl. (TBDMSi)-ether protecting group.
(ii) Deprotection of this compound yields a compound of the formula (VII) in which Pr is hydrogen. This may be converted to compounds of the formula (V) by methods analogous to those described in sections D(ii) and (iii) above.

### F: Alternative synthesis of compounds of formula (IV):

Compounds of the formula (IV) in which Q is hydrogen, fluoro, chloro, bromo or -O-(N-succinimide) may also be obtained by reference to WO95/02420 or WO95/03830.

### E(ii). Other prodrugs

Other compounds which may be used as prodrugs include p-nitrobenzyloxycarbonyl derivatives of cytotoxic compounds. Such compounds can be used in conjunction with a nitroreductase enzyme. It is believed that the nitroreductase enzyme converts the nitro group of the prodrug into a hydroxylamino or amino group, which results in the p-nitrobenzyloxycarbonyl moiety becoming activated and then self-immolating. This releases the active drug. These compounds include a compound of formula:

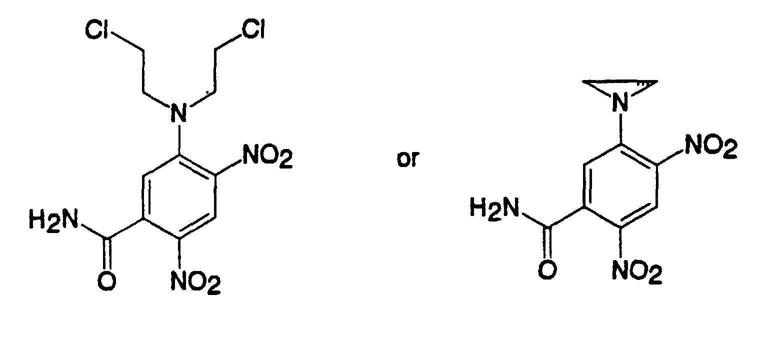

The nitroreductase enzyme of WO93/08288 requires a co-factor such as NADH or NADPH, and this may optionally be supplied as an additional component in the system of the invention.

Examples of other compounds described in the above references include prodrugs of actinomycin D, doxorubicin, daunomycan and mitomycin C. Prodrugs of the foregoing references are converted to active drugs by either nitroreductase or CPG2, although they may be modified to comprise protecting groups cleavable by other enzymes, eg β-lactamase or glucronidase.

Further prodrugs suitable for use in the invention include those of the general formula: FTLi-(PRT)_{m'} or salts thereof where FTLi is a ras inhibitor such as a farnesyltransferase inhibitor compound and PRT represents m' protecting groups capable of being cleaved from the ras inhibitor by the action of an enzyme, where m' is an integer from 1 to 5. Such compounds are disclosed in WO95/03830.

Other suitable prodrugs for use in the system of the invention include those which are derivatized with a sugar or a β-lactam derivative. For example, suitable linkers which may be attached to active drugs of the type described above are: 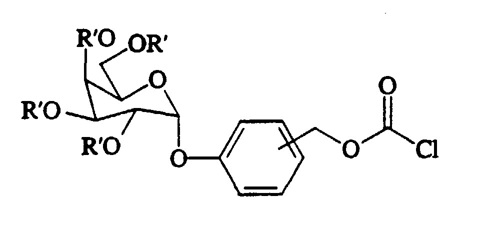 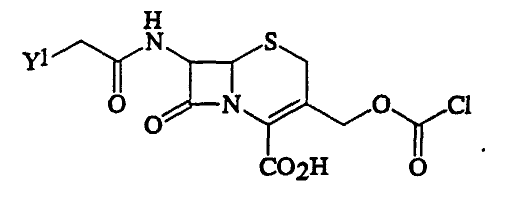 where R' is hydrogen or acetyl and Y¹ is aryl such as phenyl, benzyl or tolulyl, and these may be made in an analogous manner to the other prodrugs described above.

A further group of prodrugs are tyrphostin compounds of the general formula: PTKi-PRT_{m'} where PTKi is a compound with PTK (protein tyrosine kinase) inhibitory activity, PRT is a protecting group capable of being cleaved from the PTK inhibitor by the action of an enzyme and m' is an integer from 1 to 5.

Suitable tyrphostins such as the above may be obtained by the methods disclosed in, or analogous to those of, WO95/02420, Gazit *et al* 1989 and 1991, ibid, which are incorporated herein by reference.

### E(iii). Derivatives

Physiologically acceptable derivatives of prodrugs include salts, amides, esters and salts of esters. Esters include carboxylic acid esters in which the non-carbonyl moiety of the ester grouping is selected from straight or branched chain C₁₋₆alkyl, (methyl, n-propyl,, n-butyl or t-butyl); or C₃₋₆cyclic alkyl (e.g. cyclohexyl). Salts include physiologically acceptable base salts, eg derived from an appropriate base, such as alkali metal (e.g. sodium), alkaline earth metal (e.g. magnesium) salts, ammonium and NR_{4''} (wherein R'' is C₁₋₄ alkyl) salts. Other salts include acid addition salts, including the hydrochloride and acetate salts. Amides include non-substituted and mono- and di-substituted derivatives.

### F. Applications of the invention

The system of the invention can be used in a method of treatment of the human or animal body. Such treatment includes a method of treating the growth of neoplastic cells which comprises administering to a patient in need of treatment the system of the invention. It is also possible that the invention may be used to treat cells which are diseased through infection of the human or animal body by bacteria, viruses or parasites.

One suitable route of administration is by injection of the particles in a sterile solution. While it is possible for the prodrugs to be administered alone it is preferable to present them as pharmaceutical formulations. The formulations comprise a prodrug, together with one or more acceptable carriers thereof and optionally other therapeutic ingredients. The carrier or carriers must be "acceptable" in the sense of being compatible with the other ingredients of the formulation and not deleterious to the recipients thereof, for example, liposomes. Suitable liposomes include, for example, those comprising the positively charged lipid (N[1-(2,3-dioleyloxy)propyl]-N,N,N-triethylammonium(DOTMA), those comprising dioleoylphosphatidylethanolamine (DOPE), and those comprising 3β[N-(n',N'-dimethylaminoethane)carbamoyl]cholesterol (DC-Chol).

Formulations suitable for parenteral or intramuscular administration include aqueous and non-aqueous sterile injection solutions which may contain anti-oxidants, buffers, bacteriostatis, bactericidal antibiotics and solutes which render the formulation isotonic with the blood of the intended recipient; and aqueous and non-aqueous sterile suspensions which may include suspending agents and thickening agents, and liposomes or other microparticulate systems which are designed to target the compound to blood components or one or more organs. The formulations may be presented in unit-dose or multi-dose containers, for example sealed ampoules and vials, and may be stored in a freeze-dried (lyophilized) condition requiring only the addition of the sterile liquid carrier, for example water, for injections, immediately prior to use. Injection solutions and suspensions may be prepared extemporaneously from sterile powders, granules and tablets of the kind previously described.

It should be understood that in addition to the ingredients particularly mentioned above the formulations may include other agents conventional in the art having regard to the type of formulation in question. Of the possible formulations, sterile pyrogen-free aqueous and non-aqueous solutions are preferred.

The doses may be administered sequentially, eg. at daily, weekly or monthly intervals, or in response to a specific need of the patient. Preferred routes of administration are oral delivery and injection, typically parenteral or intramuscular injection or intratumoural injection.

In using the system of the present invention the prodrug will usually be administered following administration ligand-enzyme. Typically, the protein will be administered to the patient and then the uptake of the protein monitored, for example by recovery and analysis of a biopsy sample of targeted tissue or by injecting trace-labelled protein ligand enzyme.

The exact dosage regime will, of course, need to be determined by individual clinicians for individual patients and this, in turn, will be controlled by the exact nature of the prodrug and the cytotoxic agent to be released from the prodrug but some general guidance can be given. Chemotherapy of this type will normally involve parenteral administration of both the prodrug and modified protein and administration by the intravenous route is frequently found to be the most practical. For glioblastoma the route is often intratumoural. A typical dosage range of prodrug generally will be in the range of from about 1 to 150 mg per kg per patient per day, which may be administered in single or multiple doses. Preferably the dose range will be in the range from about 10 to 75, e.g. from about 10 to 40, mg per kg per patient per day. Other doses may be used according to the condition of the patient and other factors at the discretion of the physician.

Tumours which may be treated using the system of the present invention include any tumours capable or being treated by a LIDEPT system and thus are not limited to any one particular class of tumours. Particularly suitable tumour types include breast, colorectal and ovarian tumours, as well as pancreatic, melanoma, glioblastoma, hepatoma, small cell lung, non-small cell lung, muscle and prostate tumours. In the case of a LIDEPT system which uses VEGF, these and other types of solid tumours which comprise an actively growing vasculature are all candidates for treatment.

The system of the invention may also be used to treat infections diseases, for example, and any other condition which requires eradication of a population of cells.

It will be understood that where treatment of tumours is concerned, treatment includes any measure taken by the physician to alleviate the effect of the tumour on a patient. Thus, although complete remission of the tumour is a desirable goal, effective treatment will also include any measures capable of achieving partial remission of the tumour as well as a slowing down in the rate of growth of a tumour including metastases. Such measures can be effective in prolonging and/or enhancing the quality of life and relieving the symptoms of the disease.

The following Examples illustrate the invention.

### EXAMPLE 1

In order to demonstrate the invention, a fusion protein between CPG2 and VEGF was prepared. This fusion protein is formed between portions of c-ErbB2 (signal peptide), in order to direct expression outside of mammalian cells, CPG2 containing specific mutations which block glycosylation (N222, 264 and 272 > Q, as described in PCT/GB95/01782) and VEGF. The structure of the fusion protein is: c-ErbB2 (amino acids 1-27): Gly Ser :CPG2 (amino acids 23-415): Glu Phe Gly Gly Gly Gly Gly Thr Ala: VEGF 165 (amino acids 28-191).

The DNA and protein sequences of CPG2 are shown in SEQ ID No. 1 and 2 repectively, and this sequence was used except for the altered glycosylation sites as shown above. The sequence of VEGF can be found in Conn *et al* Proc. Natl. Acad. Sci. (1990) 87; 2628.

This fusion was cloned into the mammalian expression vector pEF Plink 2 (Marais *et al*, (1995) EMBOJ 14, 3136-3145) and transfected into COS-7 cells. In order to assess expression of the fusion protein, the culture medium from the transfected cells was analysed by immunoprecipitation, CPG2 enzyme activity and heparin binding activity.
(1) Immuno-precipitation.
   1 ml of tissue culture medium was immuno-precipitated with a CPG2 specific antiserum and the proteins eluted from the beads. The eluted proteins were anslysed by immuno-protein blot, and shown to contain a fusion protein with an apparent molecular weight of 60-64,000, which was not present in control transfected cells.
(2) The supernatants were also analysed for CPG2 activity using methotrexate as a substrate and found to contain CPG2 activity which was absent in the control transfection.
(3) Heparin-binding
   1 ml tissue culture medium was mixed with heparin-agarose, and the heparin agarose beads collected, washed and bound proteins were eluted and analysed by immuno-protein blotting using a CPG2 antiserum. The results show that supernatants from cells transfected with the fusion gene contained the CPG2-VEGF fusion protein and that this was competent to bind to heparin, whereas fusion between CPG2 and VEGF amino acids 28-136 did not bind to heparin.

These data demonstrate:
(1) The fusion protein can be synthesised and excreted by mammalian cells.
(2) The fusion protein contains CPG2 activity.
(3) The fusion protein is stable in tissue culture medium.
(4) the fusion protein is soluble.
(5) The fusion protein is able to bind to heparin, through the VEGF component.

### Example 2

### 1. Introduction

The VEGF proteins are secreted from cells via the endoplasmic reticulum (ER) and Golgi apparatus during which they become proteolytically processed and glycosylated to generate the mature proteins. (Eur J. Biochem, 211, 19, 1993). There are at least four splice variants of the gene, which encode different isoforms of the protein. The proteins that these genes encode for are referred to by the number of amino acids present in the mature protein with amino acid number 1 referring to the first amino acid in the mature protein. VEGF 165 is one of the isoforms of VEGF, which consists of 165 amino acids in the mature protein and which contains both a specific receptor interacting domain and a heparin-binding domain. By contrast, VEGF 121 is an isoform consisting of a protein of 121 amino acids which contains the specific receptor interacting domain, but not the heparin-binding domain (Science, 253, 989, 1992; Cell, 72, 835, 1993). The aim is to prepare VEGF-enzyme fusion proteins to activate a subsequently administered prodrug which will generate a cytotoxic agent at the tumour.

The bacterial enzyme Carboxypeptidase G2 (CPG2) is secreted from bacterial cells and is normally located in the periplasmic space. CPG2 catalyses the degradation of Methotrexate (MTX) and can also be used to cleave and thereby activate mustard prodrugs (J. Med. Chem, 33, 677, 1990). These prodrugs, which are relatively non-toxic are cleaved by CPG2 into active bifunctional alkylating agents, which are highly toxic to mammalian cells. If the signal peptide from CPG2 is replaced with a mammalian signal peptide, and CPG2 is expressed in mammalian cells, the protein enters the secretory pathway. However, this form of CPG2 becomes inappropriately glycosylated on three sites, resulting in an inactivate enzyme. The activity of this mammalian expressed form of CPG2 can be partially recovered if the three Asn residues that are in the core of the glycosylation motif are mutated into Gln residues. This results in a protein which retains about 10% of the activity of the bacterial enzyme.

The purpose of this invention is to target CPG2 to tumours where they will be employed to cleave and activate prodrugs (including mustard prodrugs). When CPG2 is targeted to the tumour, then the prodrug activation should only occur at that locus.

### 2. Description of the CPG2 and VEGF genes and Proteins Used

In considering the possible types of conjugated proteins they may be produced, this invention employs proteins produced from genes that are ligated so that the recombinant proteins that are produced are produced as ready-made fusions. Since VEGF is normally secreted from cells via the endoplasmic reticulum and Golgi apparatus, the aim was that the fusion proteins should pass through this secretory pathway so that the VEGF moiety would be expressed in its mature form. However, when CPG2 is processed through this secretory pathway in eukaryotic cells, it is subjected to inappropriate and inactivating glycosylation. Therefore constructions of fusions between VEGF and the form of CPG2 which cannot be glycosylated were made. The feasibility of fusions in which the VEGF moiety was either ligated to the amino-terminus (N-terminus) or the carboxyl-terminus (C-terminus) to CPG2 was tested. In addition, the presence or absence of the VEGF heparin-binding domain was examined for its ability to affect the activity of the fusion proteins. A series of fusion proteins were therefore constructed for expression in eukaryotic cells that would allow these parameters to be tested.

### 2.1 The CPG2 gene and protein

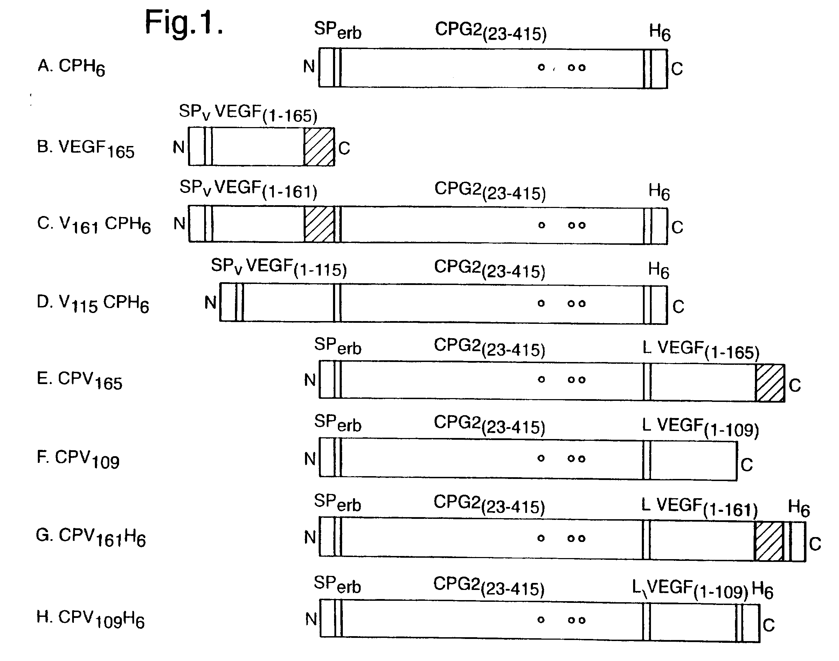

The CPG2 gene used was modified for expression in mammalian cells. The codons for the CPG2 signal peptide were replaced with the signal peptide from the human c-erbB2 protein to ensure efficient secretion of the CPG2 protein. For c-erbB2 signal sequence see Coussens *et al* (1985) Science 230; 1132-1139. Furthermore, the three Asn residues at the core of the glycosylation motifs were mutated to Gln residues to prevent glycosylation, and also a polyhistidine tag was added to the 3' end of the gene so that the fusion proteins could be purified by the nickel-NTA affinity chromatography. This construct is referred to as CPH₆ and is represented schematically in Figure 1A. For expression in mammalian cells, the gene for this construct was cloned into the mammalian expression vector pEFPlink 2. This vector was chosen since it uses the promoter from the elongation factor 1α gene to direct expression of foreign genes in mammalian cells and has been shown to be highly active in a variety of cell types. The structure of the protein that the CPH₆ gene is predicted to express is shown schematically in Fig. 1A; the complete DNA and protein sequences are given in SEQ ID Nos. 3 and 4.

### 2.2 The VEGF gene and protein

The VEGF gene in the plasmid pVEGF165 (in Bluescript) encodes for the VEGF signal peptide and a splice variant in which the mature protein consists of 165 amino acids. This encodes the specific receptor binding domain of VEGF encompassed within residues 1-121 for the mature protein. VEGF165 also encodes a heparin-binding domain, which requires the region of amino acids from 122-165 of the mature protein. In this gene there is a single glycosylation motif located at residue 75 of the mature protein (VEGF165) JBC, 266, 11947, 1991). The structure of the protein that the VEGF gene is predicted to express is shown schematically in Fig 1B; the complete DNA and protein sequences are given in SEQ ID Nos. 5 and 6.

### 3. Generation of the Fusion Proteins

In order to generate the various genes that were to be tested in the LIDEPT approach, the CPH₆ and VEGF165 genes were used. Mutations to produce the fusions were created by PCR directed mutagenesis, which has the advantage in its precise nature that it allows defined fusions to be generate at exact positions. the PCR techniques used are standard and can be found in any basic text, although an example of such a protocol is shown below.

DNA samples were heated through thermal cycles in a proprietary heat cycling instrument, in the presence of mutagenic primers, nucleotides and appropriate buffers to generate DNA samples that can then be cloned into appropriate recipient vectors. Typical heating cycles are presented below and the enzyme Taq polymerase was used to generate the DNA.
- 95°C: 60 sec
- 55°C: 30 sec
- 72°C: 30 sec
25 cycles were generally employed.

The products from these reactions were cloned into recipient vectors and analysis by dideoxy-sequencing techniques were used to verify the integrity of the fragments generated.

Fusions proteins were tested in which the VEGF and CPG2 proteins were fused to each other in both orientations and in which the VEGF moiety contains or lacks the heparin-binding domain. Four fusions were created, with CPG2 fused either to the N-, or to the C-terminus of VEGF, where the VEGF either contained or lacked the heparin binding domain. These constructs are represented schematically in Fig 1 and are described in detail below.

### 3.1 V₁₆₁-CPH₆

In this clone, the predicted protein product would be a fusion in which the CPG2 moiety was fused to the C-terminus of amino acids 1-161 of mature VEGF. Since the VEGF moiety is located at the N-terminus of the fusion, its own signal peptide will direct the protein to the secretion pathway of the cell. The c-erbB2 signal peptide was removed from the CPG2 gene. This fusion would therefore contain both the receptor-binding domain and also the heparin-binding domain of VEGF. It also contains a C-terminal polyhistidine tag for purification purposes.

The cloning of this fusion was performed as follows. The PCR was used in conjunction with oligonuleotides primers 1 and 2 (Table 1) and plasmid pVEGF165 to introduce silent mutations within the VEGF open reading frame that would destroy the internal Nco1 site located within that gene. This was performed to facilitate ease of cloning at the later stages. Next, a PCR fragment was generated by primers 3 and 4 (Table 1) in conjunction with the altered VEGF gene was digested with the restriction endonucleases Nco1 and *Bam HI* and the resulting fragment was cloned into those sites in plasmid pEFCPH₆. This strategy results in a gene that would encode the VEGF signal peptide, VEGF amino acids 1-161, fused to CPG2 amino acids 23-415, and containing a polyhistidine tag at the C-terminus for purification purposes. This construct is referred to as V₁₆₁CPH₆ and the protein is represented schematically in Fig 1C. The sequence of the V₁₆₁CPH₆ gene and the protein, it is predicted to encode, are given in SEQ ID Nos. 7 and 8.

### 3.2 V₁₁₅-CPH₆

In this clone, the CPG2 is fused to the C-terminus of VEGF. The portion of VEGF used for this clone is the first 115 amino acids of the mature protein and therefore does not contain the heparin-binding domain. As with V₁₆₁CPH₆ the signal peptide of VEGF will direct the secretion of the fusion protein and therefore the c-erbB2 signal peptide was removed.

In cloning this fusion protein, the internal Nco1 site within VEGF was destroyed with PCR and olignucleotides primers 1 and 2 (Table 1) as describe din Section 3.1, in order to facilitate easier cloning at the later stages. A PCR was performed in conjunction oligonucleotides 3 and 5 (Table 1) and the altered VEGF gene and the product was digested with the restriction endonucleases Nco1 and *Bam HI*. The resulting fragment was cloned into these sites in plasmid pEFCPH₆. This strategy produces a gene which would encode the signal peptide from VEGF and the first 115 amino acids of the mature protein fused to amino acids 22-415 of CPG2 and the polyhistidine tag. This construct is referred to as V₁₁₅CPH₆ and the protein is represented schematically in Fig 1D. The sequence of the V₁₁₅CPH₆ gene, and the protein that it is predicted to encode, are given in SEQ ID Nos. 9 and 10.

### 3.3 CPV₁₆₅

In this clone, CPG2 is fused to the N-terminus of VEGF. In this arrangement, the c-erbB2 signal peptide is used to direct secretion of the protein from the mammalian cells and so the VEGF signal peptide was removed. This fusion contains both the VEGF receptor binding domain and the heparin-binding domain. However, in the cloning of this gene, the polyhistidine tag was lost from the C-terminus of CPG2.

The cloning of this fusion was performed as follows. Standard cloning techniques employing oliognucleotides 6 and 7 (Table 1) were used to replace the polyhistidine tag at the 3' end of the CPH₆ gene with a peptide spacer of 6 amino acids and also to create a unique *Kpn I* site at that position. The structure of the linker is (Gly) 5Thr and was designed to given flexibility to the region between the two protein moieties. A PCR was performed with oligonucleotides 8 and 9 (Table 1) in conjunction with pVEGF165 to generate an altered VEGF gene. This PCR product was digested with the restriction endonucleases *Kpn I* and *Xba I* and cloned into the freshly created *Kpn I* site and *Xba I* sites of pEFCPH₆. This arrangement results in a gene whose structure is the signal peptide from c-erbB2 (amino acids 1-27-CHECK) fused to CPG2(Q)3 (amino acids 22-415) fused, via the spacer to codons 1-165 of mature VEGF. This construct is referred to as CPV₁₆₅ and the protein is represented schematically in Fig 1E. The sequence of the CPV₁₆₅ gene, and the protein it is predicted to encode, are given in SEQ ID Nos. 11 and 12.

### 3.4 CPV₁₀₉

During the cloning of CPV₁₆₅ it was noticed that one of the clones that was generated contained a mutation at codon 110 of the mature VEGF sequence. This converts an AGA (Arg) codon into a TGA (stop) codon and thus results in a truncation of VEGF at that position. Since this mutation fortuitously removes the heparin-binding domain of VEGF, we chose to characterise this fusion protein further to enable us to assess the results of having VEGF fused to the C-terminus of CPG2 in which the heparin-binding domain of VEGF was absent. This construct is referred to as CPV₁₀₉ and the protein is represented schematically in Fig 1F. The sequence of the CPV₁₀₉ gene and the protein that it is predicted to encode are given in SEQ ID Nos. 13 and 14.

### Figure 1. Schematic representation of CPG2, VEGF and the fusion proteins

Each of the proteins is represented as a bar which has variable shading to represent the different section of the fusions. The proteins are all depicted with the amino-terminus to the left and the carboxyl-terminus to the right (N and C respectively). The fusion junctions between the proteins are represented by open boxes. In the cases of CPV₁₆₅, CPV₁₀₉, CPV₁₆₅H₆ and CPV₁₀₉H₆, the peptide linker between the CPG2 and VEGF moieties is represented by a stippled box (L). Two forms of signal peptides have been employed to target the proteins for the secretory pathway. One is the signal peptide from the mammalian tyrosine kinase receptor c-erb B2 (SP_{erb}) and the other is the signal peptide from the VEGF gene (SPᵥ). Numbers in brackets indicate the amino acids present in either the CPG2 or VEGF motif for each fusion. The position of the amino acids that are required for heparin-binding by VEGF are indicated by the hatched box. The three sites in CPG2 which must be mutated to prevent glycosylation are indicated by ○. Where the fusions express a polyhistidine tag, this is indicated (H₆). The clones represented are: A, CPH₆; B. VEGF₁₆₅; C. V₁₆₁CPH₆; D. V₁₁₅CPH₆; E. CPV₁₆₅; F. CPV₁₀₉; G. CPV₁₆₁H₆; H. CPV₁₀₉H₆.

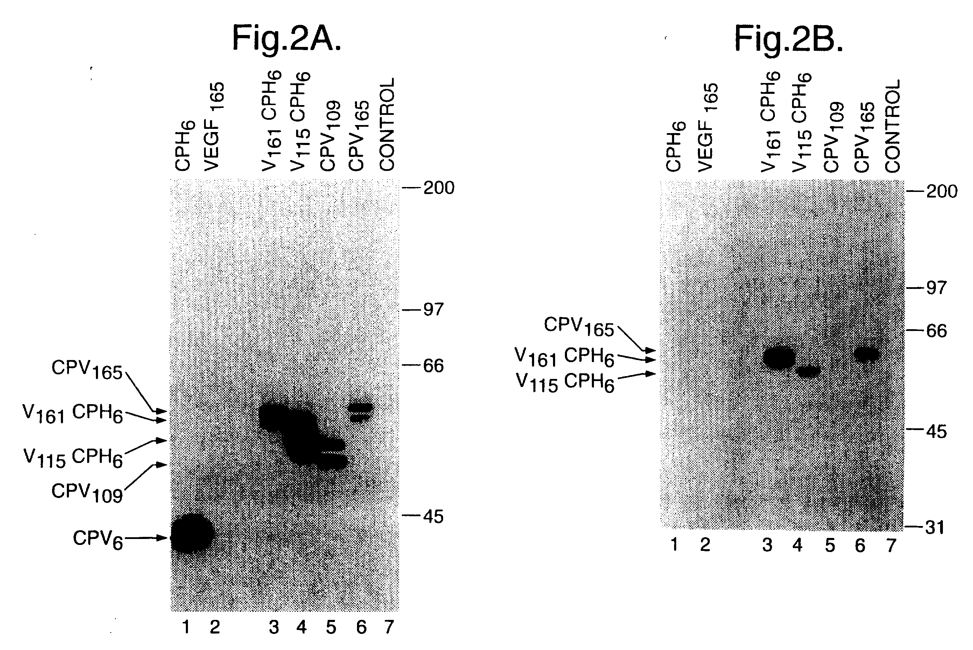

### Figure 2. Expression and heparin binding of CPH₆ and the fusion proteins

The tissue culture supernatants from the transfected COS cells as described in Table 2 were subjected to Western blot analysis and analysis for the ability to bind to heparin.

### A. Immunoprecipitation and immunoprotein blot analysis

For each sample, 500µL of the conditioned medium from cells transfected with the relevant expression vectors was made up to 0.1% v/v with Triton X-100 and then incubated with ∼5µL of a rabbit polyclonal antiserum specific for CPG2 (Marais *et al* 1996) immobilised on Protein G-Sepharose beads (Pharmacia). The complexes were formed for 6 hours at room temperature and then the beads were washed three times with 500µL of PBS containing 0.1% Triton X-100. The bound proteins were eluted from the beads, resolved on an 8% SDS-gel and analysed by immunoprotein blotting following standard techniques and using the CPG2 specific antiserum. The protein expressed in each sample is indicated above the appropriate lanes and are: lane 1, pEFCPH₆; lane 2, pEFVEGF₁₆₅; lane 3, pEFV₁₆₁CPH₆; lane 4, pEFV₁₁₅CPH₆; lane 5, pEFCPV₁₀₉; lane 6, pEFCPV₁₆₅; lane 7, control (pEFPlink.2). The position of migration of protein markers (x 10⁻³) is shown to the right of the figure and the positions of migration of the major products of each gene are indicated by the arrows to the left of the figure.

### B. Heparin-binding of the fusion proteins

For each sample, 500µL of the conditioned medium was made up to 0.1% v/v with Triton X-100 and then incubated with ∼35µL of Heparin-Sepharose beads at 4°C for ∼16 hours. The beads were washed three times with 500µL of PBS containing 0.1% Triton X-100 and the bound proteins were eluted from the beads, resolved on an 8% SDS-gel and analysed by immunoprotein blotting following standard techniques with the CPG2 specific antiserum (Marais *et al* 1996). The protein expressed in each sample is indicated above the appropriate lanes and are: lane 8, pEFCPH₆; lane 9, pEFVEGF₁₆₅; lane 10, pEFV₁₆₁CPH₆; lane 11, pEFV₁₁₅CPH₆; lane 12, pEFCPV₁₀₉; lane 13, pEFCPV₁₆₅; lane 14, control (pEFPlink.2). The position of migration of protein markers (x 10⁻³) is shown to the right of the figure and the positions of migration of the major products of each gene are indicated by the arrows to the left of the figure.

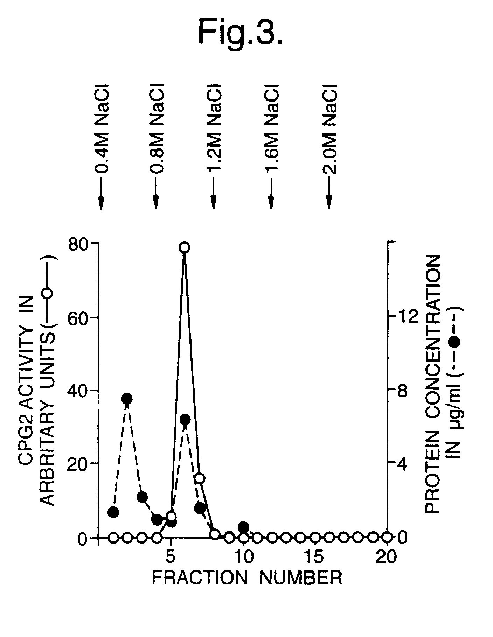

### Figure 3: Purification of CPV₁₆₅ by Heparin-Sepharose chromatography

Conditioned culture medium from mammalian cells transfected with pEFCPV₁₆₅ was subjected to a purification protocol by Heparin-Sepharose chromatography. For this protocol, 17ml of conditioned medium harvested from COS cells 72 hours post-transfection was passed over a 700µℓ Heparin-Sepharose CL 4B column which was then washed with 7ml column buffer (100mM Tris.HCl, 260µM ZnCl₂, pH 7.3). The column was eluted sequentially with 2.8mL column buffer containing 400mM NaCl, 2.8mℓ column buffer containing 800mM NaCl, 2.8mℓ column buffer containing 1.2M NaCl, 2.8 mL column buffer containing 1.6M NaCl and 2.8mℓ column buffer containing 2M NaCl at the positions indicated by the arrows. 0.7ml fractions were collected. The elution of bulk proteins is indicated (closed circles) and was determined using the Bio-Rad protein determination kit, with OD measurements at 595nm. The elution of the CPG2 activity was determined using MTX as a substrate as described in Table 2 and is indicated by the open circles.

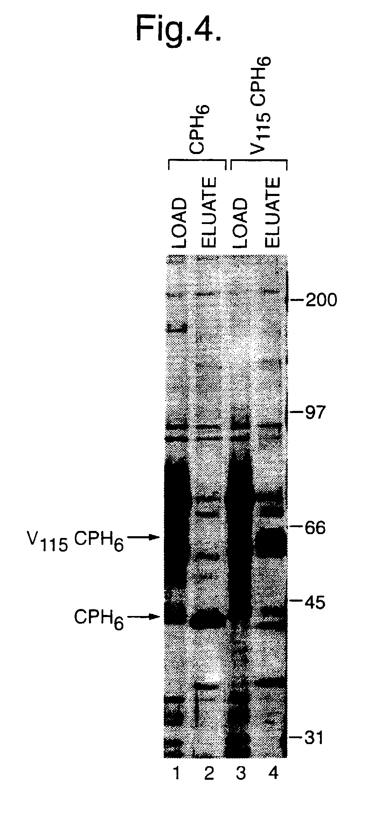

### Figure 4. Purification of fusion proteins by Ni++-NTA agarose affinity chromatography.

Sf9 cells were infected with baculoviruses encoding CPH₆ and V₁₁₅CPH₆ and the proteins were purified by Ni++-NTA agarose affinity chromatography as described in Table 5. Samples from the load fraction (LOAD, lanes 1, 3) and purified proteins (ELUATE, lanes 2, 4) were analysed by SDS-PAGE followed by silver staining to determine their purity. The samples for CPH₆ are in lanes 1 and 2 and the samples for V₁₁₅CPH₆ are in lanes 3 and 4. The position of migration of the purified proteins are indicated on the left of the figure as are the position of migration (x 10⁻³) of standard proteins, to the right of the figure.

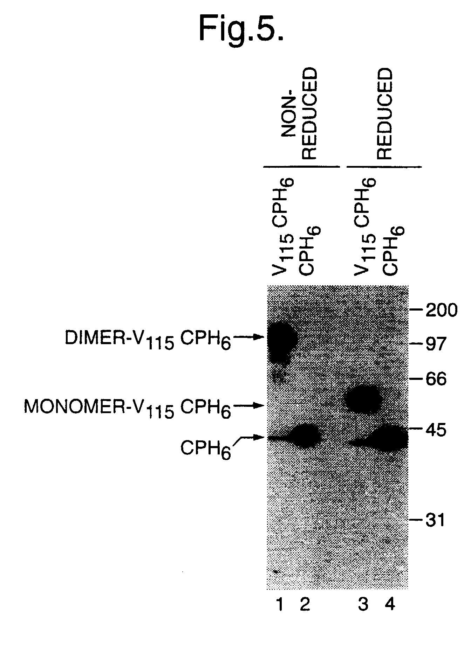

### Figure 5. Dimer analysis of fusion proteins.

V₁₁₅CPH₆ (lanes 1, 3) and CPH₆ (lanes 2, 4) expressed in Sf9 cells and purified by Ni++-NTA agarose affinity chromatography as described in Table 5 were used in this analysis. For each sample, ∼50ng of protein were heated to 65°C for 5 min in the absence (non-reduced; lanes 1, 2) or presence (reduced; lanes 3, 4) of 2-mercaptoethanol (5% v/v). The samples were resolved on a 12% SDS-gel and revealed by immunoprotein blotting with the CPG2 specific antiserum. The position of CPH₆ is indicated as are the positions of migration of dimeric (dimer-V₁₁₅CPH₆) or monomeric (monomer-V₁₁₅CPH₆) V₁₁₅CPH₆. The portion of standard proteins (x 10⁻³) is indicated to the right of the figure.

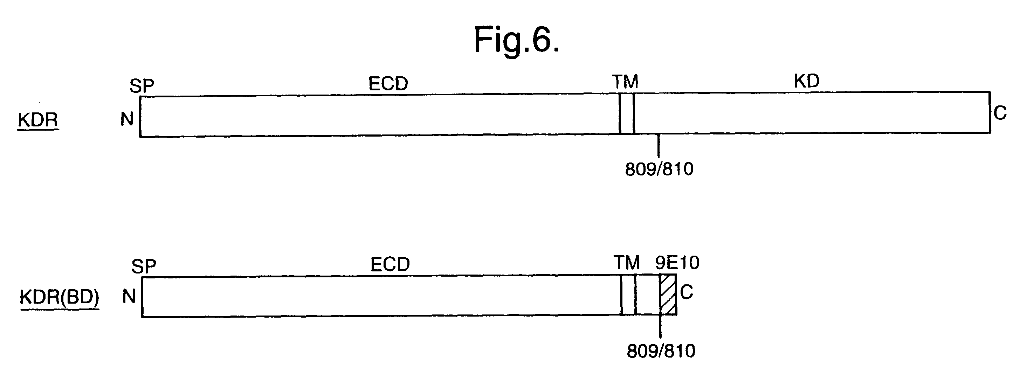

### Figure 6. Schematic representation and DNA and protein sequences of KDR(BD)

### A. Schematic representation of the KDR(BD).

The protein structures of KDR and KDR(BD) proteins are represented schematically. The proteins are in the conventional N-terminal to C-terminal orientation as indicated (N and C respectively). The top figure represents the complete KDR protein, where the individual regions are differently shaded. The regions represented are the signal peptide (SP), the extracelluar domain (ECD), transmembrane region (TM) and kinase domain (KD) are indicated. Also indicated are codons 809 and 810, where the EcoR1 site in the gene resides; this was used to truncate the protein to make KDR(BD) as shown in the lower schematic. The 9E10 epitope which was added to the C-terminus of the truncated protein is represented as a hatched box.

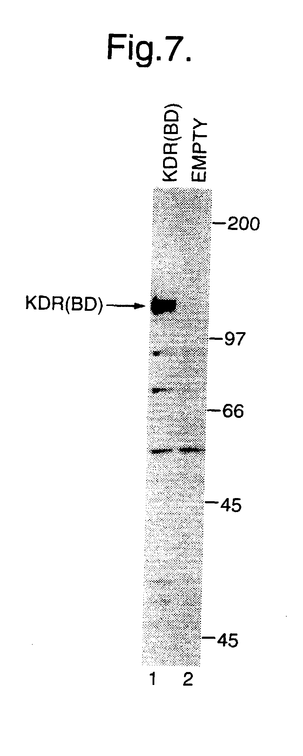

### Figure 7 : Expression of KDR(BD) in Sf9 Cells

Sf9 cells were infected with viruses expressing KDR(BD), or as a control, a virus with a defective polyhedron gene which does not encode any know protein (empty). For each sample, 10⁷ cells were extracted in lysis buffer (20mM Tris.HCl, 0.5mM EDTA, 10% v/v glycerol, 03M KCl, 1% v/v Triton X-100, 5µg/ml Leupeptin, 5µg/ml Pepstatin A, 50µg/ml phenylmethylsulphonyl flouride, 1mM benzamidine, pH8) 60 hours post infection. The protein content of the extracts was determined using the Bio-Rad protein determination kit. For each extract (KDR(BD), lane1; empty, lane 2), 4.5µg of protein was loaded onto a 7% SDS-Gel and the proteins therein detected by immunoprotein blotting, using the 9E10 mouse monoclonal antibody. The position of migration of the KDR(BD) is indicated by the arrow, and the portion of standard proteins (x 10⁻³) is indicated to the right of the figure.

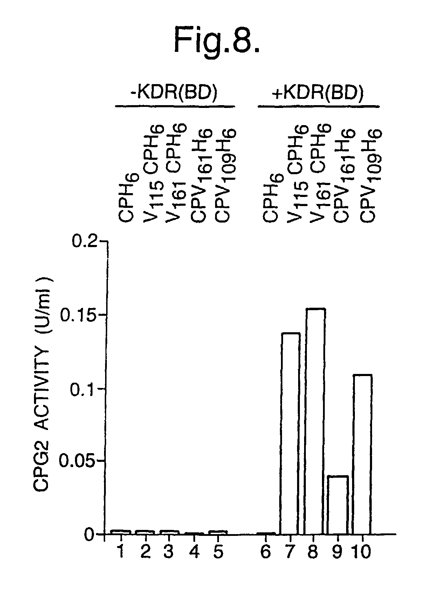

### Figure 8 : Binding of fusion proteins to KDR(BD)

The ability of the fusion proteins to bind to the KDR(BD) was tested in vitro. The KDR(BD), expressed in Sf9 cells was immunoprecipitated with the 9E10 monoclonal antibody and then the ability of purified fusion protein samples to bind to the 9E10/KDR(BD) immunocomplex was determined by measuring the CPG2 enzyme activity. For each sample, the KDR(BD) from 750µg of insect cell extract, as prepared in Fig 7 was immunoprecipitated with ∼50µg of 9E10 monoclonal antibody immobilised on protein G-Sepharose beads (+ KDR(BD); lanes 6-10). As a control, extracts from cells infected with the control virus (- KDR(BD); lanes 1-5) were used instead of the KDR(BD) expressing virus. The VEGF/CPG2 fusion proteins were also expressed in Sf9 cells and prepared as Ni++-NTA Agarose purified proteins, as described in Table 5. The fusion proteins were balanced against each other by CPG2 enzyme activity and for each fusion, the protein represented by 4.8mU of CPG2 activity was added to the 9E10/KDR(BD) immunocomplexes. The samples were incubated for 2 hours at 4°C and washed twice in wash buffer 500 (50mM Tris.HCl, 500mM NaCl, pH8) and then once in wash buffer 100 (50mM Tris.HCl, 500mM NaCl, pH8). The amount of CPG2 activity retained by the immunocomplexes was determined as described in Table 4.

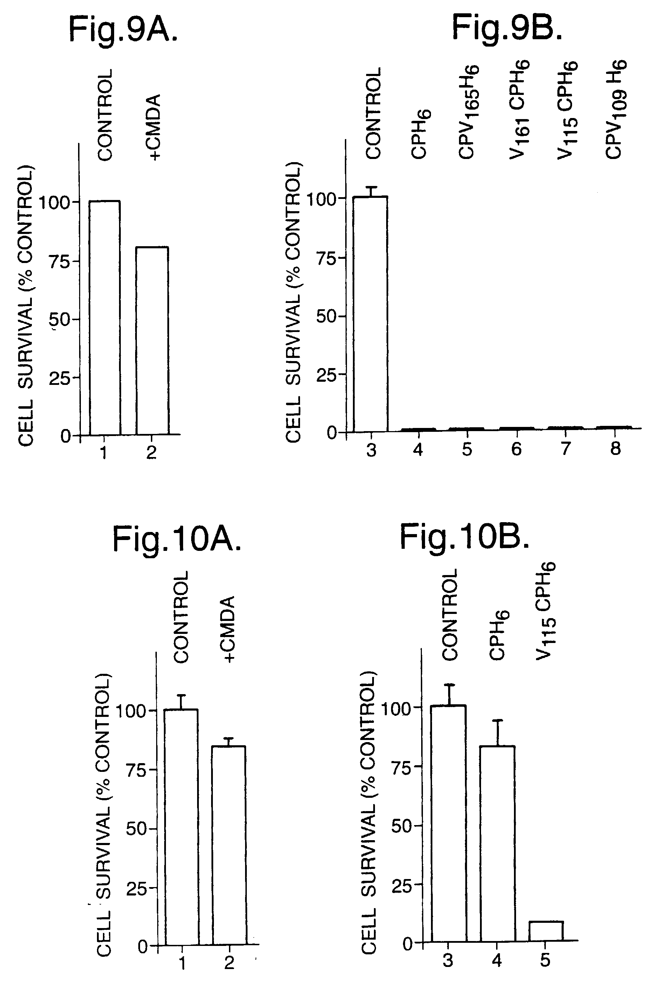

### Figure 9 : Non-specific cell cytotoxicity directed by the fusion proteins

### A. Sensitivity of NIH3T3 cells to the CMDA prodrug.

Confluent NIH3T3 cells were cultured in the absence (control, lane 1) or presence of 1mM CMDA prodrug (+CMDA, lane 2) for 18 hours. After incubation, the cells were diluted into fresh dishes (at a dilution of 100 fold) and allowed to grow for a further 6 days after which time the survival of the cells was determined by [³H] thymidine incorporation. The results are expressed in terms of the % growth in terms of the control, which were considered to represent 100% cell growth.

### B. Sensitivity of NIH3T3 cells to the CMDA prodrug in the presence of the fusion proteins.

Confluent NIH3T3 cells were cultured in the presence of 1mM CMDA prodrug in the absence of any additions (control, lane 3) or in the presence of the indicated fusion proteins (lanes 4-8). The additions were : lane 4, CPH₆; lane 5, CPV₁₆₁H₆; lane 6, V₁₆₁CPH₆; lane 7, V₁₁₅CPH₆; lane 8, pEFCPV₁₀₉H₆. For each fusion, 0.3 to 0.9 nM of the fusions were added as determined by quantitative immunoprotein blotting. The cells were incubated and processed as in section A above for cell growth. The results are expressed in terms of the % growth in terms of the control lane, which were considered to represent 100% cell growth.

### Figure 10 : VEGF dependent cell cytotoxicity directed by the fusion protein V₁₁₅CPH₆

### A. Sensitivity of Hu-V-ec cells to the CMDA prodrug.

Confluent Hu-V-ec cells were cultured in the absence (control, lane 1) or presence of 1mM CMDA prodrug (+ CMDA, lane 2) for 18 hours. After incubation, the cells were diluted into fresh dishes (at a dilution of 3 fold) and allowed to grow for a further 6 days after which time the survival of the cells was determined by [³H] thymidine incorporation. The results are expressed in terms of the % growth in terms of the control, which were considered to represent 100% cell growth.

### B. Sensitivity of Hu-V-ec cells to the CMDA prodrug in the presence of the fusion proteins.

Confluent Hu-V-ec cells were cultured in the absence of any additions (control, lane 3) or in the presence of CPH₆ (27 nM, lane 4), or of V₁₁₅CPH₆ (11nM, lane 5) for 30 min. The cells were then washed 6 times with fresh medium and incubated overnight in the presence of 1mM CMDA. The cells were re-plated at a dilution of 1/3 and allowed to grow for a further 4 days after which time the cell growth was estimated by [3H]thymidine incorporation. The results are expressed in terms of the % growth in terms of the control lane, which were considered to represent 100% cell growth.

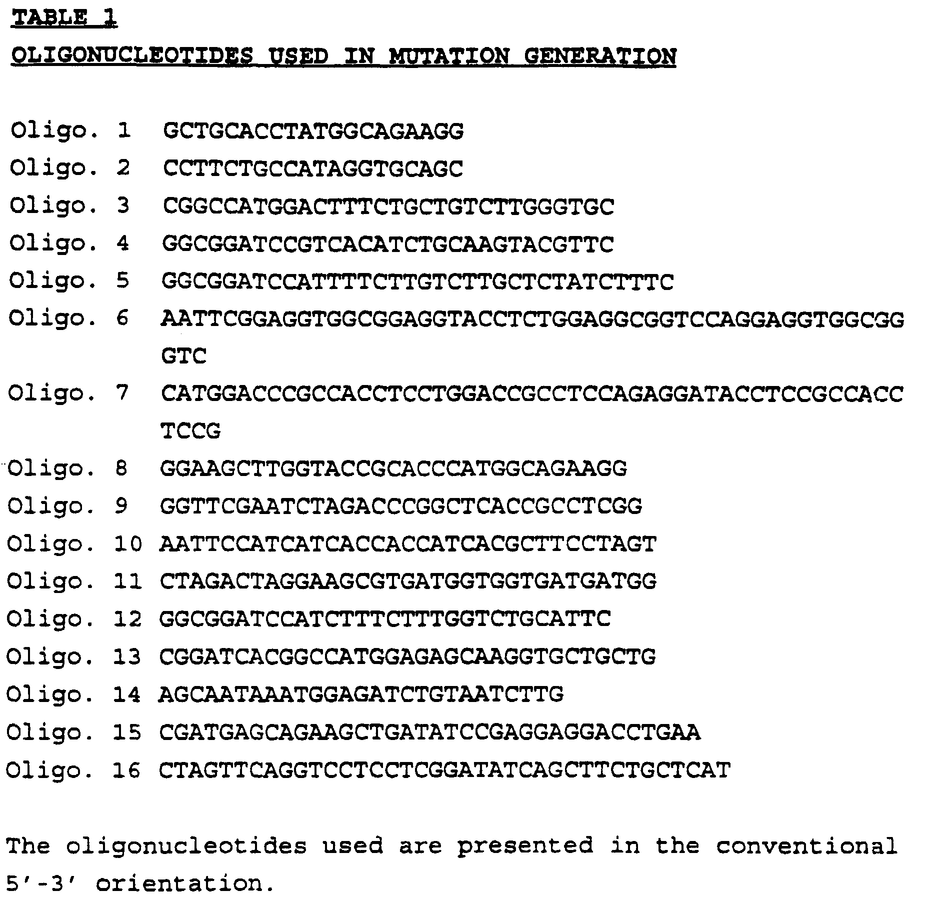

**TABLE 2**

| **ANALYSIS OF CPG2 ACTIVITY IN CONDITIONED CULTURE MEDIUM FROM THE TRANSFECTED COS CELLS** | |
|---|---|
| FUSION PROTEIN EXPRESSED | CPG2 ACTIVITY U/ml* |
| Control | 0 |
| pEFCPH₆ | 0.096 |
| pEFV₁₆₁CPH₆ | 0.011 |
| pEFV₁₁₅CPH₆ | 0.045 |
| pEFCPV₁₆₅ | 0.004 |
| pEFCPV₁₀₉ | 0.17 |
| pEFVEGF₁₆₅ | 0 |
| The tissue culture medium from COS cells transfected with the indicated vector was harvested and examined for CPG2 activity. Transfactions were performed by standard techniques using the lipfectAMINE reagent (Marals *et al* 1995). For CPG2 enzyme assay, 100µL, of culture medium was incubated in CPG2 buffer (100 mM Tris. HCl, 260 µM ZnCl₂, 50µM MTX, pH 7.3), at 25°C and the rate of change in absorbance was monitored at 320nm. From this, the CPG2 activity in terms of units CPG2 per ml of culture medium (U/ml) was calculated. | |

**TABLE 3**

| **PREDICTED AND OBSERVED SIZES OF THE EXPRESSED PROTEINS** | | | |
|---|---|---|---|
| Protein | No of aa | *M*_{*r*} (Predicted) | Apparent *M*_{*r*} (Observed) * |
| CP(Q)3-H6 | 411 | 43731 | 45000 |
| VEGF165 | 165 | 19152 | N.D. |
| V161CP(Q)3-H6 | 566 | 61710 | 60-62000 |
| V115CP(Q)3-H6 | 520 | 56386 | 54-58000 |
| CP(Q)3V165 | 574 | 62272 | 58-60000 |
| CP(Q)3V109 | 518 | 55657 | 52-55000 |
| CP(Q)3V161-H6 | 578 | 62660 | 60-62000 |
| CP(Q)3V109-H6 | 530 | 57135 | 59-61000 |
| The table represents the number of amino acids predicted in each mature fusion protein and from that the predicted molecular masses calculated; these masses therefore take into account the loss of the signal peptides. The observed masses are determined from the SDS-PAGE analysis shown in Figure 2A. | | | |

| | | | |
|---|---|---|---|
| N.D. - not determined | | | |
| * The observed sizes are calculated from the predominant band seen in each case in Figure 7A. | | | |

**TABLE 4**

| **EXPRESSION OF THE FUSION PROTEINS IN Sf9 CELLS** | |
|---|---|
| Fusion protein expressed | U/ml |
| Empty | 0 |
| CPH₆ | 0.4 |
| V₁₆₁CPCPH₆ | 0.06 |
| V₁₁₅CPH₆ | 0.2 |
| CPV₁₆₁H₆ | 0.01 |
| CPV₁₀₉H₆ | 0.04 |
| The tissue culture medium from Sf9 cells infected with viruses encoding the indicated proteins was harvested and examined for CPG2 activity. For this experiment, 10⁷ insect cells were infected at a multiplicity of infection of ∼4-10. The cells were incubated with 10 mL of tissue culture medium for 60 hours. The medium was harvested and the cells removed by centrifugation. For the MTX assays, samples of medium were incubated in CPG2 buffer (100 mM Tris. HCl, 260µM ZnCl₂, 50 µM MTX, pH 7.3), at 25°C and the rate of change in absorbance was monitored at 320 nm. From this, the CPG2 activity in terms of units CPG2 per ml of culture medium (U/ml) was calculated. | |

**TABLE 5**

| **PROTEIN PURIFICATION BY Ni++-NTA AFFINITY CHROMATOGRAPHY** | | | | |
|---|---|---|---|---|
| Fraction | CPH₆ | | V₁₁₅CPH₆ | |
| | % TOTAL PROTEIN | % CPG2 ACTIVITY * | % TOTAL PROTEIN | % CPG2 ACTIVITY * |
| LOAD | 100 | 100 | 100 | 100 |
| FLOW THROUGH | 94 | 0 | 94 | 0 |
| WASHES | 2 | 0 | 2 | 0 |
| ELUATE | 4 | 99 | 4 | 100 |
| The table shows the purification of CPH⁶ and V₁₁₅CPH₆ by NI₊₊ NTA-affinity chromatography. For each protein, 10⁷ insect cells were infected at a multiplicity of infection of 4-10. The cells were incubated with 10 mℓ of tissue culture medium for 60 hours and the medium was harvested, removing the cells by centrifugation. The condition medium was extensively dialysed against dialysis buffer (50mM Tris. HCl, 500 mM NaCl, pH8) and then loaded onto a 0.5 ml column buffer (50 mM Tris. HCl, 500 mM NaCl, 10% glycerol, 0.5% v.v NP40, 10 mM imidazole, 5Mg/ml Leupeptin, 5Mg/ml Pepstatin A, 50 mg/ml phenylmethylsulphonyl fluoride, 1mM benzamidine, pH8). The column was washed with 5ml of column buffer containing 20 mM imidazole and the bound proteins were eluted in column buffer containing 90 mM imidazole. The table shows the proportion of total protein and CPG2 activity located in each of the column fractions. | | | | |

| | | | | |
|---|---|---|---|---|
| * The CPG2 activity was determined in enzyme assay, using MTX as a substrate as described in Table 4. | | | | |

**TABLE 6**

| **KINETIC ANALYSIS OF PURIFIED PROTEINS.** | |
|---|---|
| CONSTRUCT | Kₘ (µM) |
| CPH₆ | 19.6 ± 3.2 |
| V₁₆₁CPH₆ | 18.2 ± 1.5 |
| V₁₁₅CPH₆ | 17.0 ± 1.0 |
| CPV₁₀₉H₆ | 9.1 ± 1.1 |
| Sf9 insect cells were infected with viruses expressing CPH₆, V₁₅₁CPH₆, V₁₁₅CPH₆ and CPV₁₀₉H₆. The culture medium was harvested and the fusion proteins purified by Ni++-NTA Agarose affinity chromatography as described in Table 5. The purified proteins were tested for ability to cleave MTX as described in Table 4 and standard regression analysis was used to determine the affinity (Kₘ) of each fusion for the substrate. | |

### 4. Characterisation of the Fusion Proteins

The first experiments were directed at determining whether the fusion proteins we had generated could be expressed in eukaryotic cells. We used a transient transfection system based on COS cells for this, since these cells are convenient to culture and transfect using the LipofectAMINE reagent. The genes for V₁₆₁CPH₆, V₁₁₅CPH₆, CPV₁₆₅, CPV₁₀₉ and VEGF₁₆₅. were cloned into the mammalian expression vector pEFPlink.2. These vectors are referred to as pEFV₁₆₁CPH₆, pEFV₁₁₅CPH₆, pEFCPV₁₆₅, pEFCPV₁₀₉ and pEFVEGF₁₆₅ respectively. COS cells were transfected with these plasmids or with pEFPlink.2. Since all of the proteins expressed by these clones would be expected to be secreted, the conditioned medium was collected from the transfected cells 72 hours after transfection and examined for the presence of the fusion proteins as outlined below.

### 4.1 CPG2 activity of fusion protein

First, the conditioned medium from the transfected cells was examined for the presence of CPG2 enzyme activity. Samples of culture medium were analysed for their ability to degrade the CPG2 substrate MTX. The results show that in cells transfected with pEFPlink.2, there is no detectable CPG2 activity whereas the medium from the cells transfected with pEFCPH₆ accumulated CPG2 activity to a level of 0.096 units of CPG2 per mL (U/mℓ) of medium (Table 2). The medium from the cells which were transfected with each of the fusion proteins were also found to accumulate CPG2 activity with a range of 0.004 to 0.14 U/ml culture medium. Thus it can be seen that all four fusion proteins have the ability to express CPG2 in a form that is active and which is secreted from the cells and accumulates in the culture medium. That this accumulation of CPG2 is not due to the expression of VEGF in these cells can be discounted because the medium from the cells transfected with pEFVEGF₁₆₅ do not accumulate CPG2 activity (Table 2).

### 4.2 SDS-analysis if fusion proteins

Although the results shown in section 4.1 indicate that CPG2 activity accumulates in the tissue culture medium, it does not show the presence of intact fusion proteins. In order to determine whether the secreted fusion proteins were subjected to proteolytic degredation, we examined them by sodium dodecyl sulphate-polyacrylamide gel electrophoresis (SDS-PAGE) and immunoprotein blotting. Samples of conditioned medium from each of the COS transfections were subjected to immunoprecipitation with a CPG2 specific antiserum. The immunoprecipitated proteins were resolved by SDS-PAGE and the CPG2 proteins revealed by immunoprotein blotting with the CPG2 specific antiserum.

There were no proteins which are recognised by the CPG2 specific antiserum in the conditioned medium from the cells transfected with pEFPlink.2 (Fig 2, lane 7). A protein with a Mr of ∼45,000 was recognised by the CPG2 antiserum in the medium of cells transfected with pEFCPH₆ (Fig 2, lane 1). The size of this protein is similar to the mass predicted for CPH₆ (Table 3). The culture medium from the cells transfected with pEFV₁₆₁CPH₆, pEFV₁₁₅CPH₆, pEFCPV₁₆₅ and pEFCPV₁₀₉ all accumulated proteins which were recognised by the CPG2 specific antibody and are all were larger in mass than CPH₆ (Figure 2, lanes 1, 3, 4, 5, 6). Each of the fusions exists as multiple bands on SDS-PAGE and this was attributed to variable glycosylation on the VEGF moiety. When the apparent molecular masses of the predominant bands were calculated from the SDS-gel, they were found to be close to the predicted masses expected for each fusion, when the size of the signal peptides is taken into account (Table 3). These data indicate that the fusion genes produce the expected and correct proteins. The proteins are of the correct sizes and there is no significant accumulation of free CPG2 in any of the media from the cells transfected with expression vectors encoding the fusion proteins. This indicates that the fusions are stable and not subjected to proteolytic degredation in the tissue culture medium.

### 4.3 Heparin-binding of the fusion proteins

The fusion proteins were tested for their abilities to bind to heparin with Heparin-Sepharose CL-6B (Pharmacia) which is a column matrix consisting of Sepharose beads to which heparin is covalently bound. The conditioned medium from the transfected cells was incubated with Heparin-Sepharose CL-6B and the proteins which were found to bind to the matrix were identified by immunoprotein blotting.

CPH₆ is shown by this analysis to be unable to bind to heparin (Fig 2B, lane 9). The two fusion proteins which contain the heparin binding domain of VEGF (V₁₆₁CPH₆ and CPV₁₆₅) bound to heparin with high efficiency (Fig 2B, lanes 10 and 13). In fact, when these results were quantitated it was seen that even under these crude conditions, more than 60% of V₁₆₁CPH₆ and CPV₁₆₅ were able to bind to the heparin-matrix. Weak binding of V₁₁₅CPH₆ to the heparin-matirx (Fig 7B, lane 11) but when this was quantitated, it was shown to represent less than 7% of the fusion bound to the matrix. Furthermore, it should be noted that the amount of V₁₁₅CPH₆ that was used in this analysis was far greater than either V₁₆₁CPH₆ or CPV₁₆₅ and so the figure of 7% bound probably represents an over-estimation of the true binding relative to that seen with V₁₆₁CPH₆ or CPV₁₆₅. CPV₁₀₉ was also found to bind to the heparin-matrix (Fig 2B, lane 12), but when quantiated this was found to be only ∼1.3% bound and deemed not to be significant. These results show that as predicted, when the VEGF heparin-binding domain is present in the fusion proteins, it enables them to bind to the heparin-matrix, indicating that in the context of either a N- or a C-terminal CPG2 fusion, the heparin-binding domain of VEGF functions autonomously. When this region is excluded from the fusions, then the resulting proteins show only very weak heparin binding activity.

### 4.4 Purification of the fusion proteins by Heparin-Sepharose affinity chromatography

Having shown that at least two of the fusions could bind to Heparin-Sepharose beads with high efficiency, this technique was used to purify the fusion proteins. Conditioned medium from COS cells transfected with the plasmid pEFCPV₁₆₅ was passed over a Heparin-Sepharose column. The column was washed and the bound proteins were eluted with buffer containing increasing concentrations of NaCl. The elution of the fusion protein was detected by assaying the fractions for CPG2 activity.

When cos-conditioned medium containing CPV₁₆₅ is processed over a Heparin-Sepharose column, >96% of the protein loaded does not bind. However, all of the fusion protein bound to the column, as determined by CPG2 enzyme assay. When the column was washed with buffer containing 400mM NaCl, 50% of the bound column (2% of the protein in the load sample) eluted, and this fraction did not contain any of the CPG2 activity (Fig 3). When buffer containing 800mM NaCl was applied to the column, the majority of the protein remaining (∼2% of the protein loaded) eluted from the column. This fraction was found to contain the CPG2 activity and at higher NaCl concentrations, no more CPG2 activity was eluted (Fig 3).

The apparent yield of CPG2 enzyme activity eluted from the column is ∼140%. This suggests that the activity of the purified protein is greater than that of the unpurified protein, possibly due to the presence of an inhibitory activity present in the conditioned culture medium. This makes it impossible to assess the purification fold of the sample, however, the 800mM NaCl fraction contains all of the CPG2 loaded and eluted from the column , but only ∼2% of the protein loaded. Thus, the Heparin-Sepharose column gives a > 50 fold purification, demonstrating the feasibility of this approach.

### 4.5. Expression of the fusion proteins in Sf9 insect cells

The bulk production of the fusion proteins was attempted in order to allow them to be further characterised. The insect cell system for this analysis was chosen because this system can produce high levels of foreign proteins when the cells are infected with recombinant baculoviruses. Insect cells usually allow correct formation of structures such as disulphide bonds and often perform post-translational modifications such as proteolytic cleavages and glycosylation, which occur in mammalian cells but are not seen in bacterial expression systems. Before progression to make the insect cell viruses, however, polyhistidine tags onto the C-termini of CPV₁₆₅ and CPV₁₀₉ were incorporated to allow these proteins to be purified by Ni++-NTA-Agarose affinity column chromatography.

### 4.5.1 Cloning the baculovirus vectors

A polyhistidine tag was inserted on the 3' ends of the genes for CPV₁₆₅ and CPV₁₀₉.

### 4.5.1.1 CPV₁₆₅H₆

The cloning of CPV₁₆₅H₆ was performed by using oligonucleotides 4 and 8 in a PCR reaction with pVEGF₁₆₅. The PCR product was digested with the restriction endonucleases KpnI and BamHI and this fragment was cloned back into pEFCPV₁₆₅ but in conjunction with oligonucleotides 10 and 11. to generate the polyhistidine tag at the 3' end of the gene. This cloning strategy results in the addition of the polyhistidine tag to the 3' end of the VEGF moiety of the fusion gene and therefore to the C-terminus of the protein. In constructing this gene, the terminal four codons of the VEGF gene were removed. This construct is referred to as CPV₁₆₁H₆ and the protein is represented schematically in Fig 1G. The sequence of the CPV₁₆₁H₆ gene and the protein that it is predicted to encode is shown in SEQ ID No. 16.

### 4.5.1.2 CPV₁₀₉H₆

The cloning of CPV₁₀₉H₆ was performed by using oligonucleotides 8 and 12 in a PCR reaction with pVEGF₁₆₅. The PCR product was digested with the restriction endonucleases KpnI and BamHI and this fragment was then cloned back into pEFCPV₁₆₅ but in conjunction with oligonucleotides 10 and 11 to generate the polyhistidine tag at the 3' end of the gene. This cloning strategy results in truncation of the VEGF gene at codon 110 the addition of the polyhistidine tag to that position. Thus the residues required for heparin-binding are removed. This construct is referred to as CPV₁₀₉H₆ and the protein is represented schematically in Fig 1H. The sequence of the CPV₁₀₉H₆ gene and the protein that it is predicted to encode is shown in SEQ ID No. 18.

The genes for CPH₆, V₁₆₁CPH₆, V₁₁₅CPH₆, CPV₁₆₅H₆ and CPV₁₀₉H₆ were cloned into the insect cell shuttle vector pVLPlink.2. These vectors were used to produce recombinant Baculovirus particles following standard protocols.

### 4.5.2 Expression of CPH₆ and the fusion proteins in SF9 cells

Sf9 insect cells were infected with recombinant viruses directing expression of CPH₆, V₁₆₁CPH₆, V₁₁₅CPH₆, CPV₁₆₅H₆ and CPV₁₀₉H₆. Control infections were also performed using a virus which has an empty polyhedron locus (the 'empty' virus). Since these cells are eukaryotic, all the proteins were expected to be secreted into the medium in which the cells were growing. We therefore examined the conditioned medium from the infected cells for the presence of CPG2 enzyme activity using MTX as a substrate.

Conditioned medium from cells infected with the empty virus are unable to degrade MTX (Table 4). The conditioned medium from the cells infected with the empty virus was unable to degrade MTX, indicating the absence of any CPG2 acitivy in infected insect cells. By contrast, the medium from the cells which were infected with the virus which expresses CPH₆ accumulated CPG2 protein to a level of ∼0.4 units of CPG2 per mℓ (U/mℓ) of tissue culture medium (Table 4). The medium from the cells infected with viruses with each of the other fusion proteins also accumulated CPG2 activity to between 0.01 and 0.2 U/mℓ (Table 4), indicating the secretion of the fusion proteins.

### 4.6 Ni++-Agarose Affinity chromatography of the fusion proteins

We used the insect cell produced material to determine whether the polyhistidine tags could be used to purify the expressed proteins. For this analysis, we used CPH₆ and V₁₁₅CPH₆. Sf9 cells were infected with the appropriate viruses and the proteins secreted into the conditioned tissue culture medium were subjected to a purification protocol using Ni++-NTA-Agarose (Quiagen: used according to manufacturer's instructions). The conditioned media were dialysed to remove histidine present in the medium (which may interfere with binding of H6-proteins because histidine has an imidazole ring) and then passed over the column. The column was washed and the bound proteins were eluted with imidazole. Only 6% of the protein in the conditioned medium bound to the Ni++-NTA column, and ∼33% of the bound protein was eluted from the column in the wash cycles (Table 5). When the flow through and wash fractions were assayed for CPG2 activity, none could be detected in these fractions. In the case of both CPH₆ and V₁₁₅CPH₆, the amount of protein that eluted from the 90mM imidazole wash represented ∼4% of the protein loaded onto the column (Table 5). When this was assayed, it was found to contain ∼99% of the CPG2 activity loaded onto the column in the case of CPH₆ and ∼100% of the CPG2 activity loaded onto the column in the case of V₁₁₅CPH₆. Thus, the recovery from these columns is excellent.

In order to determine the purity of the eluted protein, the samples were subjected to SDS-PAGE and silver staining. The results show that both the prepareations of CPH₆ and V₁₁₅CPH₆ were extremely pure, representing >95% of the proteins in the purified samples (Fig 4). This demonstrates the feasibility of using this approach to purify the polyhistidine tagged proteins and also underscores the fact that the insect cells can produce large amounts of the fusion proteins which are secreted and accumulate to represent ∼4% of the protein present in the medium and hence the fold purification required to prepare pure samples is only ∼25.

Using this protocol, CPH₆, V₁₆₁CPH₆, V₁₁₅CPH₆ and CPV₁₀₉H₆ were all purified from Sf9 cells by Ni++-NTA-Agarose affinity chromatography as described above. These samples were used to determine the affinity of the CPG2 moiety for the substrate MTX. The results show that the Km of all of the fusion proteins is similar to that seen for CPH₆ (Table 6), which is similar to the Km determined for bacterially produced CPG2 of ∼10µM . This indicates that fusion of CPG2 to VEGF does not impair the affinity of CPG2 for its substrate.

### 4.7 Dimer analysis of fusion proteins

VEGF produced in mammalian cells is a dimeric protein which is stabilised by inter-molecular and intra-molecular cysteine bridges. CPG2 is also a dimeric protein, but these dimers are stabilised by non-covalent interactions. In order to determine whether the VEGF/CPG2 fusion proteins which were produced in Sf9 cells are dimers stabilised by cysteine bridges, a dimer analysis was performed. CPH₆ and V₁₁₅CPH₆, both produced in insect cells were heated either in the presence or absence of the reducing agent 2-mercaptoethanol. The resultant proteins were resolved by SDS-PAGE and subjected to immunoprotein blot analysis using the CPG2 specific antiserum. The results show that following heating, either in the presence or absence of reducing agents, CPH₆ migrates as a protein with a mass of ∼45kDa (Fig 5, lanes 2, 4), showing that this protein does not exist in the form of dimers stabilised by cysteine bridges. V₁₁₅CPH₆, by contrast is seen as a protein with a Mr of ∼120 kDa in the absence of reducing agents, but as a protein with a Mr of ∼60 kDa in the presence of reducing agents (Fig 5, lanes 1, 3). These data show that V₁₁₅CPH₆ produced in Sf9 cells is a dimeric protein which is stabilised by cysteine bridges, as in mammalian cells.

### 4.8 Binding of the Fusion proteins to the VEGF receptor

We used the insect cell expressed protein to determine whether the fusion proteins could bind to the VEGF receptor by biochemical methods. For this analysis, we developed an in vitro assay in which the ability of the fusion proteins to bind to the external domain of the VEGF receptor, KDR. Receptor tyrosine kinases, of which KDR is a member, span the plasma membrane and consist of three domains. The external domain which are usually highly glycosylate contain the growth factor binding domain and thus are the sites of specific interaction with their ligands. There is then a short transmembrane domain, made of a single amino acid chain and the C-terminus which is located in the cytoplasm consists of the tyrosine kinase domain. In the assay we developed, we wished to use the extracellular domain (ECD) as a specific probe for VEGF binding. In order to achieve this, we cloned the KDR external domain and transmembrane domain for expression in SF9 cells. The internal domain was removed in this cloning and a tag for the monoclonal antibody 9E10 was added to the 3'end of the cloned fragment and this construct is referred to as the KDR binding domain (BD) (See Fig 6A). The rational for this approach was to express the KDR(BD) in Sf9 cells and prepare extracts from the infected cells. The KDR(BD) could then be immunoprecipitated with the 9E10 monoclonal antibody and used as a probe to detect binding of the VEGF/CPG2 fusions to the VEGF receptor.

### 4.8.1. Cloning of KDR BD

For this approach, the KDR gene in the plasmid pBK-CMVÆ/KDRsense was used. PCR, using oligonucleotides 13 and 14 in conjunction with the KDR gene was used to create an *Nco* 1 restriction endonuclease site at the 5' end of the gene. The KDR(BD) was then cloned as an *Nco* I/ *Eco* RI fragment into the baculovirus vector pVLPlink.2. The Eco RI site in KDR is located at codons 809 and 810 of KDR which is just on the 3' end of the sequence encoding for the transmembrane domain (See Fig 6A). A tag for the 9E10 monoclonal antibody was cloned into the *Eco* RI site using standard cloning techniques with oligonucleotides 15 and 16. The sequence of this KDR fragment, referred to as KDR(BD) is shown in SEQ ID No. 20 and it is represented shcematically in Fig 6A.

### 4.8.2. Expression of KDR BD in Sf9 cells

The expression of the KDR(BD) in insect cells was verified by immunoprotein blotting of infected insect cells using the 9E10 monoclonal antibody. The results show that in cells infected with the KDR(BD) virus there is a band with a Mr of ∼120kDa which is recognised by the 9E10 monoclonal antibody, which is not present in extracts from cells infected with the empty virus (Fig 7). The size of this band of is greater than the size expected for the cloned fragment (expected size = 92,769 Da) and this is probably due to glycosylation of KDR(BD). The data show that KDR(BD) can be expressed in Sf9 cells.

### 4.8.3. Binding of fusion proteins to the KDR(BD)

The abilities of the four VEGF/CPG2 fusion proteins to bind to KDR(BD) were tested. For this assay, extracts from insect cell that had been infected with either the KDR(BD) expressing virus, or a control (empty) virus were incubated with the 9E10 monoclonal antibody immobilised on Protein G-Sepharose beads, to precipitate the KDR(BD). The immunocomplexes were washed and then incubated with CPH₆, V₁₆₁CPH₆, V₁₁₅CPH₆, CPV₁₆₁H₆ and CPV₁₀₉H₆ protein which had been expressed in insect cells and purified by Ni++-NTA Affinity chromatography. The complexes were washed once more and the presence of the fusion proteins judged measuring CPG2 activity in the immunocomplexes.

CPH₆, was found not to interact with the KDR(BD), since no CPG2 activity is found in KDR(BD)/9E10 immunocomplexes (Fig 8, lane 6). However, V₁₆₁CPH₆, V₁₁₅CPH₆, CPV₁₆₁H₆ and CPV₁₀₉H₆ were all able to bind to the KDR(BD) as seen by the fact that with each of these fusions, CPG2 acitivty could be detected associated with the KDR(BD)/9E10 immunocomplex (Fig 8, lanes 7-10). That this interaction was specific could be shown by the fact that when the 9E10 immunocomplexes were performed with extracts from Sf9 cells infected with empty virus instead of the KDR(BD) expressing virus, no binding of the fusion proteins could be detected. (Fig 15, lanes 2-5) These data establish that all four of the fusion proteins can interact specifically with the ligand binding domain of KDR, whereas CPH₆ cannot.

### 5. CELL CYTOTOXICITY ASSAYS

Taken together, the above data establish that fusion proteins can be constructed between CPG2 and VEGF in two orientations and with VEGF moities that do, or do not, contain the heparin-binding domain of VEGF. These proteins are secreted by eukaryotic cells and are stable in terms of VEGF functions (receptor binding and where appropriate, heparin-binding) and in terms of CPG2 enzyme activity. In order to prevent glycosylation of the CPG2 moiety, the sites of inappropriate glycosylation on CPG2 were mutated. This results in a protein with only ∼10% enzyme activity compared to the wild type protein, but its affinity for its substrate is indistinguishable from bacterially expressed wild type CPG2 (see WO96/03515). The presence of a polyhistidine tag at the C-terminus of the fusions allows the purification of the fusions by Ni++-NTA Agarose chromatography and the fusions which contain the heparin-binding domain of the VEGF moiety can be purified on Heparin-Sepharose columns. We therefore progressed to an analysis of the ability of the fusion proteins to direct prodrug dependent cytotoxicity of mammalian cells.

### 5.1 Non-specific cytotoxicity assay

For the first assay, we wished to establish that CPH6 and the fusion proteins were able to direct prodrug dependent cyotoxicity of NIH3T3 cells, in a non-targeted manner. For this experiment, CPH₆, V₁₆₁CPH₆, V₁₁₅CPH₆, CPV₁₆₅H₆ and CPV₁₀₉H₆ were all purified by Ni++-NTA Agarose. Samples of the purified proteins were added to NIH3T3 cells in the presence of the CMDA prodrug and the effects on cell viability were determined by measuring cell growth by [3H]-thymidine incorporation.

Treatment of these cells with CMDA alone does not significantly affect the growth of these cells (Fig 9, lanes 1, 2). When these cells are treated with sub-nanomolar concentrations of CPH₆ or each of the fusion proteins in the presence of CMDA, complete cell death occurs (Fig 9, lanes 3-7). Thus, it can be seen that each of the fusion proteins is highly efficient in directing the specific killing of mammalian cells in the presence of the CMDA prodrug.

### 5.2 VEGF-directed cytotoxicity assay

The fusion proteins were assesed for their ability to direct the CMDA-dependent killing of cells which express specific receptors for VEGF on their surface. For this analysis, we employed human umbilical vein endothelial (Hu-V-ec) cells, which are dependent for growth on VEGF (Prog Growth Factor Res, 5, 89, 1994). These cells are not significantly sensitive to CMDA as judged by the effects of 1mM CMDA on their growth rate (Fig 10A). The cells were incubated in the presence of CPH₆, or V₁₁₅CPH₆ for 30 min to allow the fusion proteins to bind to the cell surface and then the cells were washed to remove unbound protein. CMDA was added and cell survival was determined by [3H]-thymidine incorporation.

When the analysis is performed with CPH₆, there is no significant affect on the growth rate of the cells (Fig 10B, lanes 1, 2). By contrast, when the analysis is performed with V₁₁₅CPH₆, there is a >90% inhibition of cell growth (Fig 10B, lanes 1, 3). These data show that CPH₆ does not appear to bind to the surface of Hu-V-ec cells and so is unable to direct their killing in the presence of the prodrug, as it is washed off the cells prior to the addition of the prodrug. However, V₁₁₅CPH₆ remains bound to the cell surfaces, is not washed off the cells and so is able to direct their killing when the prodrug is added. This provides a proof of the feasability of the LIDEPT approach.

### SEQUENCE INFORMATION

In the sequences SEQ ID NOS. 1 to 20, the DNA sequences are presented in the conventional 5' -> 3' orientation and the protein sequences in the conventional N- -> C-terminal orientation as indicated.

### SEQ ID Nos. 3 and 4 :DNA and Protein Sequences of CPG2

The DNA sequence is presented and below it, the predicted protein sequence. Within the DNA sequence, the engineered restriction endonuclease sites are shown in lower case. The position of the Nco 1 site at the 5' end of the gene is also indicated. Four mutations within the gene are indicated by *. The mutation at *1 represents a silent mutation at the Asn codon at position 172 of CPG2 which occurred during the PCR cloning of this gene. The mutations at *2, *3 and *4 are the Asn -> Gln mutations required to prevent glycosylation of CPG2 in mammalian cells. Numbers below the protein sequence indicate the codons of the genes from which the individual fragments are derived. The region derived from c-erbB2 is underlined and this contains codons 1 to 17 of that gene, which encompasses the signal peptide used to direct this protein into the secretory pathway. The region derived from CPG2 contains codons 23 to 415. The polyhistidine tag at the C-terminus of the clone is shown in italics within the protein sequence and the stop codon is indicated by ●.

### SEQ ID Nos 5 and 6 : DNA and Protein Sequences of VEGF.

The DNA sequence is presented and below it, the predicted protein sequence. Within the protein sequence, the signal peptide which is proteolytically cleaved from the mature protein is underlined. The amino-acid numbering therefore begins with residue -26 to indicate that fact, and residue 1 is the first amino acid in the mature protein. The portion of the single glycosylation site is indicated by *. The stop codon is indicated by ●.

In the fusion protein sequences the DNA sequence is presented and below it, the predicted protein sequence. The VEGF derived sequences are shown in bold type, and the CPG2 derived sequences in normal type. The two DNA in SEQ ID Nos. 7, 9, 11 and 13 sequences are taken from SEQ ID Nos. 3 and 5.

### SEQ ID Nos. 7 and 8 : DNA and Protein Sequences of V₁₆₁CPH₆

A silent mutation within the VEGF sequence at codon 2 (CCC->CCT) of the mature protein was created to destroy the *Nco* I site located in this gene (*). The engineered restriction endonuclease sites are shown in lower case. The numbers below the protein sequence indicate the codons for the genes from which the individual fragments are derived. The VEGF sequences are from -26 to 161 and the CPG2 sequences from 23 to 415. The signal peptide from VEGF which is cleaved proteolytically from the mature protein is underlined. The polyhistidine tag is shown in italics within the protein sequence and the stop codon indicated by ●.

### SEQ ID Nos. 9 and 10 : DNA and Protein Sequences of V₁₁₅CPH₆

A silent mutation within the VEGF sequence at codon 2 (CCC->CCT) of the mature protein was created to destroy the *Nco* I site located in this gene (*). Additionally, the VEGF gene has been truncated at codon 115 as indicated by the numbering below the protein sequence. The engineered restriction endonuclease sites are shown in lower case. The numbers below the protein sequence indicate the codons for the genes from which the individual fragments are derived. The VEGF sequences are from -26 to 115 and the CPG2 sequences from 23 to 415. This fusion therefore does not contain the amino acids responsible for heparin-binding of VEGF. The signal peptide from VEGF which is cleaved proteolytically from the mature protein is underlined. The polyhistidine tag is shown in italics within the protein sequence and the stop codon indicated by •.

### SEQ ID Nos. 11 and 12 : DNA and Protein Sequences of CPV₁₆₅

A peptide spacer was generated by additional DNA sequences between the two, encompassed by the *Eco* RI and *Kpn* 1 sites. The spacer peptide which these encode are indicated in italics and underlined, within the protein sequence. The cloning of this construct resulted in the loss of the polyhistidine tag from the 3' end of the CPG2 derived sequences and also in the removal of the sequences for the VEGF signal peptide. At the 5' end of the gene are the first 27 codons of c-erbB2, followed by CPG2 amino acids 23-415, fused via the peptide spacer to VEGF amino acids 1-165. The stop codon is indicated by ●.

### SEQ ID Nos 13 and 14 : DNA and Protein Sequences of CPV₁₀₉

A peptide spacer was generated by additional DNA sequences between the two, encompassed by the *Eco* RI and *Kpn* 1 sites. The spacer peptide which these encode are indicated in italics and underlined, within the protein sequence. The cloning of this construct resulted in the loss of the polyhistidine tag from the 3' end of the CPG2 derived sequences and also in the removal of the sequences for the VEGF signal peptide. This clone was created when, a PCR generated mutation occurred during the creation of CPV165. This converts the Arg codon 110 of mature VEGF into a stop codon, resulting in truncation of the VEGF fraction at codon 109 as indicated in the protein sequence derived from this clone (*). At the 5' end of the gene are the first 27 codons of c-erbB2, followed by CPG2 amino acids 23-415, fused via the peptide spacer to VEGF amino acids 1-109. The mutation resulting in the stop codon is indicated by ●.

### SEQ ID Nos. 15 and 16 : DNA and Protein Sequences of CPV₁₆₅H₆

The DNA sequences of VEGF and CPG2 are taken from SEQ ID No. 11. A peptide spacer was generated by additional DNA sequences between the two, encompassed by the *Eco* RI and *Kpn* 1 sites. The spacer peptide encode is indicated in italics and underlined, within the protein sequence. In this clone, a polyhistidine tag has been included in the gene at the position 3' to codon 161 of mature VEGF by creating a *Bam* HI site at codon 162 of mature VEGF as shown. The polyhistidine tag was then cloned into this position and this encodes for a C-terminal protein tag as shown in italics at the end of the protein sequence. During the cloning, for convenience, an *Eco* RI site was created 3' to the *Bam* HI site as indicated. At the 5' end of the gene are the first 27 codons of c-erbB2, followed by CPG2 amino acids 23-415, fused via the peptide spacer to VEGF amino acids 1-161 and then the polyhistidine tag. The stop codon is indicated by ●.

### SEQ ID Nos. 17 and 18 : DNA and Protein Sequences of CPV₁₀₉H₆

The two DNA sequences of VEGF and CPG2 are taken from SEQ ID No 13. A peptide spacer was generated by additional DNA sequences between the two, encompassed by the *Eco* RI and *Kpn* 1 sites. The spacer peptide which these encode is indicated in italics and underlined, within the protein sequence. In this clone, a polyhistidine tag has been included in the gene at the position 3' to codon 109 of mature VEGF by creating a *Bam* HI site at codon 110 of mature VEGF as shown. The polyhistidine tag was then cloned into this position and this encodes for a C-terminal protein tag as shown in italics at the end of the protein sequence. During the cloning, for convenience, an *Eco* RI site was created 3' to the *Bam* HI site as indicated. At the 5' end of the gene are the first 27 codons of c-erbB2, followed by CPG2 amino acids 23-415, fused via the peptide spacer to VEGF amino acids 1-109 and then the polyhistidine tag. The stop codon is indicated by ●.

### SEQ ID NOS. 19 AND 20 : DNA AND PROTEIN SEQUENCE OF KDR(BD)

The position of the engineered *Nco* 1 site at the 5' end and the endogenous *Eco* RI site at codons 809 and 810 are indicated. The signal peptide is indicated in the protein sequence as the underlined region. The transmembrane region, located between codons 746 to 770 and the protein sequence is underlined and in italics. The DNA sequence used to encode the C-terminal 9E10 tag is shown in lower case and the protein sequence it encodes is in italics. This region encodes for amino acids 1-810 of KDR fused to the 9E10 epitope. The stop codon indicated by ●.

SEQUENCE INFORMATION

Sequences of
CPG 2

SEQ ID NO. 1: Genomic DNA
SEQ ID NO. 2: Protein Sequence

ATCATGGATC CACGCACTGA AGGCGCGCGG CAAGACGCGC GGCGTGGCGA CGCTGTGCAT 60

CGGCGGGGGC GAAGGCACCG CAGTGGCACT CGAATTGCTA TAAGAACCAT GGCTGGGGAC 120

GCCCGACAAC AGGCGTCCAC CAGCTTTTTT CATTCCGACA ACCCGAACGA ACAATGCGTA 180

GAGCAGGAGA TTCC ATG CGC CCA TCC ATC CAC CGC ACA GCC ATC GCC GCC 230
Met Arg Pro Ser Ile His Arg Thr Ala Ile Ala Ala
1               5                   10

GTG CTG GCC ACC GCC TTC GTG GCG GGC ACC GCC CTG GCC CAG AAG CGC 278
Val Leu Ala Thr Ala Phe Val Ala Gly Thr Ala Leu Ala Gln Lys Arg
        15                  20                  25

GAC AAC GTG CTG TTC CAG GCA GCT ACC GAC GAG CAG CCG GCC GTG ATC 326
Asp Asn Val Leu Phe Gln Ala Ala Thr Asp Glu Gln Pro Ala Val Ile
    30                  35                  40

AAG ACG CTG GAG AAG CTG GTC AAC ATC GAG ACC GGC ACC GGT GAC GCC 374
Lys Thr Leu Glu Lys Leu Val Asn Ile Glu Thr Gly Thr Gly Asp Ala
45                  50                  55                  60

GAG GGC ATC GCC GCT GCG GGC AAC TTC CTC GAG GCC GAG CTC AAG AAC 422
Glu Gly Ile Ala Ala Ala Gly Asn Phe Leu Glu Ala Glu Leu Lys Asn
                65                  70                  75

CTC GGC TTC ACG GTC ACG CGA AGC AAG TCG GCC GGC CTG GTG GTG GGC 470
Leu Gly Phe Thr Val Thr Arg Ser Lys Ser Ala Gly Leu Val Val Gly
            80                  85                  90

GAC AAC ATC GTG GGC AAG ATC AAG GGC CGC GGC GGC AAG AAC CTG CTG 518
Asp Asn Ile Val Gly Lys Ile Lys Gly Arg Gly Gly Lys Asn Leu Leu
        95                  100                 105

CTG ATG TCG CAC ATG GAC ACC GTC TAC CTC AAG GGC ATT CTC GCG AAG 566
Leu Met Ser His Met Asp Thr Val Tyr Leu Lys Gly Ile Leu Ala Lys
    110                 115                 120

GCC CCG TTC CGC GTC GAA GGC GAC AAG GCC TAC GGC CCG GGC ATC GCC 614
Ala Pro Phe Arg Val Glu Gly Asp Lys Ala Tyr Gly Pro Gly Ile Ala
125                 130                 135                 140

GAC GAC AAG GGC GGC AAC GCG GTC ATC CTG CAC ACG CTC AAG CTG CTG 662
Asp Asp Lys Gly Gly Asn Ala Val Ile Leu His Thr Leu Lys Leu Leu
                145                 150                 155

AAG GAA TAC GGC GTG CGC GAC TAC GGC ACC ATC ACC GTG CTG TTC AAC 710
Lys Glu Tyr Gly Val Arg Asp Tyr Gly Thr Ile Thr Val Leu Phe Asn
            160                 165                 170

ACC GAC GAG GAA AAG GGT TCC TTC GGC TCG CGC GAC CTG ATC CAG GAA 758
Thr Asp Glu Glu Lys Gly Ser Phe Gly Ser Arg Asp Leu Ile Gln Glu
        175                 180                 185

GAA GCC AAG CTG GCC GAC TAC GTG CTC TCC TTC GAG CCC ACC AGC GCA 806
Glu Ala Lys Leu Ala Asp Tyr Val Leu Ser Phe Glu Pro Thr Ser Ala
    190                 195                 200

GGC GAC GAA AAA CTC TCG CTG GGC ACC TCG GGC ATC GCC TAC GTG CAG 854
Gly Asp Glu Lys Leu Ser Leu Gly Thr Ser Gly Ile Ala Tyr Val Gln
205                 210                 215                 220

GTC AAC ATC ACC GGC AAG GCC TCG CAT GCC GGC GCC GCG CCC GAG CTG 902
Val Asn Ile Thr Gly Lys Ala Ser His Ala Gly Ala Ala Pro Glu Leu
                225                 230                 235

GGC GTG AAC GCG CTG GTC GAG GCT TCC GAC CTC GTG CTG CGC ACG ATG 950
Gly Val Asn Ala Leu Val Glu Ala Ser Asp Leu Val Leu Arg Thr Met
            240                 245                 250

AAC ATC GAC GAC AAG GCG AAG AAC CTG CGC TTC AAC TGG ACC ATC GCC 998
Asn Ile Asp Asp Lys Ala Lys Asn Leu Arg Phe Asn Trp Thr Ile Ala
        255                 260                 265

AAG GCC GGC AAC GTC TCG AAC ATC ATC CCC GCC AGC GCC ACG CTG AAC 1046
Lys Ala Gly Asn Val Ser Asn Ile Ile Pro Ala Ser Ala Thr Leu Asn
    270                 275                 280

GCC GAC GTG CGC TAC GCG CGC AAC GAG GAC TTC GAC GCC GCC ATG AAG 1094
Ala Asp Val Arg Tyr Ala Arg Asn Glu Asp Phe Asp Ala Ala Met Lys
285                 290                 295                 300

ACG CTG GAA GAG CGC GCG CAG CAG AAG AAG CTG CCC GAG GCC GAC GTG 1142
Thr Leu Glu Glu Arg Ala Gln Gln Lys Lys Leu Pro Glu Ala Asp Val
                305                 310                 315

AAG GTG ATC GTC ACG CGC GGC CGC CCG GCC TTC AAT GCC GGC GAA GGC 1190
Lys Val Ile Val Thr Arg Gly Arg Pro Ala Phe Asn Ala Gly Glu Gly
            320                 325                 330

GGC AAG AAG CTG GTC GAC AAG GCG GTG GCC TAC TAC AAG GAA GCC GGC 1238
Gly Lys Lys Leu Val Asp Lys Ala Val Ala Tyr Tyr Lys Glu Ala Gly
        335                 340                 345

GGC ACG CTG GGC GTG GAA GAG CGC ACC GGC GGC GGC ACC GAC GCG GCC 1286
Gly Thr Leu Gly Val Glu Glu Arg Thr Gly Gly Gly Thr Asp Ala Ala
    350                 355                 360

TAC GCC GCG CTC TCA GGC AAG CCA GTG ATC GAG AGC CTG GGC CTG CCG 1334

Tyr Ala Ala Leu Ser Gly Lys Pro Val Ile Glu Ser Leu Gly Leu Pro
365                 370                 375                 380

GGC TTC GGC TAC CAC AGC GAC AAG GCC GAG TAC GTG GAC ATC AGC GCG     1382
Gly Phe Gly Tyr His Ser Asp Lys Ala Glu Tyr Val Asp Ile Ser Ala
                385                 390                 395

ATT CCG CGC CGC CTG TAC ATG GCT GCG CGC CTG ATC ATG GAT CTG GGC     1430
Ile Pro Arg Arg Leu Tyr Met Ala Ala Arg Leu Ile Met Asp Leu Gly
            400                 405                 410

GCC GGC AAG T GAATGCTGCC CCCCGGCTTT TCACTCGCGT TGCTCGTGTA     1480
Ala Gly Lys
        415

ACTCCACCCC CCGAGGGGGA GGCGCGGTCC GCCTTGGGGC GGCCCGGCGG CGACCGCCTC     1540

GTCACATAGA AGGAACTGCC ATGTTGTTGA CAGCAGACCA GGAAGCCATC CGCGACGCGG     1600

TGCGCGACTT CTCGCAAGCC GAACTCTGGC CCAACGCCGC GAATGGGGAC CGCGAGCACA     1660

GCTTTCCCAA GAGCCCACCA GGCCGTCGGC TGGCGTACGC AGTCTGCGTG CCCGAGGAGC     1720

ATGGCGGCGC CGGCCTCGAC TACCTCACCT CGCGCTGGTG CTGGAGGAGA TCGCGGCCGG     1780

CGACGGCGGC ACCAGCACCG CCATCAGCGT GACCAACTGC CCCGTCAACG CCATCCTCAT     1840

GCGCTACGGC AACGCGCAGC AGAAGAAGCA GTGGCTCGAG CCGCTGGCGC AGGGCCGGAT     1900

GCTCGGCGCC TTCTGCCTGA CCGAGCCGCA GGCCGGCAGC GATGCATCGA GCCTGCGCAC     1960

CACGGCGCGC AAGGACGGCG ACGGCTACGT GATCGACGGC GTGAAGCAGT TCATCACCAG     2020

CGGCAAGAAC GGCCAGGTGG CGGGATCC                                        2048

Sequences of CPH6

SEQ ID NO.3: DNA Sequence
SEQ ID NO.4: Protein Sequence

Nco 1
5' CCATGGAGCTGGCGGCCTTGTGC
N- MetGluLeuAlaAlaLeuCys
1

CGCTGGGGGCTCCTCCTCGCCCTCTTGCCC CCCGGAGCCGCGAGCACCCAAGTGTGCACC
ArgTrpGlyLeuLeuLeuAlaLeuLeuPro ProGlyAlaAlaSerThrGlnValCysThr
27

Bam HI
ggatccGCCCTGGCCCAGAAGCGCGACAAC GTGCTGTTCCAGGCAGCTACCGACGAGCAG
GlySerAlaLeuAlaGlnLysArgAspAsn ValLeuPheGlnAlaAlaThrAspGluGln
23

CCGGCCGTGATCAAGACGCTGGAGAAGCTG GTCAACATCGAGACCGGCACCGGTGACGCC
ProAlaValIleLysThrLeuGluLysLeu ValAsnIleGluThrGlyThrGlyAspAla

GAGGGCATCGCCGCTGCGGGCAACTTCCTC GAGGCCGAGCTCAAGAACCTCGGCTTCACG
GluGlyIleAlaAlaAlaGlyAsnPheLeu GluAlaGluLeuLysAsnLeuGlyPheThr

GTCACGCGAAGCAAGTCGGCCGGCCTGGTG GTGGGCGACAACATCGTGGGCAAGATCAAG
ValThrArgSerLysSerAlaGlyLeuVal ValGlyAspAsnIleValGlyLysIleLys

GGCCGCGGCGGCAAGAACCTGCTGCTGATG TCGCACATGGACACCGTCTACCTCAAGGGC
GlyArgGlyGlyLysAsnLeuLeuLeuMet SerHisMetAspThrValTyrLeuLysGly

ATTCTCGCGAAGGCCCCGTTCCGCGTCGAA GGCGACAAGGCCTACGGCCCGGGCATCGCC
IleLeuAlaLysAlaProPheArgValGlu GlyAspLysAlaTyrGlyProGlyIleAla

GACGACAAGGGCGGCAACGCGGTCATCCTG CACACGCTCAAGCTGCTGAAGGAATACGGC
AspAspLysGlyGlyAsnAlaValIleLeu HisThrLeuLysLeuLeuLysGluTyrGly

*1
GTGCGCGACTACGGCACCATCACCGTGCTG TTCAATACCGACGAGGAAAAGGGTTCCTTC
ValArgAspTyrGlyThrIleThrValLeu PheAsnThrAspGluGluLysGlySerPhe

GGCTCGCGCGACCTGATCCAGGAAGAAGCC AAGCTGGCCGACTACGTGCTCTCCTTCGAG
GlySerArgAspLeuIleGlnGluGluAla LysLeuAlaAspTyrValLeuSerPheGlu

CCCACCAGCGCAGGCGACGAAAAACTCTCG CTGGGCACCTCGGGCATCGCCTACGTGCAG
ProThrSerAlaGlyAspGluLysLeuSer LeuGlyThrSerGlyIleAlaTyrValGln

*2
GTCCAAATCACCGGCAAGGCCTCGCATGCC GGCGCCGCGCCCGAGCTGGGCGTGAACGCG
ValGlnIleThrGlyLysAlaSerHisAla GlyAlaAlaProGluLeuGlyValAsnAla

CTGGTCGAGGCTTCCGACCTCGTGCTGCGC ACGATGAACATCGACGACAAGGCGAAGAAC
LeuValGluAlaSerAspLeuValLeuArg ThrMetAsnIleAspAspLysAlaLysAsn

*3 *4
CTGCGCTTCCAATGGACCATCGCCAAGGCC GGCCAAGTCTCGAACATCATCCCCGCCAGC
LeuArgPheGlnTrpThrIleAlaLysAla GlyGlnValSerAsnIleIleProAlaSer

GCCACGCTGAACGCCGACGTGCGCTACGCG CGCAACGAGGACTTCGACGCCGCCATGAAG
AlaThrLeuAsnAlaAspValArgTyrAla ArgAsnGluAspPheAspAlaAlaMetLys

ACGCTGGAAGAGCGCGCGCAGCAGAAGAAG CTGCCCGAGGCCGACGTGAAGGTGATCGTC
ThrLeuGluGluArgAlaGlnGlnLysLys LeuProGluAlaAspValLysValIleVal

ACGCGCGGCCGCCCGGCCTTCAATGCCGGC GAAGGCGGCAAGAAGCTGGTCGACAAGGCG
ThrArgGlyArgProAlaPheAsnAlaGly GluGlyGlyLysLysLeuValAspLysAla

GTGGCCTACTACAAGGAAGCCGGCGGCACG CTGGGCGTGGAAGAGCGCACCGGCGGCGGC
ValAlaTyrTyrLysGluAlaGlyGlyThr LeuGlyValGluGluArgThrGlyGlyGly

ACCGACGCGGCCTACGCCGCGCTCTCAGGC AAGCCAGTGATCGAGAGCCTGGGCCTGCCG
ThrAspAlaAlaTyrAlaAlaLeuSerGly LysProValIleGluSerLeuGlyLeuPro

GGCTTCGGCTACCACAGCGACAAGGCCGAG TACGTGGACATCAGCGCGATTCCGCGCCGC
GlyPheGlyTyrHisSerAspLysAlaGlu TyrValAspIleSerAlaIleProArgArg

Eco RI
CTGTACATGGCTGCGCGCCTGATCATGGAT CTGGGCGCCGGCAAGgaattcCATCATCAC
LeuTyrMetAlaAlaArgLeuIleMetAsp LeuGlyAlaGlyLysGluPheHisHisHis
415

XbaI
CACCATCACGCTTCCTAGtctaga 3'
HisHisHisAlaSer • -C

SEQUENCES OF VEGF165.
SEQ ID NO.5 : DNA SEQUENCE
SEQ ID NO.6 : PROTEIN SEQUENCE
5'ATGGACTTTCTGCTGTCTTGGGTGCATTGGAGCCTTGCCTTGCTGCTCTACCTCCACCAT
N-MetAspPheLeuLeuSerTrpValHIaTrpSerLeuAlaLeuLeuLeuTyrLeuHisHis
-26
Nco 1
GCCAAGTGGTCCCAGGCTGCACCCATGGCA GAAGGAGGAGGGCAGAATCATCACGAAGTG
AlaLysTrpSerGlnAlaAlaProMetAla AspGlyGlyGlyGlnAsnHisHisGluVal
-1 1
GTGAAGTTCATGGATGTCTATCAGCGCAGC TACTGCCATCCAATCGAGACCCTGGTGGAC
ValLysPheMetAspValTyrGlnArgSer TyrCysHisProIleGluThrLeuValAsp
ATCTTCCAGGAGTACCCTGATGAGATCGAG TACATCTTCAAGCCATCCTGTGTGCCCCTG
IlePheGlnGluTyrProAspGluIleGlu TyrIlePheLysProSerCysValProLeu
ATGCGATGCGGGGGCTGCTGCAATGACGAG GGCCTGGAGTGTGTGCCCACTGAGGAGTCC
MetArgCysGluGluCysCysAsnAspGlu GlyLeuAspCysValProThrGluGluSer
AACATCACCATGCAGATTATGCGGATCAAA CCTCACCAAGGCCAGCACATAGGAGAGATG
AsnIleThrMetGlnIleMetArgIleLys ProHisGlnGlyGlnHisIleGlyGluMet
*
AGCTTCCTACAGCACAACAAATGTGAATGC AGACCAAAGAAAGATAGAGCAAGACAAGAA
SerPheLeuGlnHisAsnLysCysGluCys ArgProLysLysAspArgAlaArgGlnGlu
AATCCCTGTGGGCCTTGCTCAGAGCGGAGA AAGCATTTGTTTGTACAAGATCCGCAGACG
AsnProCysGlyProCysSerGluArgArg LysHisLeuPheValGlnAspProGlnThr
TGTAAATGTTCCTGCAAAAACACAGACTCG CGTTGCAAGGCGAGGCAGCTTGAGTTAAAC
CysLysCysSerCysLysAsnThrAspSer ArgCysLysAlaArgGlnLeuGluLeuAsn
GAACGTACTTGCAGATGTGACAAGCCGAGG CGGTGA3'
GluArgThrCysArgCysAspLysProArg Arg • -C
165

SEQUENCES OF V161CPH6.
SEQ ID NO.7 : DNA SEQUENCE
SEQ ID NO.8 : PROTEIN SEQUENCE
NcoI
5' ccATGGACTTTCTGCTGTCTTGG
N- MetAspPheLeuLeuSerTrp

GTGCATTGGAGCCTTGCCTTGCTGCTCTAC CTCCACCATGCCAAGTGGTCCCAGGCTGCA
ValHIaTrpSerLeuAlaLeuLeuLeuTyr LeuHisHisAlaLysTrpSerGlnAlaAla
1
*
CCTATGGCAGAAGGAGGAGGGCAGAATCAT CACGAAGTGGTGAAGTTCATGGATGTCTAT
ProMetAlaAspGlyGlyGlyGlnAsnHis HisGluValValLysPheMetAspValTyr

CAGCGCAGCTACTGCCATCCAATCGAGACC CTGGTGGACATCTTCCAGGAGTACCCTGAT
GlnArgSerTyrCysHisProIleGluThr LeuValAspIlePheGlnGluTyrProAsp

GAGATCGAGTACATCTTCAAGCCATCCTGT GTGCCCCTGATGCGATGCGGGGGCTGCTGC
GluIleGluTyrIlePheLysProSerCys ValProLeuMetArgCysGluGluCysCys

AATGACGAGGGCCTGGAGTGTGTGCCCACT GAGGAGTCCAACATCACCATGCAGATTATG
AsnAspGluGlyLeuAspCysValProThr GluGluSerAsnIleThrMetGlnIleMet

CGGATCAAACCTCACCAAGGCCAGCACATA GGAGAGATGAGCTTCCTACAGCACAACAAA
ArgIleLysProHisGlnGlyGlnHisIle GlyGluMetSerPheLeuGlnHisAsnLys

TGTGAATGCAGACCAAAGAAAGATAGAGCA AGACAAGAAAATCCCTGTGGGCCTTGCTCA
CysGluCysArgProLysLysAspArgAla ArgGlnGluAsnProCysGlyProCysSer

GAGCGGAGAAAGCATTTGTTTGTACAAGAT CCGCAGACGTGTAAATGTTCCTGCAAAAAC
GluArgArgLysHisLeuPheValGlnAsp ProGlnThrCysLysCysSerCysLysAsn

ACAGACTCGCGTTGCAAGGCGAGGCAGCTT GAGTTAAACGAACGTACTTGCAGATGTGAC
ThrAspSerArgCysLysAlaArgGlnLeu GluLeuAsnGluArgThrCysArgCysAsp
161
Bam HI
ggatccGCCCTGGCCCAGAAGCGCGACAAC GTGCTGTTCCAGGCAGCTACCGACGAGCAG
GlySerAlaLeuAlaGlnLysArgAspAsn ValLeuPheGlnAlaAlaThrAspGluGln
23

CCGGCCGTGATCAAGACGCTGGAGAAGCTG GTCAACATCGAGACCGGCACCGGTGACGCC
ProAlaValIleLysThrLeuGluLysLeu ValAsnIleGluThrGlyThrGlyAspAla

GAGGGCATCGCCGCTGCGGGCAACTTCCTC GAGGCCGAGCTCAAGAACCTCGGCTTCACG
GluGlyIleAlaAlaAlaGlyAsnPheLeu GluAlaGluLeuLysAsnLeuGlyPheThr

GTCACGCGAAGCAAGTCGGCCGGCCTGGTG GTGGGCGACAACATCGTGGGCAAGATCAAG
ValThrArgSerLysSerAlaGlyLeuVal ValGlyAspAsnIleValGlyLysIleLys

GGCCGCGGCGGCAAGAACCTGCTGCTGATG TCGCACATGGACACCGTCTACCTCAAGGGC
GlyArgGlyGlyLysAsnLeuLeuLeuMet SerHisMetAspThrValTyrLeuLysGly

ATTCTCGCGAAGGCCCCGTTCCGCGTCGAA GGCGACAAGGCCTACGGCCCGGGCATCGCC
IleLeuAlaLysAlaProPheArgValGlu GlyAspLysAlaTyrGlyProGlyIleAla

GACGACAAGGGCGGCAACGCGGTCATCCTG CACACGCTCAAGCTGCTGAAGGAATACGGC
AspAspLysGlyGlyAsnAlaValIleLeu HisThrLeuLysLeuLeuLysGluTyrGly

GTGCGCGACTACGGCACCATCACCGTGCTG TTCAATACCGACGAGGAAAAGGGTTCCTTC
ValArgAspTyrGlyThrIleThrValLeu PheAsnThrAspGluGluLysGlySerPhe

GGCTCGCGCGACCTGATCCAGGAAGAAGCC AAGCTGGCCGACTACGTGCTCTCCTTCGAG
GlySerArgAspLeuIleGlnGluGluAla LysLeuAlaAspTyrValLeuSerPheGlu

CCCACCAGCGCAGGCGACGAAAAACTCTCG CTGGGCACCTCGGGCATCGCCTACGTGCAG
ProThrSerAlaGlyAspGluLysLeuSer LeuGlyThrSerGlyIleAlaTyrValGln

GTCCAAATCACCGGCAAGGCCTCGCATGCC GGCGCCGCGCCCGAGCTGGGCGTGAACGCG
ValGlnIleThrGlyLysAlaSerHisAla GlyAlaAlaProGluLeuGlyValAsnAla

CTGGTCGAGGCTTCCGACCTCGTGCTGCGC ACGATGAACATCGACGACAAGGCGAAGAAC
LeuValGluAlaSerAspLeuValLeuArg ThrMetAsnIleAspAspLysAlaLysAsn

CTGCGCTTCCAATGGACCATCGCCAAGGCC GGCCAAGTCTCGAACATCATCCCCGCCAGC
LeuArgPheGlnTrpThrIleAlaLysAla GlyGlnValSerAsnIleIleProAlaSer

GCCACGCTGAACGCCGACGTGCGCTACGCG CGCAACGAGGACTTCGACGCCGCCATGAAG
AlaThrLeuAsnAlaAspValArgTyrAla ArgAsnGluAspPheAspAlaAlaMetLys

ACGCTGGAAGAGCGCGCGCAGCAGAAGAAG CTGCCCGAGGCCGACGTGAAGGTGATCGTC
ThrLeuGluGluArgAlaGlnGlnLysLys LeuProGluAlaAspValLysValIleVal

ACGCGCGGCCGCCCGGCCTTCAATGCCGGC GAAGGCGGCAAGAAGCTGGTCGACAAGGCG
ThrArgGlyArgProAlaPheAsnAlaGly GluGlyGlyLysLysLeuValAspLysAla

GTGGCCTACTACAAGGAAGCCGGCGGCACG CTGGGCGTGGAAGAGCGCACCGGCGGCGGC
ValAlaTyrTyrLysGluAlaGlyGlyThr LeuGlyValGluGluArgThrGlyGlyGly

ACCGACGCGGCCTACGCCGCGCTCTCAGGC AAGCCAGTGATCGAGAGCCTGGGCCTGCCG
ThrAspAlaAlaTyrAlaAlaLeuSerGly LysProValIleGluSerLeuGlyLeuPro

GGCTTCGGCTACCACAGCGACAAGGCCGAG TACGTGGACATCAGCGCGATTCCGCGCCGC
GlyPheGlyTyrHisSerAspLysAlaGlu TyrValAspIleSerAlaIleProArgArg

Eco RI
CTGTACATGGCTGCGCGCCTGATCATGGAT CTGGGCGCCGGCAAGgaattcCATCATCAC
LeuTyrMetAlaAlaArgLeuIleMetAsp LeuGlyAlaGlyLysGluPheHisHisHis
415

XbaI
CACCATCACGCTTCCTAGtctaga 3
HisHisHisAlaSer • -C

SEQUENCE OF V115CPH6.

SEQ ID NO.9 : DNA SEQUENCE
SEQ ID NO.10 : PROTEIN SEQUENCE
Nco 1
5' ccATG
N- Met
-26

GACTTTCTGCTGTCTTGGGTGCATTGGAGC CTTGCCTTGCTGCTCTACCTCCACCATGCC
AspPheLeuLeuSerTrpValHIaTrpSer LeuAlaLeuLeuLeuTyrLeuHisHisAla

*
AAGTGGTCCCAGGCTGCACCTATGGCAGAA GGAGGAGGGCAGAATCATCACGAAGTGGTG
LysTrpSerGlnAlaAlaProMetAlaAsp GlyGlyGlyGlnAsnHisHisGluValVal
-1 1

AAGTTCATGGATGTCTATCAGCGCAGCTAC TGCCATCCAATCGAGACCCTGGTGGACATC
LysPheMetAspValTyrGlnArgSerTyr CysHisProIleGluThrLeuValAspIle

TTCCAGGAGTACCCTGATGAGATCGAGTAC ATCTTCAAGCCATCCTGTGTGCCCCTGATG
PheGlnGluTyrProAspGluIleGluTyr IlePheLysProSerCysValProLeuMet

CGATGCGGGGGCTGCTGCAATGACGAGGGC CTGGAGTGTGTGCCCACTGAGGAGTCCAAC
ArgCysGluGluCysCysAsnAspGluGly LeuAspCysValProThrGluGluSerAsn

ATCACCATGCAGATTATGCGGATCAAACCT CACCAAGGCCAGCACATAGGAGAGATGAGC
IleThrMetGlnIleMetArgIleLysPro HisGlnGlyGlnHisIleGlyGluMetSer

TTCCTACAGCACAACAAATGTGAATGCAGA CCAAAGAAAGATAGAGCAAGACAAGAAAAT
PheLeuGlnHisAsnLysCysGluCysArg ProLysLysAspArgAlaArgGlnGluAsn
115
Bam HI
ggatccGCCCTGGCCCAGAAGCGCGACAAC GTGCTGTTCCAGGCAGCTACCGACGAGCAG
GlySerAlaLeuAlaGlnLysArgAspAsn ValLeuPheGlnAlaAlaThrAspGluGln
23

CCGGCCGTGATCAAGACGCTGGAGAAGCTG GTCAACATCGAGACCGGCACCGGTGACGCC
ProAlaValIleLysThrLeuGluLysLeu ValAsnIleGluThrGlyThrGlyAspAla

GAGGGCATCGCCGCTGCGGGCAACTTCCTC GAGGCCGAGCTCAAGAACCTCGGCTTCACG
GluGlyIleAlaAlaAlaGlyAsnPheLeu GluAlaGluLeuLysAsnLeuGlyPheThr

GTCACGCGAAGCAAGTCGGCCGGCCTGGTG GTGGGCGACAACATCGTGGGCAAGATCAAG
ValThrArgSerLysSerAlaGlyLeuVal ValGlyAspAsnIleValGlyLysIleLys

GGCCGCGGCGGCAAGAACCTGCTGCTGATG TCGCACATGGACACCGTCTACCTCAAGGGC
GlyArgGlyGlyLysAsnLeuLeuLeuMet SerHisMetAspThrValTyrLeuLysGly

ATTCTCGCGAAGGCCCCGTTCCGCGTCGAA GGCGACAAGGCCTACGGCCCGGGCATCGCC
IleLeuAlaLysAlaProPheArgValGlu GlyAspLysAlaTyrGlyProGlyIleAla

GACGACAAGGGCGGCAACGCGGTCATCCTG CACACGCTCAAGCTGCTGAAGGAATACGGC
AspAspLysGlyGlyAsnAlaValIleLeu HisThrLeuLysLeuLeuLysGluTyrGly

GTGCGCGACTACGGCACCATCACCGTGCTG TTCAATACCGACGAGGAAAAGGGTTCCTTC
ValArgAspTyrGlyThrIleThrValLeu PheAsnThrAspGluGluLysGlySerPhe

GGCTCGCGCGACCTGATCCAGGAAGAAGCC AAGCTGGCCGACTACGTGCTCTCCTTCGAG
GlySerArgAspLeuIleGlnGluGluAla LysLeuAlaAspTyrValLeuSerPheGlu

CCCACCAGCGCAGGCGACGAAAAACTCTCG CTGGGCACCTCGGGCATCGCCTACGTGCAG
ProThrSerAlaGlyAspGluLysLeuSer LeuGlyThrSerGlyIleAlaTyrValGln

GTCCAAATCACCGGCAAGGCCTCGCATGCC GGCGCCGCGCCCGAGCTGGGCGTGAACGCG
ValGlnIleThrGlyLysAlaSerHisAla GlyAlaAlaProGluLeuGlyValAsnAla

CTGGTCGAGGCTTCCGACCTCGTGCTGCGC ACGATGAACATCGACGACAAGGCGAAGAAC
LeuValGluAlaSerAspLeuValLeuArg ThrMetAsnIleAspAspLysAlaLysAsn

CTGCGCTTCCAATGGACCATCGCCAAGGCC GGCCAAGTCTCGAACATCATCCCCGCCAGC
LeuArgPheGlnTrpThrIleAlaLysAla GlyGlnValSerAsnIleIleProAlaSer

GCCACGCTGAACGCCGACGTGCGCTACGCG CGCAACGAGGACTTCGACGCCGCCATGAAG
AlaThrLeuAsnAlaAspValArgTyrAla ArgAsnGluAspPheAspAlaAlaMetLys

ACGCTGGAAGAGCGCGCGCAGCAGAAGAAG CTGCCCGAGGCCGACGTGAAGGTGATCGTC
ThrLeuGluGluArgAlaGlnGlnLysLys LeuProGluAlaAspValLysValIleVal

ACGCGCGGCCGCCCGGCCTTCAATGCCGGC GAAGGCGGCAAGAAGCTGGTCGACAAGGCG
ThrArgGlyArgProAlaPheAsnAlaGly GluGlyGlyLysLysLeuValAspLysAla

GTGGCCTACTACAAGGAAGCCGGCGGCACG CTGGGCGTGGAAGAGCGCACCGGCGGCGGC
ValAlaTyrTyrLysGluAlaGlyGlyThr LeuGlyValGluGluArgThrGlyGlyGly

ACCGACGCGGCCTACGCCGCGCTCTCAGGC AAGCCAGTGATCGAGAGCCTGGGCCTGCCG
ThrAspAlaAlaTyrAlaAlaLeuSerGly LysProValIleGluSerLeuGlyLeuPro

GGCTTCGGCTACCACAGCGACAAGGCCGAG TACGTGGACATCAGCGCGATTCCGCGCCGC
GlyPheGlyTyrHisSerAspLysAlaGlu TyrValAspIleSerAlaIleProArgArg

Eco RI
CTGTACATGGCTGCGCGCCTGATCATGGAT CTGGGCGCCGGCAAGgaattcCATCATCAC
LeuTyrMetAlaAlaArgLeuIleMetAsp LeuGlyAlaGlyLysGluPheHisHisHis
415

XbaI
CACCATCACGCTTCCTAGtctaga 3'
HisHisHisAlaSer • -C

Sequences of CPV165

SEQ ID NO. 11: DNA Sequence
SEQ ID NO. 12: Protein Sequence

Nco 1
5' CCATGGAGCTGGCGGCCTTGTGC
N- MetGluLeuAlaAlaLeuCys
1

CGCTGGGGGCTCCTCCTCGCCCTCTTGCCC CCCGGAGCCGCGAGCACCCAAGTGTGCACC
ArgTrpGlyLeuLeuLeuAlaLeuLeuPro ProGlyAlaAlaSerThrGlnValCysThr
27

Bam HI
ggatccGCCCTGGCCCAGAAGCGCGACAAC GTGCTGTTCCAGGCAGCTACCGACGAGCAG
GlySerAlaLeuAlaGlnLysArgAspAsn ValLeuPheGlnAlaAlaThrAspGluGln
23

CCGGCCGTGATCAAGACGCTGGAGAAGCTG GTCAACATCGAGACCGGCACCGGTGACGCC
ProAlaValIleLysThrLeuGluLysLeu ValAsnIleGluThrGlyThrGlyAspAla

GAGGGCATCGCCGCTGCGGGCAACTTCCTC GAGGCCGAGCTCAAGAACCTCGGCTTCACG
GluGlyIleAlaAlaAlaGlyAsnPheLeu GluAlaGluLeuLysAsnLeuGlyPheThr

GTCACGCGAAGCAAGTCGGCCGGCCTGGTG GTGGGCGACAACATCGTGGGCAAGATCAAG
ValThrArgSerLysSerAlaGlyLeuVal ValGlyAspAsnIleValGlyLysIleLys

GGCCGCGGCGGCAAGAACCTGCTGCTGATG TCGCACATGGACACCGTCTACCTCAAGGGC
GlyArgGlyGlyLysAsnLeuLeuLeuMet SerHisMetAspThrValTyrLeuLysGly

ATTCTCGCGAAGGCCCCGTTCCGCGTCGAA GGCGACAAGGCCTACGGCCCGGGCATCGCC
IleLeuAlaLysAlaProPheArgValGlu GlyAspLysAlaTyrGlyProGlyIleAla

GACGACAAGGGCGGCAACGCGGTCATCCTG CACACGCTCAAGCTGCTGAAGGAATACGGC
AspAspLysGlyGlyAsnAlaValIleLeu HisThrLeuLysLeuLeuLysGluTyrGly

GTGCGCGACTACGGCACCATCACCGTGCTG TTCAATACCGACGAGGAAAAGGGTTCCTTC
ValArgAspTyrGlyThrIleThrValLeu PheAsnThrAspGluGluLysGlySerPhe

GGCTCGCGCGACCTGATCCAGGAAGAAGCC AAGCTGGCCGACTACGTGCTCTCCTTCGAG
GlySerArgAspLeuIleGlnGluGluAla LysLeuAlaAspTyrValLeuSerPheGlu

CCCACCAGCGCAGGCGACGAAAAACTCTCG CTGGGCACCTCGGGCATCGCCTACGTGCAG
ProThrSerAlaGlyAspGluLysLeuSer LeuGlyThrSerGlyIleAlaTyrValGln

GTCCAAATCACCGGCAAGGCCTCGCATGCC GGCGCCGCGCCCGAGCTGGGCGTGAACGCG
ValGlnIleThrGlyLysAlaSerHisAla GlyAlaAlaProGluLeuGlyValAsnAla

CTGGTCGAGGCTTCCGACCTCGTGCTGCGC ACGATGAACATCGACGACAAGGCGAAGAAC
LeuValGluAlaSerAspLeuValLeuArg ThrMetAsnIleAspAspLysAlaLysAsn

CTGCGCTTCCAATGGACCATCGCCAAGGCC GGCCAAGTCTCGAACATCATCCCCGCCAGC
LeuArgPheGlnTrpThrIleAlaLysAla GlyGlnValSerAsnIleIleProAlaSer

GCCACGCTGAACGCCGACGTGCGCTACGCG CGCAACGAGGACTTCGACGCCGCCATGAAG
AlaThrLeuAsnAlaAspValArgTyrAla ArgAsnGluAspPheAspAlaAlaMetLys

ACGCTGGAAGAGCGCGCAGCAGAAGAAG CTGCCCGAGGCCGACGTGAAGGTGATCGTC
ThrLeuGluGluArgAlaGlnGlnLysLys LeuProGluAlaAspValLysValIleVal

ACGCGCGGCCGCCCGGCCTTCAATGCCGGC GAAGGCGGCAAGAAGCTGGTCGACAAGGCG
ThrArgGlyArgProAlaPheAsnAlaGly GluGlyGlyLysLysLeuValAspLysAla

GTGGCCTACTACAAGGAAGCCGGCGGCACG CTGGGCGTGGAAGAGCGCACCGGCGGCGGC
ValAlaTyrTyrLysGluAlaGlyGlyThr LeuGlyValGluGluArgThrGlyGlyGly

ACCGACGCGGCCTACGCCGCGCTCTCAGGC AAGCCAGTGATCGAGAGCCTGGGCCTGCCG
ThrAspAlaAlaTyrAlaAlaLeuSerGly LysProValIleGluSerLeuGlyLeuPro

GGCTTCGGCTACCACAGCGACAAGGCCGAG TACGTGGACATCAGCGCGATTCCGCGCCGC
GlyPheGlyTyrHisSerAspLysAlaGlu TyrValAspIleSerAlaIleProArgArg

Eco RI
CTGTACATGGCTGCGCGCCTGATCATGGAT CTGGGCGCCGGCAAGgaattcGGAGGTGGC
LeuTyrMetAlaAlaArgLeuIleMetAsp LeuGlyAlaGlyLysGluPheGlyGlyGly
415

Kpn 1
GGAggtaccGCACCCATGGCAGAAGGAGGA GGGCAGAATCATCACGAAGTGGTGAAGTTC
GlyGlyThrAlaProMetAlaAspGlyGly GlyGlnAsnHisHisGluValValLysPhe
1

ATGGATGTCTATCAGCGCAGCTACTGCCAT CCAATCGAGACCCTGGTGGACATCTTCCAG
MetAspValTyrGlnArgSerTyrCysHis ProIleGluThrLeuValAspIlePheGln

GAGTACCCTGATGAGATCGAGTACATCTTC AAGCCATCCTGTGTGCCCCTGATGCGATGC
GluTyrProAspGluIleGluTyrIlePhe LysProSerCysValProLeuMetArgCys

GGGGGCTGCTGCAATGACGAGGGCCTGGAG TGTGTGCCCACTGAGGAGTCCAACATCACC
GluGluCysCysAsnAspGluGlyLeuAsp CysValProThrGluGluSerAsnIleThr

ATGCAGATTATGCGGATCAAACCTCACCAA GGCCAGCACATAGGAGAGATGAGCTTCCTA
MetGlnIleMetArgIleLysProHisGln GlyGlnHisIleGlyGluMetSerPheLeu

CAGCACAACAAATGTGAATGCAGACCAAAG AAAGATAGAGCAAGACAAGAAAATCCCTGT
GlnHisAsnLysCysGluCysArgProLys LysAspArgAlaArgGlnGluAsnProCys

GGGCCTTGCTCAGAGCGGAGAAAGCATTTG TTTGTACAAGATCCGCAGACGTGTAAATGT
GlyProCysSerGluArgArgLysHisLeu PheValGlnAspProGlnThrCysLysCys

TCCTGCAAAAACACAGACTCGCGTTGCAAG GCGAGGCAGCTTGAGTTAAACGAACGTACT
SerCysLysAsnThrAspSerArgCysLys AlaArgGlnLeuGluLeuAsnGluArgThr

TGCAGATGTGACAAGCCGAGGCGGTGA 3'
CysArgCysAspLysProArgArg • -C
165

Sequences of CPV109

SEQ ID NO. 13: DNA Sequence
SEQ ID NO. 14: Protein Sequence

Nco 1
5' CCATGGAGCTGGCGGCCTTGTGC
N- MetGluLeuAlaAlaLeuCys
1

CGCTGGGGGCTCCTCCTCGCCCTCTTGCCC CCCGGAGCCGCGAGCACCCAAGTGTGCACC
ArgTrpGlyLeuLeuLeuAlaLeuLeuPro ProGlyAlaAlaSerThrGlnValCysThr
27

Bam HI
ggatccGCCCTGGCCCAGAAGCGCGACAAC GTGCTGTTCCAGGCAGCTACCGACGAGCAG
GlySerAlaLeuAlaGlnLysArgAspAsn ValLeuPheGlnAlaAlaThrAspGluGln
23

CCGGCCGTGATCAAGACGCTGGAGAAGCTG GTCAACATCGAGACCGGCACCGGTGACGCC
ProAlaValIleLysThrLeuGluLysLeu ValAsnIleGluThrGlyThrGlyAspAla

GAGGGCATCGCCGCTGCGGGCAACTTCCTC GAGGCCGAGCTCAAGAACCTCGGCTTCACG
GluGlyIleAlaAlaAlaGlyAsnPheLeu GluAlaGluLeuLysAsnLeuGlyPheThr

GTCACGCGAAGCAAGTCGGCCGGCCTGGTG GTGGGCGACAACATCGTGGGCAAGATCAAG
ValThrArgSerLysSerAlaGlyLeuVal ValGlyAspAsnIleValGlyLysIleLys

GGCCGCGGCGGCAAGAACCTGCTGCTGATG TCGCACATGGACACCGTCTACCTCAAGGGC
GlyArgGlyGlyLysAsnLeuLeuLeuMet SerHisMetAspThrValTyrLeuLysGly

ATTCTCGCGAAGGCCCCGTTCCGCGTCGAA GGCGACAAGGCCTACGGCCCGGGCATCGCC
IleLeuAlaLysAlaProPheArgValGlu GlyAspLysAlaTyrGlyProGlyIleAla

GACGACAAGGGCGGCAACGCGGTCATCCTG CACACGCTCAAGCTGCTGAAGGAATACGGC
AspAspLysGlyGlyAsnAlaValIleLeu HisThrLeuLysLeuLeuLysGluTyrGly

GTGCGCGACTACGGCACCATCACCGTGCTG TTCAATACCGACGAGGAAAAGGGTTCCTTC
ValArgAspTyrGlyThrIleThrValLeu PheAsnThrAspGluGluLysGlySerPhe

GGCTCGCGCGACCTGATCCAGGAAGAAGCC AAGCTGGCCGACTACGTGCTCTCCTTCGAG
GlySerArgAspLeuIleGlnGluGluAla LysLeuAlaAspTyrValLeuSerPheGlu

CCCACCAGCGCAGGCGACGAAAAACTCTCG CTGGGCACCTCGGGCATCGCCTACGTGCAG
ProThrSerAlaGlyAspGluLysLeuSer LeuGlyThrSerGlyIleAlaTyrValGln

GTCCAAATCACCGGCAAGGCCTCGCATGCC GGCGCCGCGCCCGAGCTGGGCGTGAACGCG
ValGlnIleThrGlyLysAlaSerHisAla GlyAlaAlaProGluLeuGlyValAsnAla

CTGGTCGAGGCTTCCGACCTCGTGCTGCGC ACGATGAACATCGACGACAAGGCGAAGAAC
LeuValGluAlaSerAspLeuValLeuArg ThrMetAsnIleAspAspLysAlaLysAsn

CTGCGCTTCCAATGGACCATCGCCAAGGCC GGCCAAGTCTCGAACATCATCCCCGCCAGC
LeuArgPheGlnTrpThrIleAlaLysAla GlyGlnValSerAsnIleIleProAlaSer

GCCACGCTGAACGCCGACGTGCGCTACGCG CGCAACGAGGACTTCGACGCCGCCATGAAG
AlaThrLeuAsnAlaAspValArgTyrAla ArgAsnGluAspPheAspAlaAlaMetLys

ACGCTGGAAGAGCGCGCAGCAGAAGAAG CTGCCCGAGGCCGACGTGAAGGTGATCGTC
ThrLeuGluGluArgAlaGlnGlnLysLys LeuProGluAlaAspValLysValIleVal

ACGCGCGGCCGCCCGGCCTTCAATGCCGGC GAAGGCGGCAAGAAGCTGGTCGACAAGGCG
ThrArgGlyArgProAlaPheAsnAlaGly GluGlyGlyLysLysLeuValAspLysAla

GTGGCCTACTACAAGGAAGCCGGCGGCACG CTGGGCGTGGAAGAGCGCACCGGCGGCGGC
ValAlaTyrTyrLysGluAlaGlyGlyThr LeuGlyValGluGluArgThrGlyGlyGly

ACCGACGCGGCCTACGCCGCGCTCTCAGGC AAGCCAGTGATCGAGAGCCTGGGCCTGCCG
ThrAspAlaAlaTyrAlaAlaLeuSerGly LysProValIleGluSerLeuGlyLeuPro

GGCTTCGGCTACCACAGCGACAAGGCCGAG TACGTGGACATCAGCGCGATTCCGCGCCGC
GlyPheGlyTyrHisSerAspLysAlaGlu TyrValAspIleSerAlaIleProArgArg

Eco RI
CTGTACATGGCTGCGCGCCTGATCATGGAT CTGGGCGCCGGCAAGgaattcGGAGGTGGC
LeuTyrMetAlaAlaArgLeuIleMetAsp LeuGlyAlaGlyLysGluPheGlyGlyGly
415

Kpn 1
GGAggtaccGCACCCATGGCAGAAGGAGGA GGGCAGAATCATCACGAAGTGGTGAAGTTC
GlyGlyThrAlaProMetAlaAspGlyGly GlyGlnAsnHisHisGluValValLysPhe
1

ATGGATGTCTATCAGCGCAGCTACTGCCAT CCAATCGAGACCCTGGTGGACATCTTCCAG
MetAspValTyrGlnArgSerTyrCysHis ProIleGluThrLeuValAspIlePheGln

GAGTACCCTGATGAGATCGAGTACATCTTC AAGCCATCCTGTGTGCCCCTGATGCGATGC
GluTyrProAspGluIleGluTyrIlePhe LysProSerCysValProLeuMetArgCys

GGGGGCTGCTGCAATGACGAGGGCCTGGAG TGTGTGCCCACTGAGGAGTCCAACATCACC
GluGluCysCysAsnAspGluGlyLeuAsp CysValProThrGluGluSerAsnIleThr

ATGCAGATTATGCGGATCAAACCTCACCAA GGCCAGCACATAGGAGAGATGAGCTTCCTA
MetGlnIleMetArgIleLysProHisGln GlyGlnHisIleGlyGluMetSerPheLeu

*
CAGCACAACAAATGTGAATGCAGACCAAAG AAAGATTGAGCAAGACAAGAAAATCCCTGT
GlnHisAsnLysCysGluCysArgProLys LysAsp • -C
109

GGGCCTTGCTCAGAGCGGAGAAAGCATTTG TTTGTACAAGATCCGCAGACGTGTAAATGT

TCCTGCAAAAACACAGACTCGCGTTGCAAG GCGAGGCAGCTTGAGTTAAACGAACGTACT

TGCAGATGTGACAAGCCGAGGCGGTGA 3'

SEQUENCE ID NOS. 15 and 16

Nco 1
5' CCATGGAGCTGGCGGCCTTGTGC
N- MetGluLeuAlaAlaLeuCys
1

CGCTGGGGGCTCCTCCTCGCCCTCTTGCCC CCCGGAGCCGCGAGCACCCAAGTGTGCACC
ArgTrpGlyLeuLeuLeuAlaLeuLeuPro ProGlyAlaAlaSerThrGlnValCysThr
27

Bam HI
ggatccGCCCTGGCCCAGAAGCGCGACAAC GTGCTGTTCCAGGCAGCTACCGACGAGCAG
GlySerAlaLeuAlaGlnLysArgAspAsn ValLeuPheGlnAlaAlaThrAspGluGln
23

CCGGCCCTGATCAAGACGCTGGAGAAGCTG GTCAACATCGAGACCGGCACCGGTGACGCC
ProAlaValIleLysThrLeuGluLysLeu ValAsnIleGluThrGlyThrGlyAspAla

GAGGGCATCGCCGCTGCGGGCAACTTCCTC GAGGCCGAGCTCAAGAACCTCGGCTTCACG
GluGlyIleAlaAlaGlyAsnPheLeu GluAlaGluLeuLysAsnLeuGlyPheThr

GTCACGCGAAGCAAGTCGGCCGGCCTGGTG GTGGGCGACAACATCGTGGGCAAGATCAAG
ValThrArgSerLysSerAlaGlyLeuVal ValGlyAspAsnIleValGlyLysIleLys

GGCCGCGGCGGCAAGAACCTGCTGCTGATG TCGCACATGGACACCGTCTACCTCAAGGGC
GlyArgGlyGlyLysAsnLeuLeuLeuMet SerHisMetAspThrValTyrLeuLysGly

ATTCTCGCGAAGGCCCCGTTCCGCGTCGAA GGCGACAAGGCCTACGGCCCGGGCATCGCC
IleLeuAlaLysAlaProPheArgValGlu GlyAspLysAlaTyrGlyProGlyIleAla

GACGACAAGGGCGGCAACGCGGTCATCCTG CACACGCTCAAGCTGCTGAAGGAATACGGC
AspAspLysGlyGlyAsnAlaValIleLeu HisThrLeuLysLeuLeuLysGluTyrGly

GTGCGCGACTACGGCACCATCACCGTGCTG TTCAATACCGACGAGGAAAAGGGTTCCTTC
ValArgAspTyrGlyThrIleThrValLeu PheAsnThrAspGluGluLysGlySerPhe

GGCTCGCGCGACCTGATCCAGGAAGAAGCC AAGCTGGCCGACTACGTGCTCTCCTTCGAG
GlySerArgAspLeuIleGlnGluGluAla LysLeuAlaAspTyrValLeuSerPheGlu

CCCACCAGCGCAGGCGACGAAAAACTCTCG CTGGGCACCTCGGGCATCGCCTACGTGCAG
ProThrSerAlaGlyAspGluLysLeuSer LeuGlyThrSerGlyIleAlaTyrValGln

GTCCAAATCACCGGCAAGGCCTCGCATGCC GGCGCCGCGCCCGAGCTGGGCGTGAACGCG
ValGlnIleThrGlyLysAlaSerHisAla GlyAlaAlaProGluLeuGlyValAsnAla

CTGGTCGAGGCTTCCGACCTCGTGCTGCGC ACGATGAACATCGACGACAAGGCGAAGAAC
LeuValGluAlaSerAspLeuValLeuArg ThrMetAsnIleAspAspLysAlaLysAsn

CTGCGCTTCCAATGGACCATCGCCAAGGCC GGCCAAGTCTCGAACATCATCCCCGCCAGC
LeuArgPheGlnTrpThrIleAlaLysAla GlyGlnValSerAsnIleIleProAlaSer

GCCACGCTGAACGCCGACGTGCGCTACGCG CGCAACGAGGACTTCGACGCCGCCATGAAG
AlaThrLeuAsnAlaAspValArgTyrAla ArgAsnGluAspPheAspAlaAlaMetLys

ACGCTGGAAGAGCGCGCGCAGCAGAAGAAG CTGCCCGAGGCCGACGTGAAGGTGATCGTC
ThrLeuGluGluArgAlaGlnGlnLysLys LeuProGluAlaAspValLysValIleVal

ACGCGCGGCCGCCCGGCCTTCAATGCCGGC GAAGGCGGCAAGAAGCTGGTCGACAAGGCG
ThrArgGlyArgProAlaPheAsnAlaGly GluGlyGlyLysLysLeuValAspLysAla

GTGGCCTACTACAAGGAAGCCGGCGGCACG CTGGGCGTGGAAGAGCGCACCGGCGGCGGC
ValAlaTyrTyrLysGluAlaGlyGlyThr LeuGlyValGluGluArgThrGlyGlyGly

ACCGACGCGGCCTACGCCGCGCTCTCAGGC AAGCCAGTGATCGAGAGCCTGGGCCTGCCG
ThrAspAlaAlaTyrAlaAlaLeuSerGly LysProValIleGluSerLeuGlyLeuPro

GGCTTCGGCTACCACAGCGACAAGGCCGAG TACGTGGACATCAGCGCGATTCCGCGCCGC
GlyPheGlyTyrHisSerAspLysAlaGlu TyrValAspIleSerAlaIleProArgArg

Eco RI
CTGTACATGGCTGCGCGCCTGATCATGGAT CTGGGCGCCGGCAAGgaattcGGAGGTGGC
LeuTyrMetAlaAlaArgLeuIleMetAsp LeuGlyAlaGlyLysGluPheGlyGlyGly
415

Kpn 1
GGAggtaccGCACCCATGGCAGAAGGAGGA GGGCAGAATCATCACGAAGTGGTGAAGTTC
GlyGlyThrAlaProMetAlaAspGlyGly GlyGlnAsnHisHisGluValValLysPhe
1

ATGGATGTCTATCAGCGCAGCTACTGCCAT CCAATCGAGACCCTGGTGGACATCTTCCAG
MetAspValTyrGlnArgSerTyrCysHis ProIleGluThrLeuValAspIlePheGln

GAGTACCCTGATGAGATCGAGTACATCTTC AAGCCATCCTGTGTGCCCCTGATGCGATGC
GluTyrProAspGluIleGluTyrIlePhe LysProSerCysValProLeuMetArgCys

GGGGGCTGCTGCAATGACGAGGGCCTGGAG TGTGTGCCCACTGAGGAGTCCAACATCACC
GluGluCysCysAsnAspGluGlyLeuAsp CysValProThrGluGluSerAsnIleThr

ATGCAGATTATGCGGATCAAACCTCACCAA GGCCAGCACATAGGAGAGATGAGCTTCCTA
MetGlnIleMetArgIleLysProHisGln GlyGlnHisIleGlyGluMetSerPheLeu

CAGCACAACAAATGTGAATGCAGACCAAAG AAAGATAGAGCAAGACAAGAAAATCCCTGT
GlnHisAsnLysCysGluCysArgProLys LysAspArgAlaArgGlnGluAsnProCys

GGGCCTTGCTCAGAGCGGAGAAAGCATTTG TTTGTACAAGATCCGCAGACGTGTAAATGT
GlyProCysSerGluArgArgLysHisLeu PheValGlnAspProGlnThrCysLysCys

TCCTGCAAAAACACAGACTCGCGTTGCAAG GCGAGGCAGCTTGAGTTAAACGAACGTACT
SerCysLysAsnThrAspSerArgCysLys AlaArgGlnLeuGluLeuAsnGluArgThr

Bam HI Eco RI Xba I
TGCAGATGTGACggattcgaattcCATCAT CACCACCATCACGCTTCCTAGtctaga 3'
CysArgCysAspGlySerGluPheHisHis HisHisHisHisAlaSer • -C
161

SEQUENCE ID NOS. 17 and 18

Nco 1
5' CCATGGAGCTGGCGGCCTTGTGC
N- MetGluLeuAlaAlaLeuCys
1

CGCTGGGGGCTCCTCCTCGCCCTCTTGCCC CCCGGAGCCGCGAGCACCCAAGTGTGCACC
ArgTrpGlyLeuLeuLeuAlaLeuLeuPro ProGlyAlaAlaSerThrGlnValCysThr
27

Bam HI
ggatccGCCCTGGCCCAGAAGCGCGACAAC GTGCTGTTCCAGGCAGCTACCGACGAGCAG
GlySerAlaLeuAlaGlnLysArgAspAsn ValLeuPheGlnAlaAlaThrAspGluGln
23

CCGGCCGTGATCAAGACGCTGGAGAAGCTG GTCAACATCGAGACCGGCACCGGTGACGCC
ProAlaValIleLysThrLeuGluLysLeu ValAsnIleGluThrGlyThrGlyAspAla

GAGGGCATCGCCGCTGCGGGCAACTTCCTC GAGGCCGAGCTCAAGAACCTCGGCTTCACG
GluGlyIleAlaAlaAlaGlyAsnPheLeu GluAlaGluLeuLysAsnLeuGlyPheThr

GTCACGCGAAGCAAGTCGGCCGGCCTGGTG GTGGGCGACAACATCGTGGGCAAGATCAAG
ValThrArgSerLysSerAlaGlyLeuVal ValGlyAspAsnIleValGlyLysIleLys

GGCCGCGGCGGCAAGAACCTGCTGCTGATG TCGCACATGGACACCGTCTACCTCAAGGGC
GlyArgGlyGlyLysAsnLeuLeuLeuMet SerHisMetAspThrValTyrLeuLysGly

ATTCTCGCGAAGGCCCCGTTCCGCGTCGAA GGCGACAAGGCCTACGGCCCGGGCATCGCC
IleLeuAlaLysAlaProPheArgValGlu GlyAspLysAlaTyrGlyProGlyIleAla

GACGACAAGGGCGGCAACGCGGTCATCCTG CACACGCTCAAGCTGCTGAAGGAATACGGC
AspAspLysGlyGlyAsnAlaValIleLeu HisThrLeuLysLeuLeuLysGluTyrGly

GTGCGCGACTACGGCACCATCACCGTGCTG TTCAATACCGACGAGGAAAAGGGTTCCTTC
ValArgAspTyrGlyThrIleThrValLeu PheAsnThrAspGluGluLysGlySerPhe

GGCTCGCGCGACCTGATCCAGGAAGAAGCC AAGCTGGCCGACTACGTGCTCTCCTTCGAG
GlySerArgAspLeuIleGlnGluGluAla LysLeuAlaAspTyrValLeuSerPheGlu

CCCACCAGCGCAGGCGACGAAAAACTCTCG CTGGGCACCTCGGGCATCGCCTACGTGCAG
ProThrSerAlaGlyAspGluLysLeuSer LeuGlyThrSerGlyIleAlaTyrValGln

GTCCAAATCACCGGCAAGGCCTCGCATGCC GGCGCCGCGCCCGAGCTGGGCGTGAACGCG
ValGlnIleThrGlyLysAlaSerHisAla GlyAlaAlaProGluLeuGlyValAsnAla

CTGGTCGAGGCTTCCGACCTCGTGCTGCGC ACGATGAACATCGACGACAAGGCGAAGAAC
LeuValGluAlaSerAspLeuValLeuArg ThrMetAsnIleAspAspLysAlaLysAsn

CTGCGCTTCCAATGGACCATCGCCAAGGCC GGCCAAGTCTCGAACATCATCCCCGCCAGC
LeuArgPheGlnTrpThrIleAlaLysAla GlyGlnValSerAsnIleIleProAlaSer

GCCACGCTGAACGCCGACGTGCGCTACGCG CGCAACGAGGACTTCGACGCCGCCATGAAG
AlaThrLeuAsnAlaAspValArgTyrAla ArgAsnGluAspPheAspAlaAlaMetLys

ACGCTGGAAGAGCGCGCAGCAGAAGAAG CTGCCCGAGGCCGACGTGAAGGTGATCGTC
ThrLeuGluGluArgAlaGlnGlnLysLys LeuProGluAlaAspValLysValIleVal

ACGCGCGGCCGCCCGGCCTTCAATGCCGGC GAAGGCGGCAAGAAGCTGGTCGACAAGGCG
ThrArgGlyArgProAlaPheAsnAlaGly GluGlyGlyLysLysLeuValAspLysAla

GTGGCCTACTACAAGGAAGCCGGCGGCACG CTGGGCGTGGAAGAGCGCACCGGCGGCGGC
ValAlaTyrTyrLysGluAlaGlyGlyThr LeuGlyValGluGluArgThrGlyGlyGly

ACCGACGCGGCCTACGCCGCGCTCTCAGGC AAGCCAGTGATCGAGAGCCTGGGCCTGCCG
ThrAspAlaAlaTyrAlaAlaLeuSerGly LysProValIleGluSerLeuGlyLeuPro

GGCTTCGGCTACCACAGCGACAAGGCCGAG TACGTGGACATCAGCGCGATTCCGCGCCGC
GlyPheGlyTyrHisSerAspLysAlaGlu TyrValAspIleSerAlaIleProArgArg

Eco RI
CTGTACATGGCTGCGCGCCTGATCATGGAT CTGGGCGCCGGCAAGgaattcGGAGGTGGC
LeuTyrMetAlaAlaArgLeuIleMetAsp LeuGlyAlaGlyLysGluPheGlyGlyGly
415

Kpn 1
GGAggtaccGCACCCATGGCAGAAGGAGGA GGGCAGAATCATCACGAAGTGGTGAAGTTC
GlyGlyThrAlaProMetAlaAspGlyGly GlyGlnAsnHisHisGluValValLysPhe
1

ATGGATGTCTATCAGCGCAGCTACTGCCAT CCAATCGAGACCCTGGTGGACATCTTCCAG
MetAspValTyrGlnArgSerTyrCysHis ProIleGluThrLeuValAspIlePheGln

GAGTACCCTGATGAGATCGAGTACATCTTC AAGCCATCCTGTGTGCCCCTGATGCGATGC
GluTyrProAspGluIleGluTyrIlePhe LysProSerCysValProLeuMetArgCys

GGGGGCTGCTGCAATGACGAGGGCCTGGAG TGTGTGCCCACTGAGGAGTCCAACATCACC
GluGluCysCysAsnAspGluGlyLeuAsp CysValProThrGluGluSerAsnIleThr

ATGCAGATTATGCGGATCAAACCTCACCAA GGCCAGCACATAGGAGAGATGAGCTTCCTA
MetGlnIleMetArgIleLysProHisGln GlyGlnHisIleGlyGluMetSerPheLeu

Bam HI Eco RI
CAGCACAACAAATGTGAATGCAGACCAAAG AAAGATggattcgaattcCATCATCACCAC
GlnHisAsnLysCysGluCysArgProLys LysAspGlySerGluPheHisHisHisHis
109

XbaI
CATCACGCTTCCTAGtctaga 3'
HisHisAlaSer • -C

SEQUENCE ID NOS. 19 and 20

Nco I
5' CCATGGAGAGCAAGGTGCTGCTGGCCGTC GCCCTGTGGCTCTGCGTGGAGACCCGGGCC
N- MetGluSerLysValLeuLeuAlaVal AlaLeuTrpLeuCysValGluThrArgAla
-19

GCCTCTGTGGGTTTGCCTAGTGTTTCTCTT GATCTGCCCAGGCTCAGCATACAAAAAGAC
AlaSerValGlyLeuProSerValSerLeu AspLeuProArgLeuSerIleGlnLysAsp
1

ATACTTACAATTAAGGCTAATACAACTCTT CAAATTACTTGCAGGGGACAGAGGGACTTG
IleLeuThrIleLysAlaAsnThrThrLeu GlnIleThrCysArgGlyGlnArgAspLeu

GACTGGCTTTGGCCCAATAATCAGAGTGGC AGTGAGCAAAGGGTGGAGGTGACTGAGTGC
AspTrpLeuTrpProAsnAsnGlnSerGly SerGluGlnArgValGluValThrGluCys

AGCGATGGCCTCTTCTGTAAGACACTCACA ATTCCAAAAGTGATCGGAAATGACACTGGA
SerAspGlyLeuPheCysLysThrLeuThr IleProLysValIleGlyAsnAspThrGly

GCCTACAAGTGCTTCTACCGGGAAACTGAC TTGGCCTCGGTCATTTATGTCTATGTTCAA
AlaTyrLysCysPheTyrArgGluThrAsp LeuAlaSerValIleTyrTyrTyrValGln

GATTACAGATCTCCATTTATTGCTTCTGTT AGTGACCAACATGGAGTCGTGTACATTACT
AspTyrArgSerProPheIleAlaSerVal SerAspGlnHisGlyValValTyrIleThr

GAGAACAAAAACAAAACTGTGGTGATTCCA TGTCTCGGGTCCATTTCAAATCTCAACGTG
GluAsnLysAsnLysThrValValIlePro CysLeuGlySerIleSerAsnLeuAsnVal

TCACTTTGTGCAAGATACCCAGAAAAGAGA TTTGTTCCTGATGGTAACAGAATTTCCTGG
SerLeuCysAlaArgTyrProGluLysArg PheValProAspGlyAsnArgIleSerTrp

GACAGCAAGAAGGGCTTTACTATTCCCAGC TACATGATCAGCTATGCTGGCATGGTCTTC
AspSerLysLysGlyPheThrIleProSer TyrMetIleSerTyrAlaGlyMetValPhe

TGTGAAGCAAAAATTAATGATGAAAGTTAC CAGTCTATTATGTACATAGTTGTCGTTGTA
CysGluAlaLysIleAsnAspGluSerTyr GlnSerIleMetTyrIleValValValVal

GGGTATAGGATTTATGATGTGGTTCTGAGT CCGTCTCATGGAATTGAACTATCTGTTGGA
GlyTyrArgIleTyrAspValValLeuSer ProSerHisGlyIleGluLeuSerValGly

GAAAAGCTTGTCTTAAATTGTACAGCAAGA ACTGAACTAAATGTGGGGATTGACTTCAAC
GluLysLeuValLeuAsnCysThrAlaArg ThrGluLeuAsnValGlyIleAspPheAsn

TGGGAATACCCTTCTTCGAAGCATCAGCAT AAGAAACTTGTAAACCGAGACCTAAAAACC
TrpGluTyrProSerSerLysHisGlnHis LysLysLeuValAsnArgAspLeuLysThr

CAGTCTGGGAGTGAGATGAAGAAATTTTTG AGCACCTTAACTATAGATGGTGTAACCCGG
GlnSerGlySerGluMetLysLysPheLeu SerThrLeuThrIleAspGlyValThrArg

AGTGACCAAGGATTGTACACCTGTGCAGCA TCCAGTGGGCTGATGACCAAGAAGAACAGC
SerAspGlnGlyLeuTyrThrCysAlaAla SerSerGlyLeuMetThrLysLysAsnSer

ACATTTGTCAGGGTCCATGAAAAACCTTTT GTTGCTTTTGGAAGTGGCATGGAATCTCTG
ThrPheValArgValHisGluLysProPhe ValAlaPheGlySerGlyMetGluSerLeu

GTGGAAGCCACGGTGGGGGAGCGTGTCAGA ATCCCTGCGAAGTACCTTGGTTACCCACCC
ValGluAlaThrValGlyGluArgValArg IleProAlaLysTyrLeuGlyTyrProPro

CCAGAAATAAAATGGTATAAAAATGGAATA CCCCTTGAGTCCAATCACACAATTAAAGCG
ProGluIleLysTrpTyrLysAsnGlyIle ProLeuGluSerAsnHisThrIleLysAla

GGGCATGTACTGACGATTATGGAAGTGAGT GAAAGAGACACAGGAAATTACACTGTCATC
GlyHisValLeuThrIleMetGluValSer GluArgAspThrGlyAsnTyrThrValIle

CTTACCAATCCCATTTCAAAGGAGAAGCAG AGCCATGTGGTCTCTCTGGTTGTGTATGTC
LeuThrAsnProIleSerLysGluLysGln SerHisValValSerLeuValValTyrVal

CCACCCCAGATTGGTGAGAAATCTCTAATC TCTCCTGTGGATTCCTACCAGTACGGCACC
ProProGlnIleGlyGluLysSerLeuIle SerProValAspSerTyrGlnTyrGlyThr

ACTCAAACGCTGACATGTACGGTCTATGCC ATTCCTCCCCCGCATCACATCCACTGGTAT
ThrGlnThrLeuThrCysThrValTyrAla IleProProProHisHisIleHisTrpTyr

TGGCAGTTGGAGGAAGAGTGCGCCAACGAG CCCAGCCAAGCTGTCTCAGTGACAAACCCA
TrpGlnLeuGluGluGluCysAlaAsnGlu ProSerGlnAlaValSerValThrAsnPro

TACCCTTGTGAAGAATGGAGAAGTGTGGAG GACTTCCAGGGAGGAAATAAAATTGAAGTT
TyrProCysGluGluTrpArgSerValGlu AspPheGlnGlyGlyAsnLysIleGluVal

AATAAAAATCAATTTGCTCTAATTGAAGGA AAAAACAAAACTGTAAGTACCCTTGTTATC
AsnLysAsnGlnPheAlaLeuIleGluGly LysAsnLysThrValSerThrLeuValIle

CAAGCGGCAAATGTGTCAGCTTTGTACAAA TGTGAAGCGGTCAACAAAGTCGGGAGAGGA
GlnAlaAlaAsnValSerAlaLeuTyrLys CysGluAlaValAsnLysValGlyArgGly

GAGAGGGTGATCTCCTTCCACGTGACCAGG GGTCCTGAAATTACTTTGCAACCTGACATG
GluArgValIleSerPheHisValThrArg GlyProGluIleThrLeuGlnProAspMet

CAGCCCACTGAGCAGGAGAGCGTGTCTTTG TGGTGCACTGCAGACAGATCTACGTTTGAG
GlnProThrGluGlnGluSerValSerLeu TrpCysThrAlaAspArgSerThrPheGlu

AACCTCACATGGTACAAGCTTGGCCCACAG CCTCTGCCAATCCATGTGGGAGAGTTGCCC
AsnLeuThrTrpTyrLysLeuGlyProGln ProLeuProIleHisValGlyGluLeuPro

ACACCTGTTTGCAAGAACTTGGATACTCTT TGGAAATTGAATGCCACCATGTTCTCTAAT
ThrProValCysLysAsnLeuAspThrLeu TrpLysLeuAsnAlaThrMetPheSerAsn

AGCACAAATGACATTTTGATCATGGAGCTT AAGAATGCATCCTTGCAGGACCAAGGAGAC
SerThrAsnAspIleLeuIleMetGluLeu LysAsnAlaSerLeuGlnAspGlnGlyAsp

TATGTCTGCCTTGCTCAAGACAGGAAGACC AAGAAAAGACATTGCGTGGTCAGGCAGCTC
TyrValCysLeuAlaGlnAspArgLysThr LysLysArgHisCysValValArgGlnLeu

ACAGTCCTAGAGCGTGTGGCACCCACGATC ACAGGAAACCTGGAGAATCAGACGACAAGT
ThrValLeuGluArgValAlaProThrIle ThrGlyAsnLeuGluAsnGlnThrThrSer

ATTGGGGAAAGCATCGAAGTCTCATGCACG GCATCTGGGAATCCCCCTCCACAGATCATG
IleGlyGluSerIleGluValSerCysThr AlaSerGlyAsnProProProGlnIleMet

TGGTTTAAAGATAATGAGACCCTTGTAGAA GACTCAGGCATTGTATTGAAGGATGGGAAC
TrpPheLysAspAsnGluThrLeuValGlu AspSerGlyIleValLeuLysAspGlyAsn

CGGAACCTCACTATCCGCAGAGTGAGGAAG GAGGACGAAGGCCTCTACACCTGCCAGGCA
ArgAsnLeuThrIleArgArgValArgLys GluAspGluGlyLeuTyrThrCysGlnAla

TGCAGTGTTCTTGGCTGTGCAAAAGTGGAG GCATTTTTCATAATAGAAGGTGCCCAGGAA
CysSerValLeuGlyCysAlaLysValGlu AlaPhePheIleIleGluGlyAlaGlnGlu

AAGACGAACTTGGAAATCATTATTCTAGTA GGCACGGCGGTGATTGCCATGTTCTTCTGG
LysThrAsnLeuGluIleIleIleLeuVal GlyThrAlaValIleAlaMetPhePheTrp
745

CTACTTCTTGTCATCATCCTACGGACCGTT AAGCGGGCCAATGGAGGGGAACTGAAGACA
LeuLeuLeuValIleIleLeuArgThrVal LysArgAlaAsnGlyGlyGluLeuLysThr
770

GGCTACTTGTCCATCGTCATGGATCCAGAT GAACTCCCATTGGATGAACATTGTGAACGA
GlyTyrLeuSerIleValMetAspProAsp GluLeuProLeuAspGluHisCysGluArg

Eco RI HindIII
CTGCCTTATGATGCCAGCAAATGGGAATTC aagcttcccggggtcgacatcgatgagcag
LeuProTyrAspAlaSerLysTrpGluPhe LysLeuProGlyValAspIleAspGluGln
810

Xba I
aagctgatatccgaggaggacctgaactagtctaga 3'
LysLeuIleSerGluGluAspLeuAsn • -C

## Claims

1. A two component system for use in association with one another comprising:
(a) a fusion protein or conjugate of a ligand with an enzyme, said ligand being :
(i) a naturally occuring polypeptide whose biological role is to bind to a cognate receptor on the surface of a cell;
(ii) a fragment of said polypeptide which still binds to its cognate receptor; or
(iii) a derivative of (i) or (ii) with altered receptor specificity; and
(b) a prodrug which can be converted into an active drug by said enzyme.

2. A system according to claim 1 wherein the enzyme is a bacterial carboxypeptidase.

3. A system according to claim 2 wherein the carboxypeptidase is CPG2.

4. A system according to claim 3 wherein the CPG2 has been altered at one or more glycosylation sites to prevent glycosylation at said site or sites.

5. A system according to any one of claims 2 to 4 wherein the prodrug is a nitrogen mustard prodrug.

6. A system according to any one of claims 1 to 5 wherein the ligand is vascular endothelial growth factor (VEGF).

7. A fusion protein comprising a ligand and an enzyme capable of converting a prodrug into a toxic agent, said ligand being:
(i) a naturally occuring polypeptide whose biological role is to bind to a cognate receptor on the surface of a cell;
(ii) a fragment of said polypeptide which still binds to its cognate receptor; or
(iii) a derivative of (i) or (ii) with altered receptor specificity.

8. A fusion protein as decribed in SEQ ID No. 8, 10, 12, 14, 16 or 18 or as encoded by the DNA of SEQ ID No. 7, 9, 11, 13, 15 or 17.

9. A system according to claim 1 comprising a fusion protein according to claim 8 and a prodrug which can be converted into an active drug by CPG2.

10. A system according to any one of claims 1 to 6 or 9 for use in a method of treatment or therapy of the human or animal body.

11. Use of a fusion protein or conjugate of a ligand with an enzyme capable of binding to a tumour and a cell and a prodrug capable of being converted by said enzyme to an active drug, in the manufacture of a medicament for use in the treatment of the tumour, said ligand being:
(i) a naturally occuring polypeptide whose biological role is to bind to a cognate receptor on the surface of a cell;
(ii) a fragment of said polypeptide which still binds to its cognate receptor; or
(iii) a derivative of (i) or (ii) with altered receptor specificity.

12. A process for preparing a two component system for use in association with one another comprising:
(a) fusion protein or conjugate of a ligand with an enzyme said ligand being:
(i) a naturally occuring polypeptide whose biological role is to bind to a cognate receptor on the surface of a cell;
(ii) a fragment of said polypeptide which still binds to its cognate receptor; or
(iii) a derivative of (i) or (ii) with altered receptor specificity; and
(b) a prodrug which can be converted into an active drug by said enzyme;
said process comprising preparing a fusion protein or conjugate of a ligand with an enzyme,
and placing said fusion protein or conjugate in association with a prodrug which can be converted into an active drug by said enzyme.

13. A process for preparing a fusion protein comprising a ligand which ligand comprises:
(i) a naturally occuring polypeptide whose biological role is to bind to a cognate receptor on the surface of a cell;
(ii) a fragment of said polypeptide which still binds to its cognate receptor; or
(iii) a derivative of (i) or (ii) with altered receptor specificity; and
an enzyme capable of converting a prodrug into a toxic agent, said process comprising culturing a host cell which contains an expression vector which comprises a nucleic encoding said fusion protein, said culturing being carried out under conditions in which said fusion protein is expressed, and recovering the fusion protein in substantially isolated form.

14. A nucleic acid encoding a fusion protein as claimed in claim 7 or 8.

15. A vector comprising a nucleic acid as claimed in claim 14.

16. A vector as claimed in claim 15 comprising a bacterial carboxypeptidase gene which has been altered by substitution, deletion or insertion at one or more glycosylation sites, fused in-frame to a gene encoding a ligand or fragment or derivative thereof.

17. A host cell which contains an expression vector comprising a vector as claimed in claim 15 or 16 operably linked to a promoter compatible with the host cell for expression of the fusion protein.

18. A product containing:
(a) fusion protein or conjugate of a ligand with an enzyme, said ligand being:
(i) a naturally occuring polypeptide whose biological role is to bind to a cognate receptor on the surface of a cell;
(ii) a fragment of said polypeptide which still binds to its cognate receptor; or
(iii) a derivative of (i) or (ii) with altered receptor specificity; and
(b) a prodrug which can be converted into an active drug by said enzyme;
as a combined preparation for separate or sequential use in the treatment of a tumour.

## Patentansprüche

1. Zwei-Komponenten-System zum kombinierten Einsatz, umfassend:
(a) ein Fusionsprotein oder ein Konjugat aus einem Liganden mit einem Enzym, wobei der Ligand:
(i) ein natürlich vorkommendes Polypeptid, dessen biologische Rolle darin besteht, sich an einen zugehörigen Rezeptor an der Oberfläche einer Zelle zu binden;
(ii) ein Fragment des Polypeptids, das sich immer noch an seinen zugehörigen Rezeptor bindet; oder
(iii) ein Derivat von (i) oder (ii) mit veränderter Rezeptorspezifität ist; und
(b) ein Proarzneimittel, das durch das Enzym in ein aktives Arzneimittel umgewandelt werden kann.

2. System nach Anspruch 1, worin das Enzym eine bakterielle Carboxypeptidase ist.

3. System nach Anspruch 2, worin die Carboxypeptidase CPG2 ist.

4. System nach Anspruch 3, worin die CPG2 an einer oder mehreren Glykosylierungsstellen verändert worden ist, um Glykosylierung an dieser Stelle oder diesen Stellen zu verhindern.

5. System nach einem der Ansprüche 2 bis 4, worin das Proarzneimittel ein Stickstoff-Lost-Proarzneimittel ist.

6. System nach einem der Ansprüche 1 bis 5, worin der Ligand Vaskulär-Endothel-Wachstumsfaktor (VEGF) ist.

7. Fusionsprotein, das einen Liganden und ein Enzym umfaßt, das fähig ist, ein Proarzneimittel in einen toxischen Stoff umzuwandeln, wobei der Ligand:
(i) ein natürlich vorkommendes Polypeptid, dessen biologische Rolle darin besteht, sich an einen zugehörigen Rezeptor an der Oberfläche einer Zelle zu binden;
(ii) ein Fragment des Polypeptids, das sich immer noch an seinen zugehörigen Rezeptor bindet; oder
(iii) ein Derivat von (i) oder (ii) mit veränderter Rezeptorspezifität ist.

8. Fusionsprotein, wie in den Seq.-ID Nr. 8, 10, 12, 14, 16 oder 18 beschrieben oder wie von der DNA mit Seq.-ID Nr. 7, 9, 11, 13, 15 oder 17 kodiert.

9. System nach Anspruch 1, das ein Fusionsprotein nach Anspruch 8 und ein Proarzneimittel umfaßt, das durch CPG2 in ein aktives Arzneimittel umgewandelt werden kann.

10. System nach einem der Ansprüche 1 bis 6 oder 9 zur Verwendung in einem Verfahren zur Behandlung oder Therapie eines Menschen- oder Tierkörpers.

11. Verwendung eines Fusionsproteins oder Konjugats aus einem Liganden mit einem Enzym, das zur Bindung an einen Tumor und eine Zelle fähig ist, und einem Proarzneimittel, das durch das Enzym in ein aktives Arzneimittel umgewandelt werden kann, zur Herstellung eines Medikaments zur Behandlung des Tumors, wobei der Ligand:
(i) ein natürlich vorkommendes Polypeptid, dessen biologische Rolle darin besteht, sich an einen zugehörigen Rezeptor an der Oberfläche einer Zelle zu binden;
(ii) ein Fragment des Polypeptids, das sich immer noch an seinen zugehörigen Rezeptor bindet; oder
(iii) ein Derivat von (i) oder (ii) mit veränderter Rezeptorspezifität ist.

12. Verfahren zur Herstellung eines Zwei-Komponenten-Systems zum kombinierten Einsatz, umfassend:
(a) ein Fusionsprotein oder ein Konjugat aus einem Liganden mit einem Enzym, wobei der Ligand:
(i) ein natürlich vorkommendes Polypeptid, dessen biologische Rolle darin besteht, sich an einen zugehörigen Rezeptor an der Oberfläche einer Zelle zu binden;
(ii) ein Fragment des Polypeptids, das sich immer noch an seinen zugehörigen Rezeptor bindet; oder
(iii) ein Derivat von (i) oder (ii) mit veränderter Rezeptorspezifität ist; und
(b) ein Proarzneimittel, das durch das Enzym in ein aktives Arzneimittel umgewandelt werden kann;
wobei das Verfahren die Herstellung eines Fusionsproteins oder Konjugats aus einem Liganden mit einem Enzym und das Kombinieren des Fusionsproteins oder Konjugats mit einem Proarzneimittel, das durch das Enzym in ein aktives Arzneimittel umgewandelt werden kann, umfaßt.

13. Verfahren zur Herstellung eines Fusionsproteins, umfassend einen Liganden, der umfaßt:
(i) ein natürlich vorkommendes Polypeptid, dessen biologische Rolle darin besteht, sich an einen zugehörigen Rezeptor an der Oberfläche einer Zelle zu binden;
(ii) ein Fragment des Polypeptids, das sich immer noch an seinen zugehörigen Rezeptor bindet; oder
(iii) ein Derivat von (i) oder (ii) mit veränderter Rezeptorspezifität; und ein Enzym, das fähig ist, ein Proarzneimittel in einen toxischen Stoff umzuwandeln, wobei das Verfahren das Kultivieren einer Wirtszelle umfaßt, die einen Expressionsvektor enthält, der eine für das Fusionsprotein kodierende Nukleinsäure umfaßt, wobei das Kultivieren unter Bedingungen durchgeführt wird, bei denen das Fusionsprotein exprimiert wird, sowie das Gewinnen des Fusionsproteins in im wesentlichen isolierter Form.

14. Nukleinsäure, die für ein Fusionsprotein nach Anspruch 7 oder 8 kodiert.

15. Vektor, der eine Nukleinsäure nach Anspruch 14 umfaßt.

16. Vektor nach Anspruch 15, umfassend ein bakterielles Carboxypeptidase-Gen, das durch Substitution, Deletion oder Insertion an einer oder mehreren Glykosylierungsstellen verändert worden ist, im Rahmen fusioniert mit einem Gen, das für einen Liganden oder ein Fragment oder ein Derivat davon kodiert.

17. Wirtszelle, die einen Expressionsvektor enthält, der einen Vektor nach Anspruch 15 oder 16 umfaßt, der mit einem mit der Wirtszelle kompatiblen Promotor zur Expression des Fusionsproteins operabel verbunden ist.

18. Produkt, das folgendes enthält:
(a) ein Fusionsprotein oder Konjugat aus einem Liganden mit einem Enzym, wobei der Ligand:
(i) ein natürlich vorkommendes Polypeptid, dessen biologische Rolle darin besteht, sich an einen zugehörigen Rezeptor an der Oberfläche einer Zelle zu binden;
(ii) ein Fragment des Polypeptids, das sich immer noch an seinen zugehörigen Rezeptor bindet; oder
(iii) ein Derivat von (i) oder (ii) mit veränderter Rezeptorspezifität ist; und
(b) ein Proarzneimittel, das durch das Enzym in ein aktives Arzneimittel umgewandelt werden kann;
als kombiniertes Präparat zur getrennten oder sequentiellen Verwendung zur Behandlung eines Tumors.

## Revendications

1. Système à deux composants à utiliser en association l'un avec l'autre, comprenant :
a) une protéine de fusion ou un conjugué d'un ligand avec une enzyme, le ligand étant :
(i) un polypeptide naturel dont le rôle biologique consiste à se lier à un récepteur de même orgine à la surface d'une cellule ;
(ii) un fragment du polypeptide, qui se lie encore à son récepteur de même origine, ou
(iii) un dérivé de (i) ou (ii) avec une spécificité de récepteur altérée, et
b) un promédicament qui peut être converti en un médicament actif par l'enzyme.

2. Système suivant la revendication 1, dans lequel l'enzyme est une carboxypeptidase bactérienne.

3. Système suivant la revendication 2, dans lequel la carboxypeptidase est la CPG2.

4. Système suivant la revendication 3, dans lequel la CPG2 a été modifiée à un ou plusieurs sites de glycosylation pour prévenir la glycosylation à ce ou ces sites.

5. Système suivant l'une quelconque des revendications 2 à 4, dans lequel le promédicament est une moutarde à l'azote.

6. Système suivant l'une quelconque des revendications 1 à 5, dans lequel le ligand est un facteur de croissance vasculaire (VEGF).

7. Protéine de fusion comprenant un ligand et une enzyme capable de convertir un promédicament en un agent toxique, le ligand étant :
(i) un polypeptide naturel dont le rôle biologique consiste à se lier à un récepteur de même orgine à la surface d'une cellule ;
(ii) un fragment du polypeptide, qui se lie encore à son récepteur de même origine, ou
(iii) un dérivé de (i) ou (ii) avec une spécificité de récepteur altérée.

8. Protéine de fusion telle que décrite dans SEQ ID N° 8, 10, 12, 14, 16 ou 18 ou telle que codée par l'ADN de SEQ ID N° 7, 9, 11, 13, 15 ou 17.

9. Système suivant la revendication 1, comprenant une protéine de fusion suivant la revendication 8 et un promédicament qui peut être converti en médicament actif par la CPG2.

10. Système suivant l'une quelconque des revendications 1 à 6 ou 9, à utiliser dans un procédé de traitement d'une thérapie du corps humain ou d'un animal.

11. Utilisation d'une protéine de fusion ou d'un conjugué d'un ligand avec une enzyme capable de se lier à une tumeur et une cellule et un promédicament capable d'être converti par l'enzyme en un médicament actif, dans la préparation d'un médicament à utiliser dans le traitement de la tumeur, le ligand étant :
(i) un polypeptide naturel dont le rôle biologique consiste à se lier à un récepteur de même orgine à la surface d'une cellule ;
(ii) un fragment du polypeptide, qui se lie encore à son récepteur de même origine, ou
(iii) un dérivé de (i) ou (ii) avec une spécificité de récepteur altérée.

12. Procédé de préparation d'un système à deux composants à utiliser en association l'un avec l'autre, comprenant :
a) une protéine de fusion ou un conjugué d'un ligand avec une enzyme, le ligand étant :
(i) un polypeptide naturel dont le rôle biologique consiste à se lier à un récepteur de même origine à la surface d'une cellule ;
(ii) un fragment du polypeptide, qui se lie encore à son récepteur de même origine, ou
(iii) un dérivé de (i) ou (ii) avec une spécificité de récepteur altérée, et
b) un promédicament qui peut être converti en un médicament actif par l'enzyme ;
le procédé comprenant la préparation d'une protéine de fusion ou d'un conjugué d'un ligand avec une enzyme, et la mise de la protéine de fusion ou du conjugué en association avec un promédicament qui peut être converti en un médicament actif par l'enzyme.

13. Procédé de préparation d'une protéine de fusion comprenant un ligand, lequel ligand étant :
(i) un polypeptide naturel dont le rôle biologique consiste à se lier à un récepteur de même orgine à la surface d'une cellule ;
(ii) un fragment du polypeptide, qui se lie encore à son récepteur de même origine, ou
(iii) un dérivé de (i) ou (ii) avec une spécificité de récepteur altérée, et
une enzyme capable de convertir un promédicament en un agent toxique,
le procédé comprenant la mise en culture d'une cellule hôte qui contient un vecteur d'expression qui comprend un acide nucléique codant la protéine de fusion, la mise en culture étant réalisée dans des conditions dans lesquelles la protéine de fusion est exprimée, et la récupération de la protéine de fusion sous forme sensiblement isolée.

14. Acide nucléique codant une protéine de fusion suivant la revendication 7 ou 8.

15. Vecteur comprenant un acide nucléique suivant la revendication 14.

16. Vecteur suivant la revendication 15, comprenant un gène d'une carboxypeptidase bactérienne, qui a été altéré par une substitution, délétion ou insertion à un ou plusieurs sites de glycosylation, fusionné en cadre à un gène codant un ligand ou un fragment ou dérivé de celui-ci.

17. Cellule hôte qui contient un vecteur d'expression comprenant un vecteur suivant la revendication 15 ou 16, lié de manière fonctionnelle à un promoteur compatible avec la cellule hôte pour l'expression de la protéine de fusion.

18. Produit contenant :
a) une protéine de fusion ou un conjugué d'un ligand avec une enzyme, le ligand étant :
(i) un polypeptide naturel dont le rôle biologique consiste à se lier à un récepteur de même orgine à la surface d'une cellule ;
(ii) un fragment du polypeptide, qui se lie encore à son récepteur de même origine, ou
(iii) un dérivé de (i) ou (ii) avec une spécificité de récepteur altérée, et
b) un promédicament qui peut être converti en un médicament actif par l'enzyme,
sous forme d'une préparation combinée pour une utilisation séparée ou séquentielle dans le traitement d'une tumeur.
